# EUROPEAN PATENT APPLICATION

(11) **EP 2 256 113 A1**
(43) Date of publication of application: **01.12.2010**
(21) Application number: 10171186.9
(22) Date of filing: 01.08.2006
(51) Int. Cl.: C07D 403/12, A61K 31/501, A61P 31/12

(54) **Inhibitors of serine proteases**

(30) Priority: 02.08.2005 US 704772 P
(62) Divisional of application: 06800633.7
(71) Applicant: VERTEX PHARMACEUTICALS INCORPORATED, Cambridge, MA 02139-4242 (US)
(72) Inventor: Lyons, Steve, Sudbury, MA 01776 (US); Perni, Robert B., Marlborough, MA 01752 (US)
(74) Representative: Cohausz & Florack

(57) **Abstract**

This invention relates to a mixture of diastereomeric compounds of Formula I: 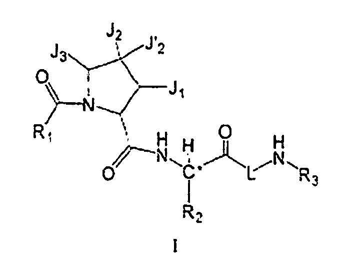 or a pharmaceutically acceptable salt or mixtures thereof, wherein
C* represents a diastereomeric carbon atom.

## Description

CLAIM OF PRIORITY

This patent application claims the benefit of U.S. provisional patent application serial no. 60/704,772, filed on August 2, 2005, which is hereby incorporated by reference.

FIELD OF THE INVENTION

The present invention relates to compounds that inhibit serine protease activity, particularly the activity of hepatitis C virus NS3-NS4A protease. As such, they act by interfering with the life cycle of the hepatitis C virus and are useful as antiviral agents. The invention further relates to compositions comprising these compounds either for ex vivo use or for administration to a patient suffering from HCV infection. The invention also relates to methods of treating an HCV infection in a patient by administering a composition comprising a compound of this invention.

BACKGROUND OF THE INVENTION

Infection by hepatitis C virus ("HCV") is a compelling human medical problem. HCV is recognized as the causative agent for most cases of non-A, non-B hepatitis, with an estimated human prevalence of 3% globally [A. Alberti et al., "Natural History of Hepatitis C," J. Hepatology, 31., (Suppl. 1), pp. 17-24 (1999)]. Nearly four million individuals may be infected in the United States alone [M.J. Alter et al., "The Epidemiology of Viral Hepatitis in the United States, Gastroenterol. Clin. North Am., 23, pp. 437-455 (1994); M. J. Alter "Hepatitis C Virus Infection in the United States," J. Hepatology, 31., (Suppl. 1), pp. 88-91 (1999)].

Upon first exposure to HCV only about 20% of infected individuals develop acute clinical hepatitis while others appear to resolve the infection spontaneously. In almost 70% of instances, however, the virus establishes a chronic infection that persists for decades [S. Iwarson, "The Natural Course of Chronic Hepatitis," FEMS Microbiology Reviews, 14, pp. 201-204 (1994); D. Lavanchy, "Global Surveillance and Control of Hepatitis C," J. Viral Hepatitis, 6, pp. 35-47 (1999)]. This usually results in recurrent and progressively worsening liver inflammation, which often leads to more severe disease states such as cirrhosis and hepatocellular carcinoma [M.C. Kew, "Hepatitis C and Hepatocellular Carcinoma", FEMS Microbiology Reviews, 14, pp. 211-220 (1994); I. Saito et. al., "Hepatitis C Virus Infection is Associated with the Development of Hepatocellular Carcinoma," Proc. Natl. Acad. Sci. USA, 87, pp. 6547-6549 (1990)]. Unfortunately, there are no broadly effective treatments for the debilitating progression of chronic HCV.

The HCV genome encodes a polyprotein of 3010-3033 amino acids [Q.L. Choo, et. al., "Genetic Organization and Diversity of the Hepatitis C Virus." Proc. Natl. Acad. Sci. USA, 88, pp. 2451-2455 (1991); N. Kato et al., "Molecular Cloning of the Human Hepatitis C Virus Genome From Japanese Patients with Non-A, Non-B Hepatitis," Proc. Natl. Acad. Sci. USA, 87, pp. 9524-9528 (1990); A. Takamizawa et. al., "Structure and Organization of the Hepatitis C Virus Genome Isolated From Human Carriers," J. Virol 65, pp. 1105-1113 (1991)]. The HCV nonstructural (NS) proteins are presumed to provide the essential catalytic machinery for viral replication. The NS proteins are derived by proteolytic cleavage of the polyprotein [R. Bartenschlager et. al., "Nonstructural Protein 3 of the Hepatitis C Virus Encodes a Serine-Type Proteinase Required for Cleavage at the NS3/4 and NS4/5 Junctions," J. Virol., 67, pp. 3835-3844 (1993); A. Grakoui et. al., "Characterization of the Hepatitis C Virus-Encoded Serine Proteinase: Determination of Proteinase-Dependent Polyprotein Cleavage Sites," J. Virol., 67, pp. 2832-2843 (1993); A. Grakoui et. al., "Expression and Identification of Hepatitis C Virus Polyprotein Cleavage Products," J. Virol., 67, pp. 1385-1395 (1993); L. Tomei et. al., "NS3 is a serine protease required for processing of hepatitis C virus polyprotein", J. Viral, 67, pp. 4017-4026 (1993)].

The HCV NS protein 3 (NS3) is essential for viral replication and infectivity [Kolykhaloy, Journal of Virology, Volume 74, pp. 2046 -2051 2000 "Mutations at the HCV NS3 Serine Protease Catalytic Triad abolish infectivity of HCV RNA in Chimpanzees]. It is known that mutations in the yellow fever virus NS3 protease decrease viral infectivity [Chambers, T.J. et. al., "Evidence that the N-terminal Domain of Nonstructural Protein NS3 From Yellow Fever Virus is a Serine Protease Responsible for Site-Specific Cleavages in the Viral Polyprotein", Proc. Natl. Acad. Sci. USA, 87, pp. 8898-8902 (1990)]. The first 181 amino acids of NS3 (residues 1027-1207 of the viral polyprotein) have been shown to contain the serine protease domain of NS3 that processes all four downstream sites of the HCV polyprotein [C. Lin et al., "Hepatitis C Virus NS3 Serine Proteinase: Trans-Cleavage Requirements and Processing Kinetics", J. Virol., 68, pp. 8147-8157 (1994)].

The HCV NS3 serine protease and its associated cofactor, NS4A, helps process all of the viral enzymes, and is thus considered essential for viral replication. This processing appears to be analogous to that carried out by the human immunodeficiency virus aspartyl protease, which is also involved in viral enzyme processing. HIV protease inhibitors, which inhibit viral protein processing, are potent antiviral agents in man indicating that interrupting this stage of the viral life cycle results in therapeutically active agents. Consequently, HCV NS3 serine protease is also an attractive target for drug discovery.

Until recently, the only established therapy for HCV disease was interferon treatment. However, interferons have significant side effects [M. A. Walker et al., "Hepatitis C Virus: An Overview of Current Approaches and Progress," DDT, 4, pp. 518-29 (1999); D. Moradpour et al., "Current and Evolving Therapies for Hepatitis C," Eur. J. Gastroenterol. Hepatol., 11, pp. 1199-1202 (1999); H. L. A. Janssen et al. "Suicide Associated with Alfa-Interferon Therapy for Chronic Viral Hepatitis," J. Hepatol., 21, pp. 241-243 (1994); P.F. Renault et al., "Side Effects of Alpha Interferon," Seminars in Liver Disease, 9, pp. 273-277. (1989)] and induce long term remission in only a fraction (≈ 25%) of cases [O. Weiland, "Interferon Therapy in Chronic Hepatitis C Virus Infection", FEMS Microbiol. Rev., 14, pp. 279-288 (1994)]. Recent introductions of the pegylated forms of interferon (PEG-INTRON® and PEGASYS®) and the combination therapy of ribavirin and interferon (REBETROL®) have resulted in only modest improvements in remission rates and only partial reductions in side effects. Moreover, the prospects for effective anti-HCV vaccines remain uncertain.

Thus, there is a need for more effective anti-HCV therapies. Such inhibitors would have therapeutic potential as protease inhibitors, particularly as serine protease inhibitors, and more particularly as HCV NS3 protease inhibitors. Specifically, such compounds may be useful as antiviral agents, particularly as anti-HCV agents.

BRIEF SUMMARY OF THE INVENTION

This invention relates to compounds of formula I, or a pharmaceutically acceptable salt or mixtures thereof, wherein the variables are described herein.

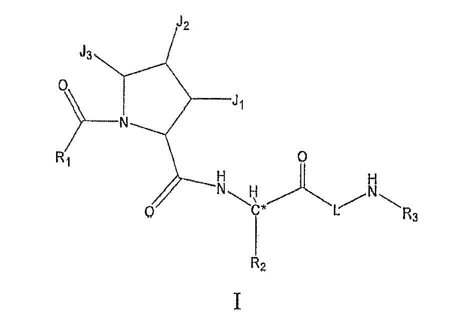

In another aspect, the invention also relates to pharmaceutical compositions that include the above compounds and uses thereof. Such compositions can be used to pre-treat devices that are to be inserted into a patient, to treat biological samples, and for direct administration to a patient. In each case, the composition will be used to lessen the risk of or the severity of the HCV infection.

Advantageously, mixtures of compounds of formula I, where the *R* isomer at position C* is present in an amount greater than 50%, unexpectedly have substantially more bioavailability than mixtures where the *S* isomer at position C* is present in an amount of 50 % or greater. Unexpectedly, the *R* isomer at the C* position is about 2 times more bioavailable than the *S* isomer at the C* position. Additionally, the *R* isomer at C* position converts, in vivo, to the *S* isomer at C* position at a higher percentage than the *S* isomer converts, in vivo, to the *R* isomer at the C* position. These properties enhance the therapeutic effectiveness of compounds of formula I with greater than 50% *R* isomer at position C* as inhibitors of serine protease activity, such as inhibiting the activity of hepatitis C virus NS3-NS4A protease.

The high bioavailability and the favorable isomer conversion properties at position C* deliver enhanced therapeutic effectiveness in compounds of the present invention, such as (1S,3aR,6aS)-2-[(2S)-2-[[(2S)-2-cyclohexyl-1-oxo-2-[(pyrazinylcarbonyl)amino]ethyl]amino]-3,3-dimethyl-1-oxobutyl]-N-[(1R)-1-[2-(cyclopropylamino)-1,2-dioxoethyl]butyl]octahydro-eyelopenta[c]pyrrole-1-carboxamide, as compared to compounds of 50% or greater *S* isomer at position C*.

DETAILED DESCRIPTION OF THE FIGURES

Figures 1A-B are plots of the mean (±SD) plasma concentrations of a compound of formula (I) with greater than 50% *R* isomer at position C* and a compound with 50% or less *R* isomer at position C* versus time following oral administration of the compound.

Figures 2A-B are plots of the mean (±SD) plasma concentrations of a compound of formula (I) with greater than 50% *R* isomer at position C* and a compound with 50% or less *R* isomer at position C* versus time following oral administration of the compound.

Figures 3A-B are plots of the mean (±SD) plasma concentrations of a compound of formula (I) with greater than 50% *R* isomer at position C* and a compound with 50% or less *R* isomer at position C* versus time following oral administration of the compound.

DETAILED DESCRIPTION OF THE INVENTION

I. DEFINITIONS

For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001, the entire contents of which are hereby incorporated by reference.

As described herein, compounds of the invention may optionally be substituted with one or more substituents, such as are illustrated generally above, or as exemplified by particular classes, subclasses, and species of the invention.

As used herein the term "aliphatic" encompasses the terms alkyl, alkenyl, alkynyl, each of which being optionally substituted as set forth below.

As used herein, an "alkyl" group refers to a saturated aliphatic hydrocarbon group containing 1-8 (e.g., 1-6 or 1-4) carbon atoms. An alkyl group can be straight or branched. Examples of alkyl groups include, but are not limited to, methyl, ethyl, propyl, isopropyl, butyl, isobutyl, sec-butyl, tert-butyl, n-pentyl, n-heptyl, or 2-ethylhexyl. An alkyl group can be substituted (i.e., optionally substituted) with one or more substituents such as halo, cycloaliphatic [e.g., cycloalkyl or cycloalkenyl], heterocycloaliphatic [e.g., heterocycloalkyl or haterocycloalkenyl], aryl, heteroaryl, alkoxy, aroyl, heteroaryl, acyl [e.g., (aliphatic)carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [e.g., aliphaticamino, cycloaliphaticamino, or heterocycloaliphaticamino], sulfonyl [e.g., aliphatic-SO₂-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroarylalkoxy, alkoxycarbonyl, alkylcarbonyloxy, or hydroxy. Without limitation, some examples of substituted alkyls include carboxyalkyl (such as HOOC-alkyl, alkoxycarbonylalkyl, and alkylcarbonyloxyalkyl), cyanoalkyl, hydroxyalkyl, alkoxyalkyl, acylalkyl, aralkyl, (alkoxyaryl)alkyl, (sulfonylamino)alkyl (such as (alkyl-SO₂-amino)alkyl), aminoalkyl, amidoalkyl, (cycloaliphatic)alkyl, or haloalkyl.

As used herein, an "alkenyl" group refers to an aliphatic carbon group that contains 2-8 (e.g., 2-6 or 2-4) carbon atoms and at least one double bond. Like an alkyl group, an alkenyl group can be straight or branched. Examples of an alkenyl group include, but are not limited to, allyl, isoprenyl, 2-butenyl, and 2-hexenyl. An alkenyl group can be optionally substituted with one or more substituents such as halo, cycloaliphatic [e.g., cycloalkyl or cycloalkenyl], heterocycloaliphatic [e.g., heterocycloalkyl or heterocycloalkenyl], aryl, heteroaryl, alkoxy, aroyl, heteroaroyl, acyl [e.g., (aliphatic)carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (heterocycloalkyl)carbonylamino, (heteracycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [e.g., aliphaticamino, cycloaliphaticamino, heterocycloaliphaticamino, or aliphaticsulfonylamino], sulfonyl [e.g., alkyl-SO₂-, cycloaliphatic-SO₂- or aryl-SO₂-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroaralkoxy, alkoxycarbonyl, alkylcarbonyloxy, or hydroxy. Without limitation, some examples of substituted alkenyls include cyanoalkenyl, alkoxyalkenyl, acylalkenyl, hydroxyalkenyl, aralkenyl, (alkoxyaryl)alkenyl, (sulfonylamino)alkenyl (such as (alkyl-SO₂-amino)alkenyl), aminoalkenyl, amidoalkenyl, (cycloaliphatic)alkenyl, or haloalkenyl.

As used herein, an "alkynyl" group refers to an aliphatic carbon group that contains 2-8 (e.g., 2-6 or 2-4) carbon atoms and has at least one triple bond. An alkynyl group can be straight or branched. Examples of an alkynyl group include, but are not limited to, propargyl and butynyl. An alkynyl group can be optionally substituted with one or more substituents such as aroyl, heteroaroyl, alkoxy, cycloalkyloxy, heterocycloalkyloxy, aryloxy, heteroaryloxy, aralkyloxy, nitro, carboxy, cyano, halo, hydroxy, sulfo, mercapto, sulfanyl [e.g., aliphaticsulfanyl or cycloaliphatiesulfanyl], sulfinyl [e.g., aliphaticsulfinyl or cycloaliphaticsulfinyl], sulfonyl [e.g., aliphatic-SO₂-, aliphaticamino-SO₂-, or cycloaliphatic-SO₂-], amido [e.g., aminocarbonyl, alkylaminocarbonyl, alkylcarbonylamino, cycloalkylaminocarbonyl, heterocycloalkylaminocarbonyl, cycloalkylcarbonylamino, arylanninocarbonyl, arylcarbonylamino, aralkylcarbonylamino, (heterocycloalkyl)carbonylamino, (cycloalkylalkyl)carbonylamino, heteroaralkylcarbonylamino, heteroarylcarbonylamino or heteroarylaminocarbonyl], urea, thiourea, sulfamoyl, sulfamide, alkoxycarbonyl, alkylcarbonyloxy, cycloaliphatic, heterocycloaliphatic, aryl, heteroaryl, acyl [e.g., (cycloaliphatic)caxbonyl or (heterocycloaliphatic)carbonyl], amino [e.g., aliphatzcamino], sulfoxy, oxo, carboxy, carbamoyl, (cycloaliphatic)oxy, (heterocycloaliphatic)oxy, or (heteroaryl)alkoxy.

As used herein, an "amido" encompasses both "aminocarbonyl" and "carbonylamino". These terms when used alone or in connection with another group refers to an amido group such as -N(R^{x})-C(O)-R^{Y} or -C(O)-N(R^{X})₂, when used terminally, and -C(O)-N(R^{X})- or -N(R^{X})-C(O)- when used internally, wherein R^{X} and R^{Y} are defined below. Examples of amido groups include alkylamido (such as alkylcarbonylamino or alkylaminocarbonyl), (heterocycloaliphatic)axnido, (heteroaralkyl)amido, (heteroaryl)amido, (heterocycloalkyl)alkylamido, arylamido, aralkylamido, (cycloalkyl)alkylamido, or cycloalkylamido.

As used herein, an "amino" group refers to -NR^{X}R^{Y} wherein each of R^{X} and R^{Y} is independently hydrogen, aliphatic, cycloaliphatic, (cycloaliphatic)aliphatic, aryl, araliphatic, heterocycloaliphatic, (heterocycloaliphatic)aliphatic, heteroaryl, carboxy, sulfanyl, sulfinyl, sulfonyl, (aliphatic)carbonyl, (cycloaliphatic)carbonyl, ((cycloaliphatic)aliphatic)carbonyl, arylcarbonyl, (araliphatic)carbonyl, (heterocycloaliphatic)carbonyl, ((heterocycloaliphatic)aliphatic)carbonyl, (heteroaryl)carbonyl, or (heteroaraliphatic)carbonyl, each of which being defined herein and being optionally substituted. Examples of amino groups include alkylamino, dialkylamino, or arylamino. When the term "amino" is not the terminal group (e.g., alkylcarbonylamino), it is represented by -NR^{X}-. R^{X} has the same meaning as defined above.

As used herein, an "aryl" group used alone or as part of a larger moiety as in "aralkyl", "aralkoxy", or "aryloxyalkyl" refers to monocyclic (e.g., phenyl); bicyclic (e.g., indenyl, naphthalenyl, tetrahydronaphthyl, tetrahydroindenyl); and tricyclic (e.g., fluorenyl tetrahydrofluorenyl, or tetrahydroanthracenyl, anthracenyl) ring systems in which the monocyclic ring system is aromatic or at least one of the rings in a bicyclic or tricyclic ring system is aromatic. The bicyclic and tricyclic groups include benzofused 2-3 membered carbocyclic rings. For example, a benzofused group includes phenyl fused with two or more C₄₋₈-arbocyclic moieties. An aryl is optionally substituted with one or more substituents including aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic ring of a benzofused bicyclic or tricyclic aryl); nitro; carboxy; amido; acyl [ e.g., aliphaticcarbonyl; (cycloaliphatic)carbonyl; ((cycloaliphatic)aliphatic)carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphatic-SO₂- or ainino-SO₂-]; sulfinyl [e.g., aliphatic-S(O)- or cycloaliphatic-S(O)-]; sulfanyl [e.g., aliphatic-S-]; cyano; halo; hydroxy; mercapto; sulfoxy; urea; thiourea; sulfamoyl; sulfamide; or carbamoyl. Alternatively, an aryl can be unsubstituted.

Non-limiting examples of substituted aryls include haloaryl [e.g., mono-, di ( such as p,m-dihaloaryl), and (trihalo)aryl]; (carboxy)aryl [e.g., (alkoxycarbonyl)aryl, ((aralkyl)carbonyloxy)aryl, and (alkoxycarbonyl)aryl]; (amido)aryl [e.g., (aminocarbonyl)aryl, (((alkylamino)alkyl)aminocarbonyl)aryl, (alkylcarbonyl)aminoaryl, (arylaminocarbonyl)aryl, and (((heteroaryl)amino)carbonyl)aryl]; aminoaryl [e.g., ((alkylsulfonyl)aniino)aryl or ((dialkyl)amino)aryl]; (cyanoalkyl)aryl; (alkoxy)aryl; (sulfamoyl)aryl [e.g., (aminosulfonyl)aryl]; (alkylsulfonyl)aryl; (cyano)aryl; (hydroxyalkyl)aryl; ((alkoxy)alkyl)aryl; (hydroxy)aryl, ((carboxy)alkyl)aryl; (((dialkyl)amino)alkyl)aryl; (nitroalkyl)aryl; (((alkylsulfonyl)amino)alkyl)aryl; ((heterocycloaliphatic)carbonyl)aryl; ((alkylsulfonyl)alkyl)aryl; (cyanoalkyl)aryl; (hydroxyalkyl)aryl; (alkylcarbonyl)aryl; alkylaryl; (trihaloalkyl)aryl; p-amino-m-alkoxycarbonylaryl; p-amino-m-cyanoaryl; p-halo-m-aminoaryl; or (m-(heterocycloaliphatic)-o-(alkyl))aryl.

As used herein, an "araliphatic" such as an "aralkyl" group refers to an aliphatic group (e.g., a C1-4 alkyl group) that is substituted with an aryl group. "Aliphatic," "alkyl," and "aryl" are defined herein. An example of an araliphatic such as an aralkyl group is benzyl.

As used herein, an "aralkyl" group refers to an alkyl group (e.g., a C1-4 alkyl group) that is substituted with an aryl group. Both "alkyl" and "aryl" have been defined above. An example of an aralkyl group is benzyl. An aralkyl is optionally substituted with one or more substituents such as aliphatic [e.g., alkyl, alkenyl, or alkynyl, including carboxyalkyl, hydroxyalkyl, or haloalkyl such as trifluoromethyl], cycloaliphatic [e.g., cycloalkyl or cycloalkenyl], (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxy, alkoxycarbonyl, alkylcarbonyloxy, amido [e.g., aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylammo, heteroarylcarbonylamino, or heteroaralkylcarbonylamino], cyano, halo, hydroxy, acyl, mercapto, alkylsulfanyl, sulfoxy, urea, thiourea, sulfamoyl, sulfamide, oxo, or carbamoyl.

As used herein, a "bicyclic ring system" includes 8-12 (e.g., 9,10, or 11) membered structures that form two rings, wherein the two rings have at least one atom in common (e.g., 2 atoms in common). Bicyclic ring systems include bicycloaliphatics (e.g., bicycloalkyl or bicycloalkenyl), bicycloheteroaliphatics, bicyclic aryls, and bicyclic heteroaryls.

As used herein, a cycloaliphatic" group encompasses a "cycloalkyl" group and a "cycloalkenyl" group, each of which being optionally substituted as set forth below.

As used herein, a "cycloalkyl group refers to a saturated carbocyclic mono- or bicyclic (fused or bridged) ring of 3-10 (e.g., 5-10) carbon atoms. Examples of cycloalkyl groups include cyclopropyl, cyclobutyl, cyclopentyl, cyclohexyl, cycloheptyl, adamantyl, norbornyl, cubyl, octahydro-indenyl, decahydro-naphthyl, bicyclo[3.2.1]octyl, bicyolo[2.2.2]octyl, bicyclo[3.3.1]nonyl, bicyclo[3.3.2.]decyl, bicyclo[2.2.2]octyl, adamantyl, azacycloalkyl, or ((aminocarbonyl)cycloalkyl)cycloalkyl. A "cycloalkenyl" group, as used herein, refers to a non-aromatic carbocyclic ring of 3-10 (e.g., 4-8) carbon atoms having one or more double bonds. Examples of cycloalkenyl groups include cyclopentenyl, 1,4-cyclohexa-di-enyl, cycloheptenyl, cyclooctenyl, hexahydro-indenyl, octahydro-naphthyl, cyclohexenyl, cyclopentenyl, bicyclo[2.2.2]octenyl, or bicyclo[3.3.1]nonenyl. A cycloalkyl or cycloalkenyl group can be optionally substituted with one or more substituents such as aliphatic [e.g., alkyl, alkenyl, or alkynyl], cycloaliphatic, (cycloaliphatic) aliphatic, heterocycloaliphatic, (heterocycloaliphatic) aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic)aliphatic)carbonylamino, (aryl)carbonylamino, (araliphatic)carbonylamino, (heterocycloaliphatic)carbonylamino, ((heterocycloaliphatic)aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)caxbonyl], cyano, halo, hydroxy, mercapto, sulfony1 [e.g., alkyl-SO₂- and aryl-SO₂-], sulfinyl [e.g., alkyl-S(O)-], sulfanyl [e.g., alkyl-S-], sulfoxy, urea, thiourea, sulfamoyl, sulfamide, oxo, or carbamoyl.

As used herein, "cyclic moiety" includes cycloaliphatic, heterocycloaliphatic, aryl, or heteroaryl, each of which has been defined previously.

As used herein, the term "heteracycloaliphatic" encompasses a heterocycloalkyl group and a heterocycloalkenyl group, each of which being optionally substituted as set forth below.

As used herein, a "heterocycloalkyl" group refers to a 3-10 membered mono- or bicylic (fused or bridged) (e.g., 5- to 10-membered mono- or bicyclic) saturated ring structure, in which one or more of the ring atoms is a heteroatom (e.g., N, O, S, or combinations thereof). Examples of a heterocycloalkyl group include piperidyl, piperazyl, tetrahydropyranyl, tetrahydrofuryl, 1,4-dioxolanyl, 1,4-dithianyl, 1,3-diaxolan.yl, oxazolidyl, isoxazolidyl, morpholinyl, thiomorpholyl, octahydrobenzofuryl, octahydrochromenyl, octahydrothiochromenyl, octahydroindolyl, octahydropyrindinyl, decahydroquinolinyl, octahydrobenzo[b]thiopheneyl, 2-oxa-bicyclo[2.2.2]octyl, 1-aza-bicyclo[2.2.2]octyl, 3-aza-bicyclo[3.2.1]octyl, anad 2,6-dioxa-tricyclo[3.3.1.03,7]nonyl. A monocyclic heterocycloalkyl group can be fused with a phenyl moiety such as tetrahydroisoquinoline. A "heterocycloalkenyl" group, as used herein, refers to a mono- or bicylic (e.g., 5- to 10-membered mono- or bicyclic) non-aromatic ring structure having one or more double bonds, and wherein one or more of the ring atoms is a heteroatom (e.g., N, O, or S). Monocyclic and bicycloheteroaliphatics are numbered according to standard chemical nomenclature.

A heterocycloalkyl or heterocycloalkenyl group can be optionally substituted with one or more substituents such as aliphatic [e.g., alkyl, alkenyl, or alkynyl], cycloaliphatic, (cycloaliphatic)aliphatic, heterocycloaliphatic, (heterocycloaliphatic)aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic) aliphatic)carbonylamino, (aryl)carbonylamino, (araliphatic)carbonylamino, (heterocycloaliphatic)carbonylamino, ((heterocycloaliphatic) aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)carbonyl], nitro, cyano, halo, hydroxy, mercapto, sulfonyl [e.g., alkylsulfonyl or arylsulfonyl], sulfinyl [e.g., alkylsulfinyl], sulfanyl [e.g., alkylsulfanyl], sulfoxy, urea, thiourea, sulfamoyl, sulfamide, oxo, or carbamoyl.

A "heteroaryl" group, as used herein, refers to a monocyclic, bicyclic, or tricyclic ring system having 4 to 15 ring atoms wherein one or more of the ring atoms is a heteroatom (e.g., N, O, S, or combinations thereof) and in which the monocyclic ring system is aromatic or at least one of the rings in the bicyclic or tricyclic ring systems is aromatic. A heteroaryl group includes a benzofused ring system having 2 to 3 rings. For example, a benzofused group includes benzo fused with one or two 4 to 8 membered heterocycloaliphatic moieties (e.g., indolizyl, indolyl, isoindolyl, 3H-indolyl, indolinyl, benzo[b]furyl, benzo[b]thiophenyl, quinolinyl, or isoquinolinyl). Some examples of heteroaryl are azetidinyl, pyridyl, 1H-indazo[y], furyl, pyrrolyl, thienyl, thiazolyl, oxazolyl, imidazolyl, tetrazolyl, benzofuryl, isoquinolinyl, benzthiazolyl, xanthene, thioxanthene, phenothiazine, dihydroindole, benzo[1,3]dioxole, benzo[b]furyl, benzo[b]thiophenyl, indazolyl, benzimidazolyl, benzthiazolyl, puryl, cinnolyl, quinolyl, quinazolyl,cinmolyl, phthalazyl, quinazolyl, quinoxalyl, isoquinolyl, 4H-quinolizyl, benzo-1,2,5-thiadiazolyl, or 1,8-naphthyridyl.

Without limitation, monocyclic heteroaryls include furyl, thiophenyl, 2H-pyrrolyl, pyrrolyl, oxazolyl, thazolyl, imidazolyl, pyrazolyl, isoxazolyl, isothiazolyl, 1,3,4-thiadiazolyl, 2H-pyranyl, 4-H-pranyl, pyridyl, pyridazyl, pyrimidyl, pyrazolyl, pyrazyl, or 1,3,5-triazyl. Monocyclic heteroaryls are numbered according to standard chemical nomenclature.

Without limitation, bicyclic heteroaryls include indolizyl, indolyl, isoindolyl, 3H-indolyl, indolinyl, benzo[b]furyl, benzo[b]thiophenyl, quinolinyl, isoquinolinyl, indolizyl, isoindolyl, indolyl, benzo[b]furyl, bexo[b]thiophenyl, indazolyl, benzimidazyl, benzthiazolyl, purinyl, 4H-quinolizyl, quinolyl, isoquinolyl, cinnolyl, phthalazyl, quinazolyl, quinoxalyl, 1,8-naphthyridyl, or pteridyl. Bicyclic heteroaryls are numbered according to standard chemical nomenclature.

A heteroaryl is optionally substituted with one or more substituents such as aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic or heterocyclic ring of a bicyclic or tricyclic heteroaryl); carboxy; amido; acyl [ e.g., aliphaticcarbonyl; (cycloaliphatic)carbonyl; ((cycloaliphatic)aliphatic)carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphaticsulfonyl or aminosulfonyl]; sulfinyl [e.g., aliphaticsulfinyl]; sulfanyl [e.g., aliphaticsulfanyl]; nitro; cyano; halo; hydroxy; mercapto; sulfoxy; urea; thiourea; sulfamoyl; sulfamide; or carbamoyl. Alternatively, a heteroaryl can be unsubstituted.

Non-limiting examples of substituted heteroaryls include (halo)heteroaryl [e.g., mono- and di-(halo)heteroaryl]; (carboxy)heteroaryl [e.g., (alkoxycarbonyl)heteroaryl]; cyanoheteroaryl; aminoheteroaryl [e.g., ((alkylsulfonyl)amino)heteroaryl and((dialkyl)amino)heteroaryl]; (amido)heteroaryl [e.g., aminocarbonylheteroaryl, ((alkylcarbonyl)amino)heteroaryl, ((((alkyl)amino)alkyl)aminocarbonyl)heteroaryl, (((heteroaryl)amino)carbonyl)heteroaryl, ((heterocycloaliphatic)carbonyl)heteroaryl, and ((alkylcarbonyl)amino)heteroaryl]; (cyanoalkyl)heteroaryl; (alkoxy)heteroaryl; (sultamoyl)heteroaryl [e.g., (aminosulfonyl)heteroaryl]; (sulfonyl)heteroaryl [e.g., (alkylsulfonyl)heteroaryl]; (hydroxyalkyl)heteroaryl; (alkoxyalkyl)heteroaryl; (hydroxy)heteroaryl; ((carboxy)alkyl)heteroaryl; (((dialkyl)amino)alkyl]heteroaryl; (heterocycloaliphatic)heteroaryl; (cycloaliphatic)heteroaryl; (nitroalkyl)heteroaryl; (((alkylsulfonyl)amino)alkyl)heteroaryl; ((alkylsulfonyl)alkyl)heteroaryl; (cyanoalkyl)heteroaryl; (acyl)heteroaryl [e.g., (alkylcarbonyl)heteroaryl]; (alkyl)heteroaryl, and (haloalkyl)heteroaryl [e.g., trihaloalkylheteroaryl].

A "heteroaraliphatic (such as a heteroaralkyl group) as used herein, refers to an aliphatic group (e.g., a C₁₋₄-alkyl group) that is substituted with a heteroaryl group. "Aliphatic," "alkyl," and "heteroaryl" have been defined above.

A "heteroaralkyl" group, as used herein, refers to an alkyl group (e.g., a C₁₋₄-alkyl group) that is substituted with a heteroaryl group. Both "alkyl" and "heteroaryl" have been defined above. A heteroaralkyl is optionally substituted with one or more substituents such as alkyl (including carboxyalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxy, alkoxycarbonyl, alkylcarbonyloxy, aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl.

As used herein, an "acyl" group refers to a formyl group or R^{X}-C(O)- (such as alkyl-C(O)-, also referred to as "alkylcarbonyl") where R^{X} and "alkyl" have been defined previously. Acetyl and pivaloyl are examples of acyl groups.

As used herein, an "aroyl" or "heteroaroyl" refers to an aryl-C(O)- or a heteroaryl-C(O)-. The aryl and heteroaryl portion of the aroyl or heteroaroyl is optionally substituted as previously defined.

As used herein, an "alkoxy" group refers to an alkyl-O- group where "alkyl" has been defined previously.

As used herein, a "carbamoyl" group refers to a group having the structure -O-CO-NR^{X}R^{Y} or -NR^{X}-CO-O-R^{Z} wherein R^{X} and R^{Y} have been defined above and R^{Z} can be aliphatic, aryl, araliphatic, heterocycloaliphatic, heteroaryl, or heteroaraliphatic.

As used herein, a "carboxy group refers to -COOH, -COOR^{X}, -OC(O)H, -OC(O)R^{X} when used as a terminal group; or -OC(O)- or -C(O)O- when used as an internal group.

As used herein, a "haloaliphatic" group refers to an aliphatic group substituted with 1-3 halogen. For instance, the term haloalkyl includes the group -CF₃.

As used herein, a "mercapto" group refers to -SH.

As used herein, a "sulfo" group refers to -SO3H or -SO3R^{X} when used terminally or S(O)3- when used internally.

As used herein, a "sulfamide" group refers to the structure -NR^{X}-S(O)₂-NR^{Y}R^{Z} when used terminally and -NR^{X}-S(O)₂-NR^{Y}- when used internally, wherein R^{X}, R^{Y}, and R^{Z} have been defined above.

As used herein, a "sulfonamide" group refers to the structure -S(O)₂-NR^{X}R^{Y} or -NR^{X}-S(O)2-R^{Z} when used terminally; or -S(O)₂-NR^{X}- or -NR^{x} -S(O)₂- when used internally, wherein R^{X}, R^{Y}, and R^{Z} are defined above.

As used herein a "sulfanyl" group refers to -S-R^{X} when used terminally and -S- when used internally, wherein R^{X} has been defined above. Examples of sulfanyls include aliphatic-S-, cycloaliphatic-S-, aryl-S-, or the like.

As used herein a "sulfinyl" group refers to -S(O)-R^{X} when used terminally and -S(O)-when used internally, wherein R^{X} has been defined above. Exemplary sulfinyl groups include aliphatic-S(O)-, aryl-S(O)-, (cycloaliphatic(aliphatic)) -S(O)-, cycloalkyl-S(O)-, heterocycloaliphatic-S(O)-, heteroaryl-S(O)-, or the like.

As used herein, a "sulfonyl" group refers to-S(O)₂-R^{X} when used terminally and - S(O)₂- when used internally, wherein R^{X} has been defined above. Exemplary sulfonyl groups include aliphatic-S(O)₂-, aryl-S(O)₂-, (cycloaliphatic(aliplaatic))-S(O)₂-, cycloaliphatic-S(O)₂-, heterocycloaliphatic-S(O)₂-, heteroaryl-S(O)₂-, (cycloaliphatic(amido(aliphatic)))-S(O)₂-or the like.

As used herein, a "sulfoxy" group refers to -O-SO-R^{X} or -SO-O-R^{X}, when used terminally and -O-S(O)- or -S(O)-O- when used internally, where R^{X} has been defined above.

As used herein, a "halogen" or "halo" group refers to fluorine, chlorine, bromine or iodine.

As used herein, an "alkoxycarbonyl," which is encompassed by the term carboxy, used alone or in connection with another group refers to a group such as alkyl-O-C(O)-.

As used herein, an "alkoxyalkyl" refers to an alkyl group such as alkyl-O-alkyl-, wherein alkyl has been defined above.

As used herein, a "carbonyl" refer to -C(O)-.

As used herein, an oxo" refers to =O.

As used herein, an "aminoalkyl" refers to the structure (R^{X})₂N-alkyl-.

As used herein, a "cyanoalkyl" refers to the structure (NC)-alkyl-.

As used herein, a "urea" group refers to the structure -NR^{X}-CO-NR^{Y}R^{Z} and "thiourea" group refers to the structure -NR^{X}-CS-NR^{Y}R^{Z} when used terminally and -NR^{X}-CO-NR^{Y}- or -NR^{X}-CS-NR^{Y}- when used internally, wherein R^{X}, R^{Y}, and R^{Z} have been defined above.

As used herein, a "guanidine" group refers to the structure -N=C(N(R^{X}R^{Y}))N(R^{X}R^{Y})
or
-NR^{X}-C(=NR^{X})NR^{X}R^{Y} wherein R^{X} and R^{Y} have been defined above.

As used herein, the term "amidino" group refers to the structure -C=(NR^{X})N(R^{X}R^{Y}) wherein R^{X} and R^{Y} have been defined above.

In general, the term "vicinal" refers to the placement of substituents on a group that includes two or more carbon atoms, wherein the substituents are attached to adjacent carbon atoms.

In general, the term "geminal" refers to the placement of substituents on a group that includes two or more carbon atoms, wherein the substituents are attached to the same carbon atom.

The terms "terminally" and "internally" refer to the location of a group within a substituent. A group is terminal when the group is present at the end of the substituent not further bonded to the rest of the chemical structure. Carboxyalkyl, i.e., R^{X}O(O)C-alkyl is an example of a carboxy group used terminally. A group is internal when the group is present in the middle of a substituent to at the end of the substituent bound to the rest of the chemical structure. Alkylcarboxy (e.g., alkyl-C(O)O- or alkyl-OC(O)-) and alkylcarboxyaryl (e.g., alkyl-C(O)O-aryl- or alkyl-O(CO)-aryl-) are examples of carboxy groups used internally.

As used herein, "cyclic group" includes mono-, bi-, and tri-cyclic ring systems including cycloaliphatic, heterocycloaliphatic, aryl, or heteroaryl, each of which has been previously defined.

As used herein, a "bridged bicyclic ring system" refers to a bicyclic heterocyclicalipahtic ring system or bicyclic cycloaliphatic ring system in which the rings are bridged. Examples of bridged bicyclic ring systems include, but are not limited to, adamantanyl, norbornanyl, bicyclo[3.2.1]octyl, bicyclo[2.2.2]octyl, bicyclo[3.3.1]nonyl, bicyclo[3.2.3]nonyl, 2-oxabicyclo[2.2.2]octyl, 1-azabicyclo[2.2.2]octyl, 3-azabicyclo[3.2.1]octyl, and 2,6-dioxa-tricyclo[3.3.1.03,7]nonyl. A bridged bicyclic ring system can be optionally substituted with one or more substituents such as alkyl (including carboxyalkyl, hydroxyalkyl, and haloalkyl such as trifluoromethyl), alkenyl, alkynyl, cycloalkyl, (cycloalkyl)alkyl, hetexocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxy, alkoxycarbonyl, alkylcarbonyloxy, aminocarbonyl, alkylcarbonylamino, cycloalkylcarbonylamino, (cymoalkylalkyl)carbonylamino, arylcarbonylamino, aralkylcarbonylamino, (heterocycloalkyl)carbonylamino, (heterocycloalkylalleyl)carbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl.

As used herein, an "aliphatic chain" refers to a branched or straight aliphatic group (e.g., alkyl groups, alkenyl groups, or alkynyl groups). A straight aliphatic chain has the structure
-[CH₂]ᵥ-, where v is 1-6. A branched aliphatic chain is a straight aliphatic chain that is substituted with one or more aliphatic groups. A branched aliphatic chain has the structure
-[CHQ]ᵥ- where Q is hydrogen or an aliphatic group; however, Q shall be an aliphatic group in at least one instance. The term aliphatic chain includes alkyl chains, alkenyl chains, and alkynyl chains, where alkyl, alkenyl, and alkynyl are defined above.

The term "tricyclic fused ring system" refers to a cycloaliphatic, heterocycloaliphatic, aryl or heteroaryl system containing three rings, each ring sharing at least two common atoms with at least one other ring. Non-limiting examples of a tricyclic fused ring system include anthracene, xanthene, 1H-phenalene, tetradecahydrophenanthrene, acridine and phenothiazine.

The phrase "optionally substituted" is used interchangeably with the phrase "substituted or unsubstituted." As described herein, compounds of the invention can optionally be substituted with one or more substituents, such as are illustrated generally above, or as exemplified by particular classes, subclasses, and species of the invention. As described herein, the variables in formula I, e.g., R₁, R₂, and R₃, and other variables contained therein encompass specific groups, such as alkyl and aryl. Unless otherwise noted, each of the specific groups for the variables R₁, R₂, and R₃, and other variables contained therein can be optionally substituted with one or more substituents described herein. Each substituent of a specific group is further optionally substituted with one to three of halo, cyano, oxo, alkoxy, hydroxy, amino, nitro, aryl, haloalkyl, and alkyl. For instance, an alkyl group can be substituted with alkylsulfanyl and the alkylsulfanyl can be optionally substituted with one to three of halo, cyano, oxo, alkoxy, hydroxy, amino, nitro, aryl, haloalkyl, and alkyl. As an additional example, the cycloalkyl portion of a (cycloalkyl)carbonylamino can be optionally substituted with one to three of halo, cyano, alkoxy, hydroxy, nitro, haloalkyl, and alkyl. When two alkoxy groups are bound to the same atom or adjacent atoms, the two alkoxy groups can form a ring together with the atom(s) to which they are bound.

In general, the term "substituted," whether preceded by the term "optionally" or not, refers to the replacement of hydrogen radicals in a given structure with the radical of a specified substituent. Specific substituents are described above in the definitions and below in the description of compounds and examples thereof. Unless otherwise indicated, an optionally substituted group can have a substituent at each substitutable position of the group, and when more than one position in any given structure can be substituted with more than one substituent selected from a specified group, the substituent can be either the same or different at every position. A ring substituent, such as a heterocycloalkyl, can be bound to another ring, such as a cycloalkyl, to form a spiro-bicyclic ring system, e.g., both rings share one common atom. As one of ordinary skill in the art will recognize, combinations of substituents envisioned by this invention are those combinations that result in the formation of stable or chemically feasible compounds.

The phrase "stable or chemically feasible," as used herein, refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40° C or less, in the absence of moisture or other chemically reactive conditions, for at least a week.

As used herein, an effective amount is defined as the amount required to confer a therapeutic effect on the treated patient, and is typically determined based on age, surface area, weight, and condition of the patient. The interrelationship of dosages for animals and humans (based on milligrams per meter squared of body surface) is described by Freireich et al., Cancer Chemother. Rep., 50: 219 (1966). Body surface area may be approximately determined from height and weight of the patient. See, e.g., Scientific Tables, Geigy Pharmaceuticals, Ardsley, New York, 537 (1970). As used herein, "patient" refers to a mammal, including a human.

Unless otherwise stated, structures depicted herein are also meant to include all isomeric (e.g., enantiomeric, diastereomeric, and geometric (or conformational)) forms of the structure; for example, the R and *S* configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹³C- or ¹⁴C-enriched carbon are within the scope of this invention. Such compounds are useful, for example, as analytical tools or probes in biological assays.

A compound of formula (I) that is acidic in nature (e.g., having a carboxyl or phenolic hydroxyl group) can form a pharmaceutically acceptable salt such as a sodium, potassium, calcium, or gold salt. Also within the scope of the invention are salts formed with pharmaceutically acceptable amines such as ammonia, alkyl amines, hydroxyalkylamines, and N-methylglycamine. A compound of formula I can be treated with an acid to form acid addition salts. Examples of such acids include hydrochloric acid, hydrobromic acid, hydroiodic acid, sulfuric acid, methanesulfonic acid, phosphoric acid, p-bromophenylsulfonic acid, carbonic acid, succinic acid, citric acid, benzoic acid, oxalic acid, malonic acid, salicylic acid, malic acid, fumaric acid, ascorbic acid, maleic acid, acetic acid, and other mineral and organic acids well known to those skilled in the art. The acid addition salts can be prepared by treating a compound of formula I in its free base form with a sufficient amount of an acid (e.g., hydrochloric acid) to produce an acid addition salt (e.g., a hydrochloride salt). The acid addition salt can be converted back to its free base form by treating the salt with a suitable dilute aqueous basic solution (e.g., sodium hydroxide, sodium bicarbonate, potassium carbonate, or ammonia). Compounds of formula (I) can also be, for example, in a form of achiral compounds, racemic mixtures, optically active compounds, pure diastereomers, or a mixture of diastereomers.

The terms "50% or less *R* isomer" is used interchangeably with "50% or greater *S* isomer".

The following abbreviations have the following meanings. If an abbreviation is not defined, it has its generally accepted meaning.
BEMP = 2-tert-butylimino-2- diethylamino- 1,3-dimethylperhydro-1,3,2- diazaphosphorine Boc = t-butoxycarbonyl
BOP = benzotriazol-1-yloxy-tris (dimathylamino)phosphonium hexafluorophosphate
bd = broad doublet
bs = broad singlet
d = doublet
dd = doublet of doublets
DIC = diisopropylearbodiimide
DMF = dimethylformamide
DMAP = dimethylaminopyridine
DMSO = dimethylsulfoxide
EDC = ethyl-1-(3-dimethyaminopropyl)carbodiimide
eq. = equivalents
EtOAc = ethyl acetate
g = grams
HOBT= 1-hydroxybenzotriazole
DIPEA = Hunig's base = diisopropylethylamine
L = liter
m = multiplet
M = molar
max = maximum
meq = milliequivalent
mg = milligram
mL = milliliter
mm = millimeter
mmol = millimole
MOC = methoxyoxycarbonyl
N = normal
ng = nanogram
nm = nanometers
OD = optical density
PEPC = 1-(3-(1-pyrrolidinyl)propyl)-3-ethylcarbodiimide
PP-HOBT = piperidine-piperidine-1-hydroxybenzotrizole
psi = pounds per square inch
Ph = phenyl
q = quartet
quint. = quintet
rpm = rotations per minute
s = singlet
t = triplet
TFA = trifluoroacetic acid
THF = tetrahydrofuran
tlc = thin layer chromatography
µL = microliter
UV = ultra-violet

II. COMPOUNDS

Compounds of the present invention provide desirable therapeutic treatments because they were observed to have a greater bioavailability when the *R* isomer at position C* was greater than 50% of the mixture (e.g., about 60%, about 70%, about 80%, about 85%, about 90%, about 95%, or about 98%). Unexpectedly, the *R* isomer at the C* position is about 2 times more bioavailable than the *S* isomer at the C* position. Additionally, the *R* isomer at C* position converts in vivo to the *S* isomer at C* position at a higher percentage than the *S* at the C* position. These properties enhance the therapeutic effectiveness of compounds of formula I with greater than 50% *R* isomer at position C* as inhibitors of serine protease activity, such as inhibiting the activity of hepatitis C virus NS3-NS4A protease. For instance, some embodiments of the present invention that were greater than 50% *R* isomer at C* had measured Ki(app)'s of less than 3 µM (e.g., about 2 µM, about 1.5 µM , or about 1.190 µM), IC₅₀'s of less than about 0.9 µM (e.g., about 0.883 µM), and a CC₅₀ of greater than 100 µM.

The high bioavailability and the favorable isomer conversion properties at position C* deliver enhanced therapeutic effectiveness in compounds of the present invention, such as (1S,3a*R*,6a*S*)-2-[(2*S*)-2-[[(2*S*)-2-cyclohexyl-1-oxo-2-[(pyrazinylcarbonyl)amino] ethyl] amino] -3,3-dimethyl-1-oxobutyl]-N-[(1*R*)-1-[2-(cyclopropylamino)-1,2-dioxoethyl]butyl]octahydro-cyclopenta[c]pyrrole-1-carboxamide, as compared to compounds of 50% or greater *S* isomer at position C*.

The present invention provides a compound of formula I 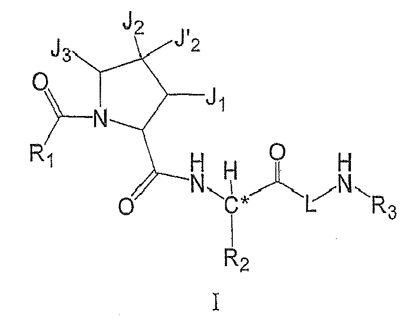 or a pharmaceutically acceptable salt or mixtures thereof, wherein

C* represents a diasteromeric carbon atom; and
the *R* isomer is greater than 50% of the mixture relative to the *S* isomer at the C* position.

R₁ is RW-, P₃-, or P₄-L₂-P₃-.

R is an optionally substituted aliphatic, an optionally substituted cycloaliphatic, an optionally substituted heterocycloaliphatic, an optionally substituted aryl, or an optionally substituted heteroaryl.

W is a bond, -NR₄-, -O- or -S-.

R₄ is H, an optionally substituted aliphatic, an optionally substituted cycloaliphatic, an optionally substituted heterocycloaliphatic, an optionally substituted aryl, or an optionally substituted heteroaryl.

P₃- is

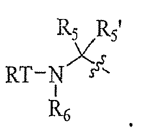

T is -C(O)-, -OC(O)-, -NHC(O)-, -C(O)C(O)-, or -SO2-.

Each of R₅ and R₅' is independently H, an optionally substituted aliphatic, an optionally substituted cycloaliphatic, an optionally substituted heterocycloaliphatic, an optionally substituted phenyl, or an optionally substituted heteroaryl.

R₆ is an optionally substituted aliphatic, an optionally substituted heteroaryl, an optionally substituted phenyl; or R₅ and R₆, together with the atoms to which they are attached, may form a 5- to 7-membered optionally substituted monocyclic heterocycloaliphatic, or a 6- to 12-membered optionally substituted bicyclic heterocycloaliphatic, in which each heterocycloaliphatic ring optionally contains an additional heteroatom selected from -O-, -S- or -NR₅₀-.

R₅₀ is H, an optionally substituted aliphatic, an optionally substituted heteroaryl, or an optionally substituted phenyl.

P₄-L₂-P₃ is

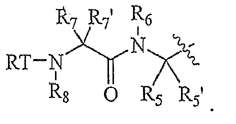

Each of R₇ and R₇' is independently H, an optionally substituted aliphatic, an optionally substituted cycloaliphatic, an optionally substituted heterocycloaliphatic, an optionally substituted phenyl, or an optionally substituted heteroaryl; or R₇ and R₇', together with the atom to which they are attached, may form a 3- to 7-membered cycloaliphatic or heterocycloaliphatic ring; or R₇ and R₆, together with the atoms to which they are attached, may form a 5- to 7-membered optionally substituted monocyclic heterocycloaliphatic, a 5- to 7-membered optionally substituted monocyclic heteroaryl, a 6- to 12-membered optionally substituted bicyclic heterocycloaliphatic, or a 6- to 12-membered optionally substituted bicyclic heteroaryl, in which each heterocycloaliphatic or heteroaryl ring optionally contains an additional heteroatom selected from -O-, -S- or -NR₅₀-, or when R₅ and R₆, together with the atoms to which they are attached, may form a ring; R₇ and the ring system formed by R₅ and R₆ may form an 8- to 14-membered optionally substituted bicyclic fused ring system, wherein the bicyclic fused ring system is optionally further fused with an optionally substituted phenyl to form an optionally substituted 10- to 16-membered tricyclic fused ring system.

R₈ is H or a protecting group.

R₂ is an optionally substituted aliphatic, an optionally substituted cycloaliphatic, an optionally substituted heterocycloaliphatic, an optionally substituted heteroaryl, or an optionally substituted phenyl.

R₃ is H, an optionally substituted aliphatic, an optionally substituted cycloaliphatic, an optionally substituted heterocycloaliphatic, an optionally substituted aryl, or an optionally substituted heteroaryl.

L is a bond, -CF₂-, -C(O)-, or -SO₂-.

Each of J₁, J₂, J'₂, and J₃ is independently halogen, -OR', -OC(O)N(R')₂, -NO₂, -CN, - CF₃, -OCF₃, -R', oxo, thioxo, -N(R')₂, -SR', -COR', -SO₂R', -SO₂N(R')₂, -SO₃R', -C(O)R', -C(O)C(O)R', -C(O)CH₂C(O)R', -C(S)R', -C(O)OR', -OC(O)R', -C(O)N(R')₂, -OC(O)N(R')₂, -C(S)N(R')₂, -(CH₂)₀₋₂NHC(O)R', -N(R')N(R')COR', -N(R')N(R')C(O)OR', -N(R')N(R')CON(R')₂, -N(R')SO₂R', -N(R')SO₂N(R')₂, -N(R')C(O)OR', -N(R')C(O)R', -N(R')C(S)R', -N(R')C(O)N(R')₂, -N(R')C(S)N(R')₂, -N(COR')COR', -N(OR')R', - C(=NH)N(R')₂, -C(O)N(OR')R', -C(=NOR')R', -OP(O)(OR')₂, -P(O)(R')₂, -P(O)(OR')₂, or - P(O)(H)(OR'), or one selected from J₂ and J'₂ is H, wherein two R' groups together with the atoms to which they are bound may form a 3- to 10-membered aromatic or non-aromatic ring system having up to 3 heteroatoms independently selected from N, O, or S, wherein the ring is optionally fused to a C₆-C₁₀ aryl, a C₅-C₁₀ heteroaryl, a C₃-C₁₀ cycloalkyl, or a C₃-C₁₀ heterocycloaliphatic, and wherein any ring has up to 3 substituents each independently selected from J₂, or one of J₂ or J'₂ is hydrogen.

Each R' is independently selected from H, C₁-C₁₂ aliphatic, C₃-C₁₀ cycloalkyl, or C₃-C₁₀ cycloalkenyl, C₃-C₁₀ cycloalkyl-C₁-C₁₂ aliphatic, C₃-C₁₀ cycloalkenyl-C₁-C₁₂ aliphatic, Ce-Cioaryl, C₆₋₁₀ aryl-C₁-C₁₂ aliphatic, 3- to 10-membered heterocycloaliphatic, 6-to 10-membered heterocycloaliphatic-C₁-C₁₂ aliphatic, 5- to 10-membered heteroaryl, or 5- to 10-membered heteroaryl-C₁-C₁₂aliphatic, wherein R' has up to 3 substituents each independently selected from J₂.

In several embodiments, J₁ and J₂, together with the atoms to which they are attached, may form a C₈ to C₁₂ optionally substituted bicyclic ring.

In several embodiments, J₁ and J₃, together with the atoms to which they are attached, may form a C₈ to C₁₂ optionally substituted bicyclic ring.

In several embodiments, J₂ and J'_{2,} together with the carbon atom to which they are attached, may form an optionally substituted 5-10 membered cycloaliphatic, or an optionally substituted 5- to 10-membered heterocycloaliphatic ring.

In several embodiments, J₂ and J₃, together with the atoms to which they are attached, may form a C₈ to C₁₂ optionally substituted bicyclic ring.

Compounds of the present invention can contain one or more asymmetric centers. These asymmetric centers can independently be in either the *R* or *S* configuration. Certain compounds of the invention can also exhibit geometrical isomerism. The present invention also includes individual geometrical isomers and stereoisomers and mixtures thereof, including racemic mixtures, of compounds according to the invention.

In several embodiments, a compound of the present invention includes the moiety 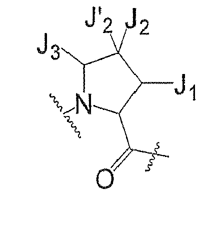 which is one selected from: 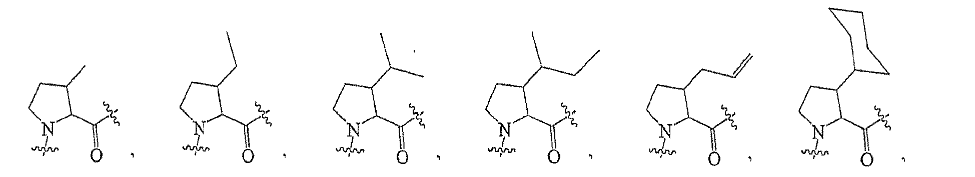 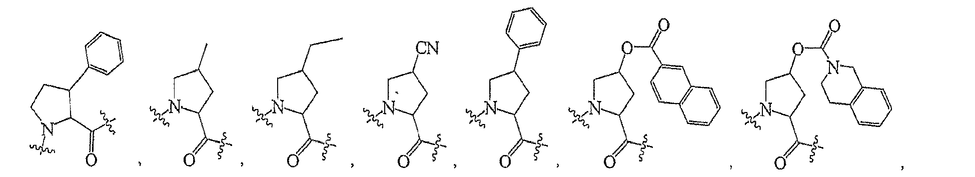 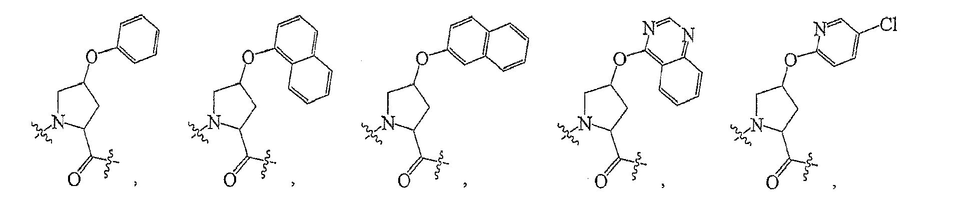 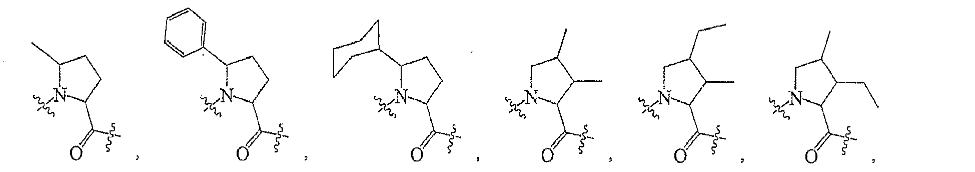 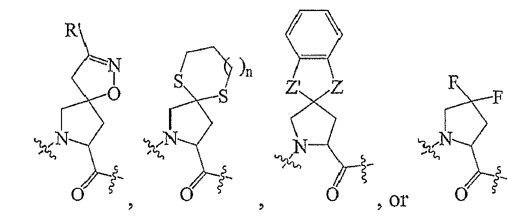 wherein n is 0 or 1 and each of Z and Z' is independently -CR'R'-, -S- or -O-

In several embodiments, a compound of formula I includes a structure wherein J₁ and J₂, together with the atoms to which they are attached, form an optionally substituted mono- or bicyclic ring such that the 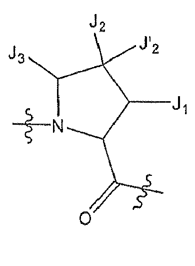 moiety is

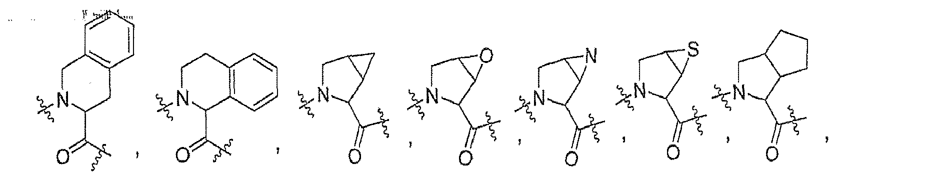

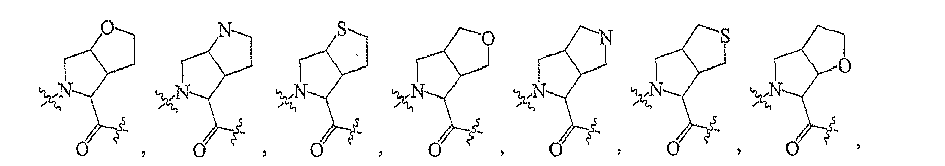

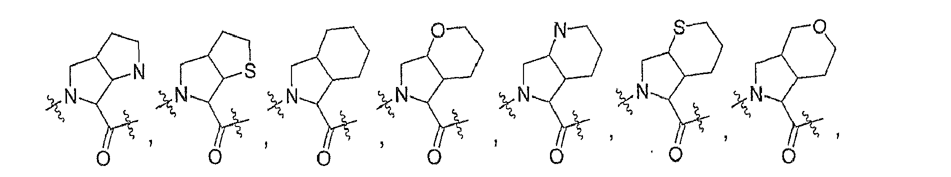

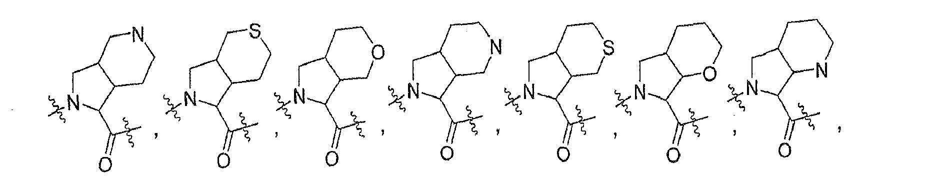

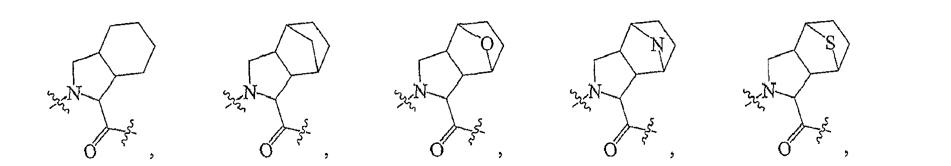

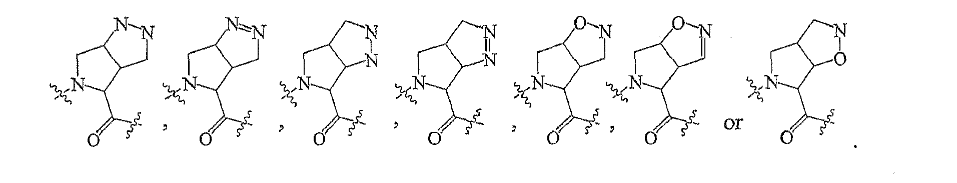

In several embodiments, a compound of formula I includes a structure wherein J₁ and J₂, together with the atoms to which they are attached, form an optionally substituted mono- or bicyclic ring such that the 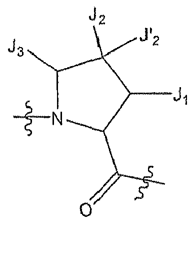 moiety is

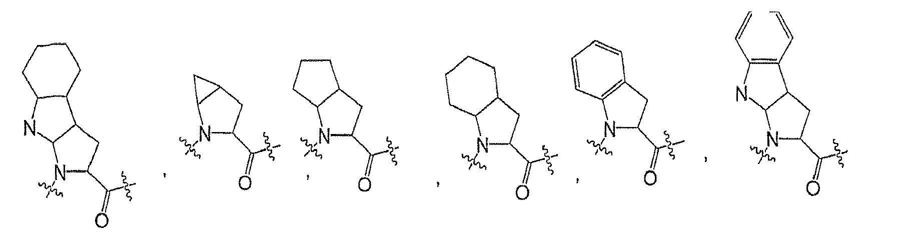

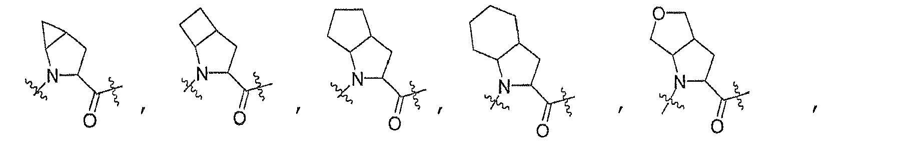

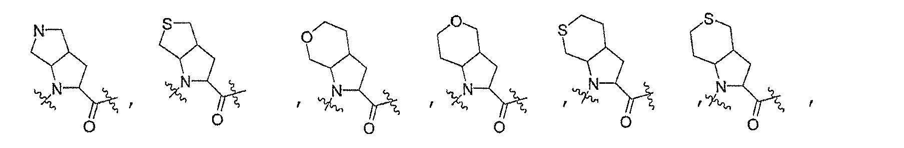

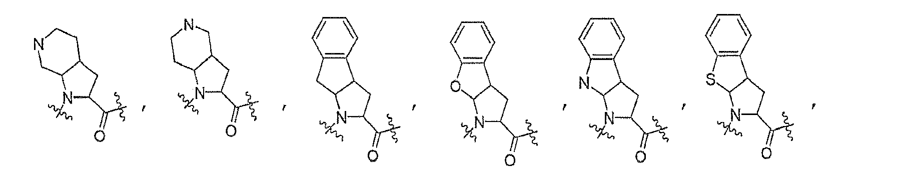

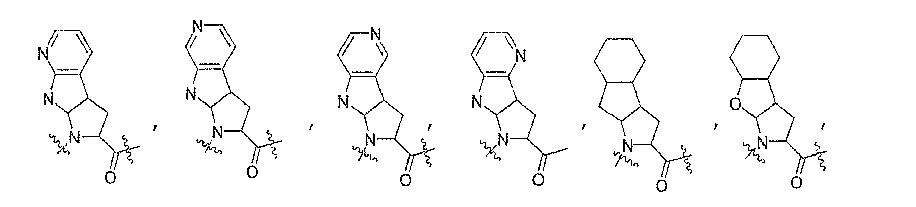

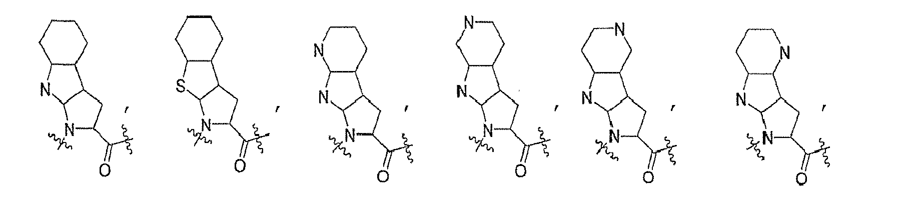

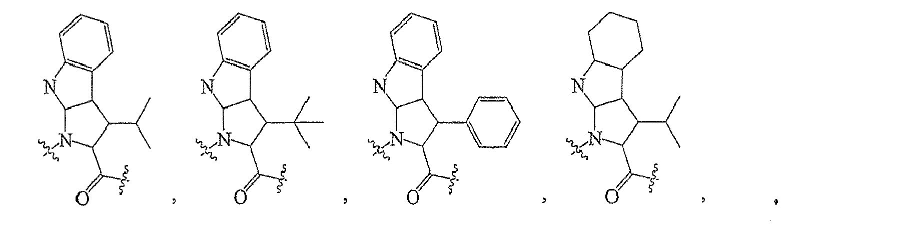

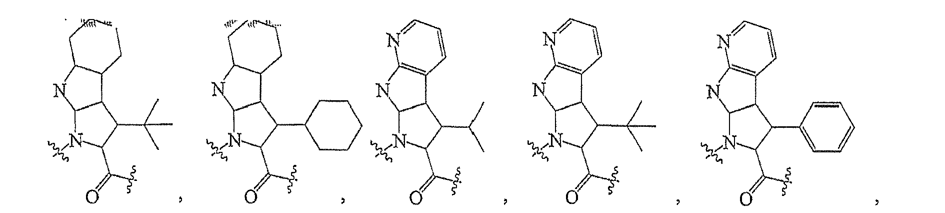

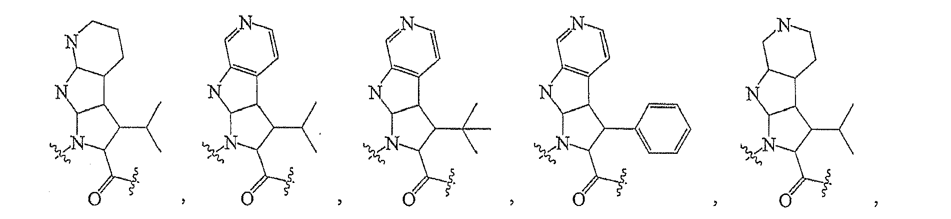

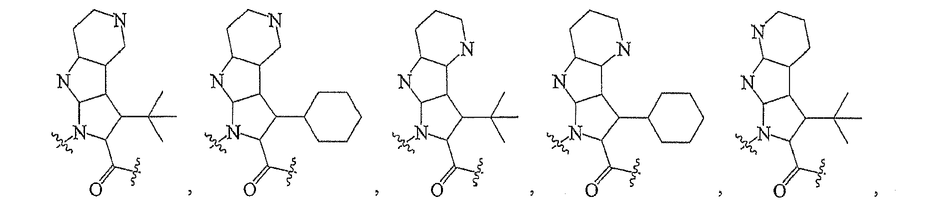

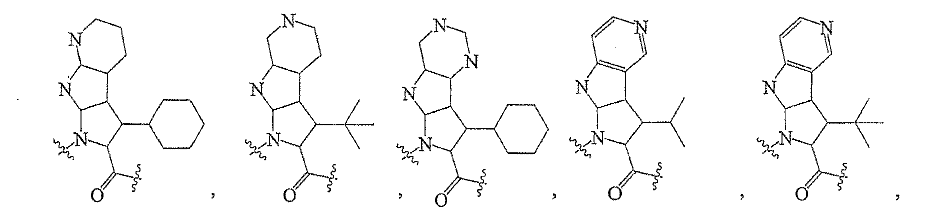

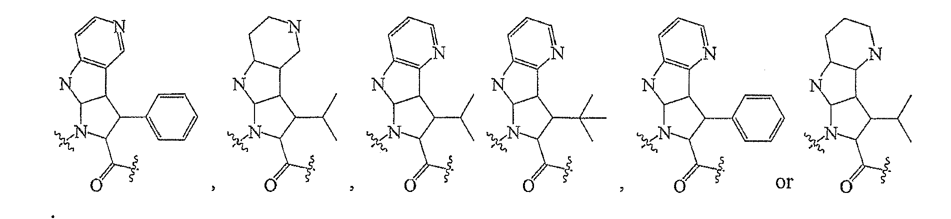

In several embodiments, J₁ and J₃ together with the atoms to which they are attached form an optionally substituted monocyclic ring such that the

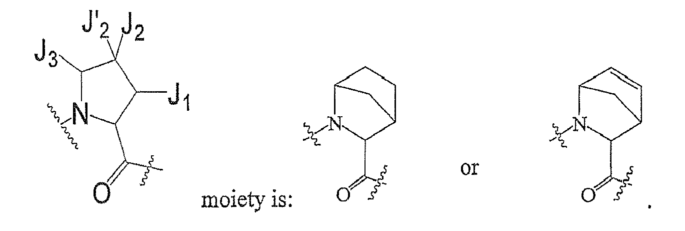

In several embodiments, a compound of formula I includes a structure wherein J₁ and J₂ together with the atoms to which they are attached form a monocyclic ring such that the

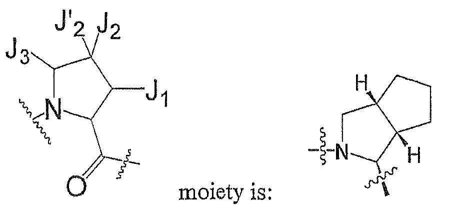

In several embodiments, L is -C(O)- .

In several embodiments, R₁ is RW-. For example, R₁ is RW-, wherein R is an optionally substituted aryl or an optionally substituted heteroaryl and W is -O-. In other embodiments, R is optionally substituted aliphatic or optionally substituted cycloaliphatic. For example, R is an optionally substituted aralkyl or an optionally substituted heteroaralkyl.

In several embodiments R₁ is

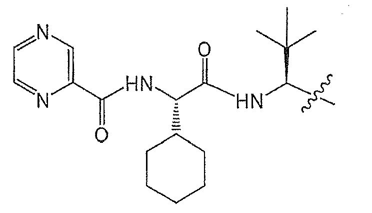

In several embodiments, R₁ is RW-.

In several embodiments, R is an optionally substituted aliphatic, an optionally substituted cycloaliphatic, an optionally substituted heterocycloaliphatic, an optionally substituted aryl, or an optionally substituted heteroaryl; and W is a bond, -O-, -S-, or -NR₄-.

In several embodiments, R is an optionally substituted aliphatic or an optionally substituted cycloaliphatic.

In several embodiments, R is an optionally substituted aralkyl or an optionally substituted heteroaralkyl.

In some embodiments R is

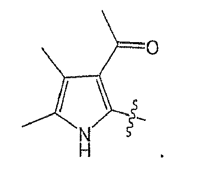

In other embodiments, R is 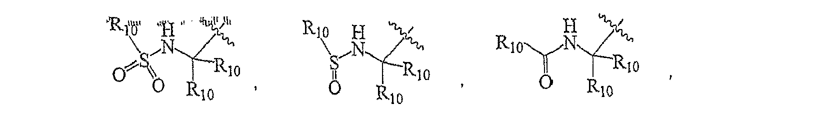 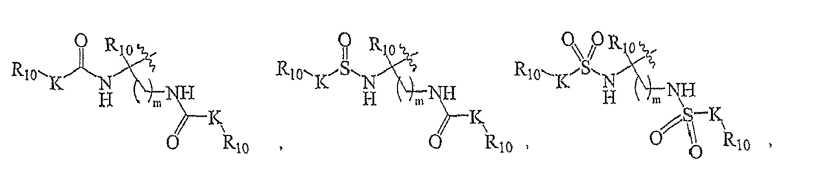 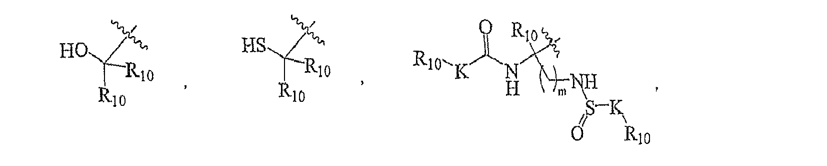 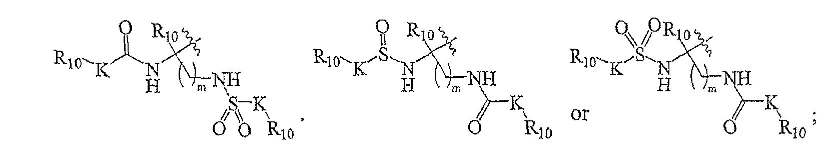 wherein R₁₀ is independently H, (C₁-C₁₂)-aliphatic, (C₆-C₁₀)-aryl, (C₆-C₁₀)-aryl-(C₁-C₁₂)aliphatic, (C₃-C₁₀)-cycloalkyl or -cycloalkenyl, [(C₃-C₁₀)-cycloalkyl or -cycloalkenyl]-(C₁-C₁₂)-aliphatic, (3 to 10 membered)-heterocycloaliphatic-, (6 to 10 membered)-heterocycloaliphatic-(C₁-C₁₂)aliphatic-, (5 to 10 membered)-heteroaryl-, or (5 to 10 membered)-heteroaryl-(C₁-C₁₂)-aliphatic-.

Each K is a bond, (C₁-C₁₂)-aliphatic, -O-, -S-, NR₉-, -C(O)-, or -C(O)NR₉-, wherein R₉ is hydrogen or (C₁-C₁₂)-aliphatic; and m is 1-3.

In some embodiments, R is:

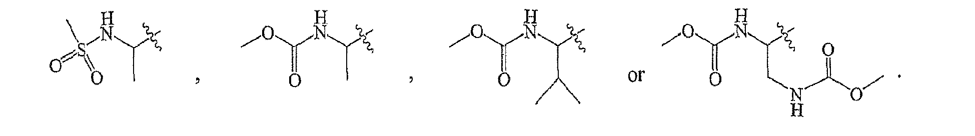

In further embodiments, R is: 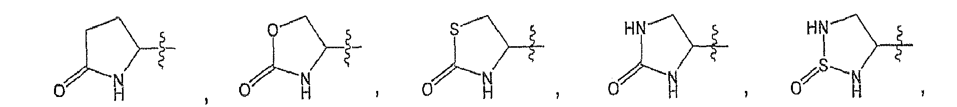 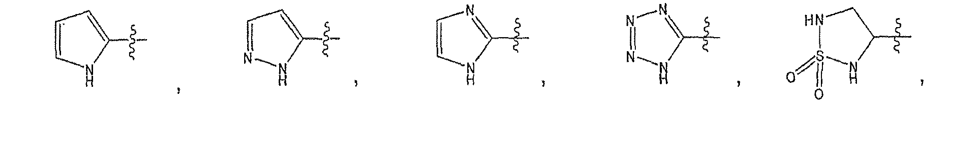 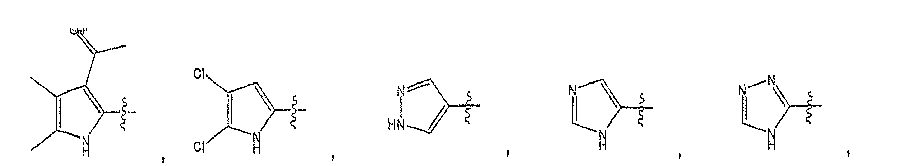 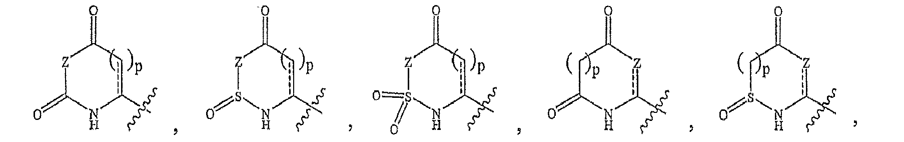 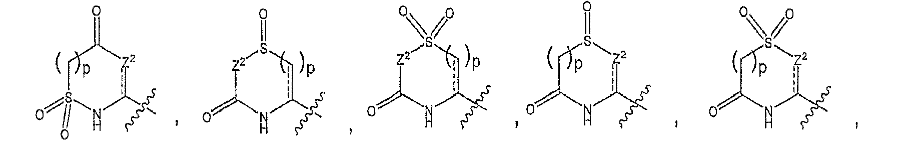 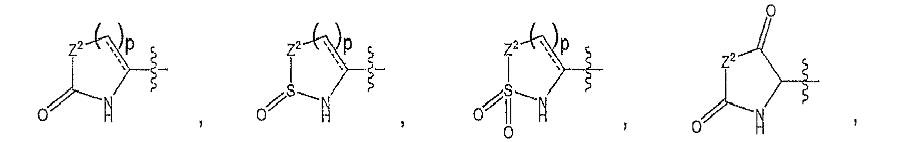 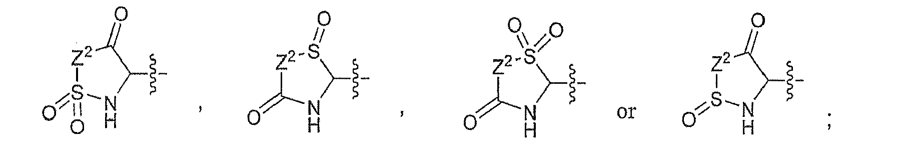 wherein each Z² is independently O, S, NR₁₀, or C(R₁₀)₂.

Each R₁₀ is hydrogen, (C₁-C₁₂)-aliphatic, (C₆-C₁₀)-aryl, (C₆-C₁₀)-aryl-(C₁-C₁₂)aliphatic, (C₃-₁₀)-cycloalkyl or -cycloalkenyl, [(C₃-C₁₀)-cycloalkyl or -cycloalkenyl]-(C₁-C₁₂)-aliphatic, (3 to 10 membered)-heterocycloaliphatic-, (6 to 10 membered)-heterocycloaliphatic-(C₁-C₁₂)aliphatic-, (5 to 10 membered)-heteroaryl-, or (5 to 10 membered)-heteroaryl-(C₁-C₁₂)-aliphatic-; p is independently 1 or 2; and is independently a single bond or a double bond.

In several embodiments, RW- is

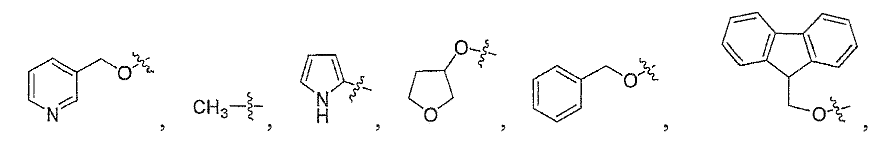

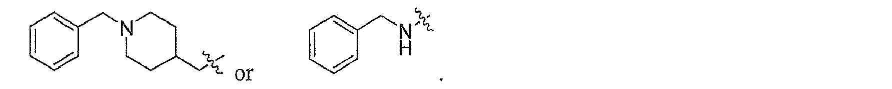

In several embodiments, R₁ is P₃, P₃ is 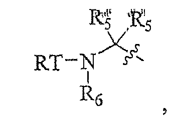 and each of R₅ and R'₅ is independently an optionally substituted aliphatic, an optionally substituted cycloaliphatic, an optionally substituted heterocycloaliphatic, an optionally substituted aryl, or an optionally substituted heteroaryl; R₆ is an optionally substituted aliphatic, an optionally substituted heteroaryl, an optionally substituted phenyl, or R₅ and R₆ together with the atoms to which they are attached form a 5- to 7- membered optionally substituted monocyclic heterocycle, or a 6- to 12- membered optionally substituted bicyclic heterocycle, in which each heterocycle ring optionally contains an additional heteroatom selected from -O-, -S- or -NR₅₀-; and T is -C(O)-, -OC(O)-, -NHC(O)-, - C(O)C(O)- or -SO₂-.

In several embodiments, T is -C(O)-.

In several embodiments, T is -OC(O)-.

In several embodiments, T is -NHC(O)-.

In several embodiments, T is -C(O)C(O)-.

In several embodiments, T is -S(O)₂-.

The mixture of diastereomeric compounds according to claim 6, wherein R₁ is P₄-L₂-P₃-; and P₄-L₂-P₃- is 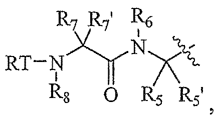 wherein each of R₇ and R₇' is independently H, an optionally substituted aliphatic, an optionally substituted heteroaryl, or an optionally substituted phenyl; or R₇ and R₇', together with the atom to which they are attached, may form a 3- to 7-membered cycloaliphatic or heterocycloaliphatic ring; or R₇ and R₆, together with the atoms to which they are attached, may form a 5- to 7-membered optionally substituted monocyclic heterocycloaliphatic, a 5- to 7-membered optionally substituted monocyclic heteroaryl, a 6- to 12-membered optionally substituted bicyclic heterocycloaliphatic, or a 6- to 12-membered optionally substituted bicyclic heteroaryl, in which each heterocycloaliphatic or heteroaryl ring optionally contains an additional heteroatom selected from -O-, -S- or -NR₅₀-; or when R₅ and R₆, together with the atoms to which they are attached, form a ring, R₇ and the ring system formed by R₅ and R₆ may form an 8- to 14-membered optionally substituted bicyclic fused ring system, wherein the bicyclic fused ring system is optionally further fused with an optionally substituted phenyl to form an optionally substituted 10- to 16-membered tricyclic fused ring system; R₈ is H or a protecting group; R₅₀ is H, an optionally substituted aliphatic, an optionally substituted heteroaryl, or an optionally substituted phenyl.

In several embodiments, R₇' is H; and R₇ is C₁-C₆ alkyl., C₃-C₁₀ cycloalkyl, C₃-C₁₀ cycloalkyl-C₁-₁₂ alkyl, C₆-C₁₀ aryl, C₆-C₁₀ aryl-C₁-C₆ alkyl, 3- to 10-membered heterocycloaliphatic, 6- to 10-membered heterocycloaliphatic-C₁-C₁₂ alkyl, 5- to 10-membered heteroaryl, or 5- to 10-membered heteroaryl-C₁-C₁₂ alkyl.

The mixture of diastereomeric compounds of claim 22, wherein R₇ is

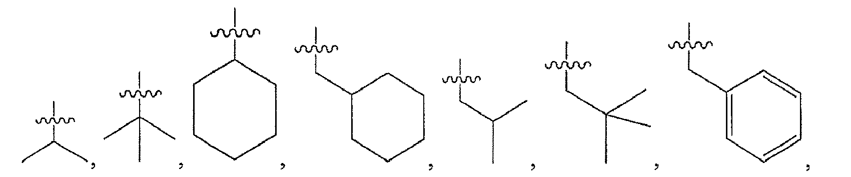

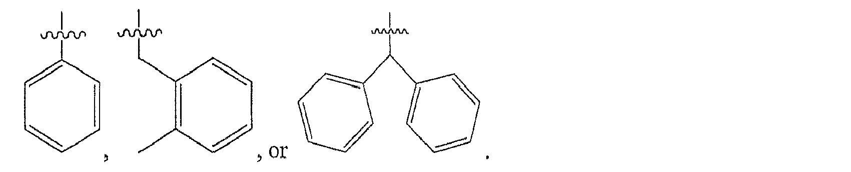

In several embodiments, R₇ and R₇', together with the atom to which they are attached, form a 3- to 7-membered optionally substituted cycloaliphatic ring.

In several embodiments, R₇ and R₇', together with the atom to which they are attached, form

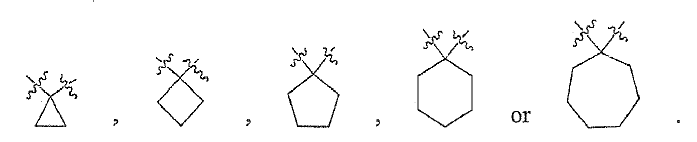

In some embodiments, R is

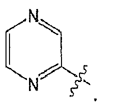

In several embodiments, a compound of formula I includes a structure wherein R₃ is optionally substituted aliphatic (e.g., optionally substituted (C₁-C₆)-alkyl), optionally substituted cycloaliphatic (e.g., optionally substituted (C₁-C₆)-cycloalkyl), optionally substituted heterocycloaliphatic, optionally substituted aryl, or optionally substituted heteroaryl.

In some embodiments, R₂ is an optionally substituted aliphatic, an optionally substituted phenyl, an optionally substituted cycloaliphatic, or an optionally substituted heterocycloaliphatic.

In some embodiments, R₂ is optionally substituted aliphatic or optionally substituted phenyl.

In other embodiments, R₂ is optionally substituted aliphatic, optionally substituted cycloaliphatic, or optionally substituted heterocycloaliphatic.

In some embodiments, R₂ is:

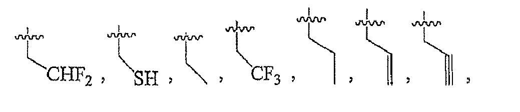

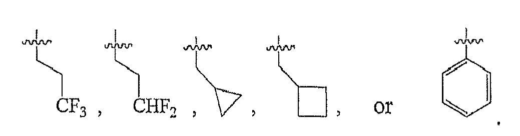

In several embodiments, R₂ is n-propyl.

In several embodiments, R₃ is optionally substituted (C₁-C₆)-alkyl or optionally substituted (C₁-C₆)-cycloalkyl.

In several embodiments, a compound of formula I includes a structure wherein R₂ is optionally substituted (C₁-C₆)-aliphatic (e.g., optionally substituted (C₁-C₆)-alkyl), optionally substituted (C₁-C₆)-cycloaliphatic (e.g., optionally substituted (C₁-C₆)-cycloalkyl), optionally substituted heterocycloaliphatic, optionally substituted aryl, or optionally substituted heteroaryl.

In some embodiments, R₃ is optionally substituted (C₁-C₇)-aliphatic, optionally substituted cycloaliphatic, optionally substituted aryl or optionally substituted heteroaryl.

In some embodiments, R₃ is:

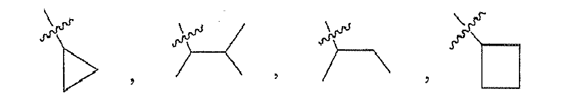

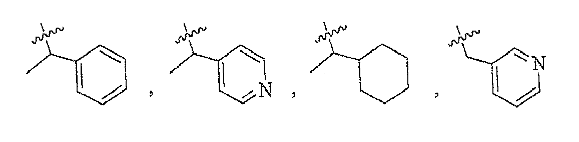

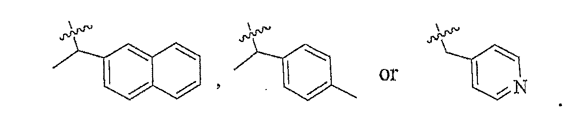

In several embodiments, R₃ is cyclopropyl.

In several embodiments, T is a bond and R is optionally substituted (haterocycloaliphatic)aliphatic. In other examples, T is a bond and R is an optionally substituted aryl or an optionally substituted heteroaryl.

In several embodiment, T is -C(O)- and R is optionally substituted heteroaryl, optionally substituted aryl.

In several embodiments, the compound of formula I includes

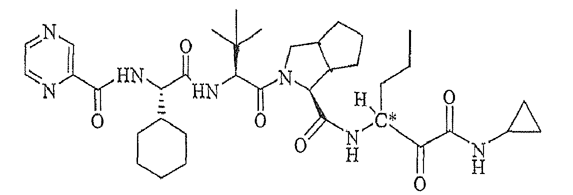

wherein C* represents a mixture of *R* and *S* isomers wherein the *R* isomer is at least 50% of the mixture.

In several embodiments, the percentage of the *R* isomer in the mixture is greater than 60%, (e.g., greater than 70 %, greater than 80 %, greater than 90 %, greater than 95 %, greater than 98 %, or greater than 99 %).

In several embodiments, the ratio of *R* to *S* isomers at C* is greater than 60 to 40.

In several embodiments, the ratio of *R* to *S* isomers at C* is greater than 70 to 30.

In several embodiments, the ratio of *R* to *S* isomers at C* is greater than 80 to 20.

In several embodiments, the ratio of *R* to *S* isomers at C* is greater than 90 to 10.

In several embodiments, the ratio of *R* to *S* isomers at C* is greater than 95 to 5.

In several embodiments, the ratio of *R* to *S* isomers at C* is greater than 98 to 2.

In several embodiments, the ratio of *R* to *S* isomers at C* is greater than 99 to 1.

The invention is intended to include compounds wherein R₁ and R₂ contain structural elements of a serine protease inhibitor. Compounds having the structural elements of a serine protease inhibitor include, but are not limited to, the compounds of the following publications: WO 97/43310, US 20020016294, WO 01/81325, WO 02/08198, WO 01/77113, WO 02/08187, WO 02/08256, WO 02/08244, WO 03/006490, WO 01/74768, WO 99/50230, WO 98/17679, WO 02/48157, US 20020177725, WO 02/060926, US 20030008828, WO 02/48116, WO 01/64678, WO 01/07407, WO 98/46630, WO 00/59929, WO 99/07733, WO 00/09588, US 20020016442, WO 00/09543, WO 99/07734, US 6,018,020, US 6,265,380, US 6,608,027, US 20020032175, US 20050080017, WO 98/22496, US 5,866,684, WO 02/079234, WO 00/31129, WO 99/38888, WO 99/64442, WO 2004072243, and WO 02/18369, which are incorporated herein by reference.

Non-limiting examples of the compounds of the invention include: (1*S*,3a*R*,6a*S*)-2-[(2*S*)-2-[[(2*S*)-2-cyclohexyl-1-oxo-2-[(pyrazinylcarbonyl)amino]ethyl]amino]-3,3-dimethyl-1-oxobutyl]-N-[(1*R*)-1-[2-(cyclopropylamino)-1,2-dioxoethyl]butyl]octahydro-cyclopenta[c]pyrrole-1-carboxamide.

In several embodiments of the present invention, the *R* isomer at the C* position is greater than 50% of the mixture, and the mixture has a Ki(app) of less than 1.5 µM when determined using a two-day (48 hour) HCV replicon incubation assay, as described herein.

In several embodiments, the *R* isomer at the C* position is greater than 50% of the mixture, and the mixture has an IC₅₀ of less than 1 µM and a CC₅₀ of more than 90 µM when determined using a two-day (48 hour) HCV replicon incubation assay.

In several embodiments, the *R* isomer at the C* position is greater than 50% of the mixture and the mixture includes a Ki(app) of about 1.190 µM, an IC₅₀ of about 0.883 µM, and a CC₅₀ of greater than 100 µM determined using a two-day (48 hour) HCV replicon incubation assay.

In several embodiments, mixtures containing greater than 50% *R* isomer at the C* position have a higher bioavailability than mixtures with 50% or less *R* isomer at the C* position.

In several embodiments, the *R* isomer at the C* position is greater than 50% of the mixture, and the mixture has a bioavailability of greater than 90 %.

In several embodiments, the *R* isomer at the C* position is about 2 times as bioavailable as the *S* isomer at the C* position.

In several embodiments, the *R* isomer at the C* position is greater than 50% of the mixture, and the mixture is more readily absorbed than a mixture including 50% or less of the *R* isomer at the C* position.

In several embodiments, the *R* isomer at the C* position is greater than 50% of the mixture, and the mixture has a longer half-life than mixtures with 50% or less *R* isomer at the C* position.

**III. SYNTHETIC SCHEMES**

The compounds of the invention can be prepared by known methods. An example, of such methods is illustrated in Scheme 1.

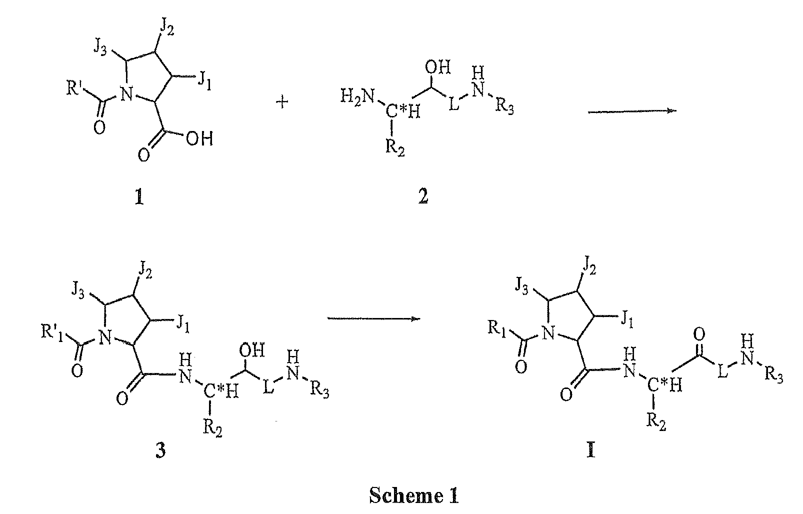

Referring to Scheme **1**, a pyrrolidine acid of formula **1** is reacted with an amino-alcohol of formula **2** in the presence of a coupling reagent such as, e.g., EDC and HOBt to give the corresponding amide of formula **3**. Oxidation of amide **3** provides the compounds of Formula I. Suitable oxidizing agents include, e.g., Dess-Martin periodane and sodium hypochlorite in the presence of TEMPO. The pyrrolidine acids of formula **1** used in Scheme 1 may be prepared by methods described in WO 03/006490 and WO 02/18369. Aminoalcohols of formula 2 wherein L is -C(O)- may be prepared by methods described in WO 02/18369. In Formula **I,** R'₁ represents an N-protecting group that may be removed for further elaboration of R'₁ according to known methods. Alternatively, R'₁ represents R₁. Thus, the attachment of the amino-alcohol of formula **2** can be achieved before or after elaboration of the R₁ moiety.

The mixtures of *R* and *S* isomers, at position C*, of the present invention can be processed by known techniques. One example of such techniques includes diluting a pure *R* or *S* isomer (at position C*) with an appropriate amount of *S* or *R* isomer, respectively. Another example of such techniques includes diluting a mixture of a known *R* to *S* (at position C*) ratio with an appropriate volume of pure *R* isomer at C*, a volume of pure *S* isomer at C*, or a volume of a mixture of a known *R* and *S* isomer (at C*) ratio. Another technique includes conducting a synthetic pathway that provides a desired ratio of *R* isomer at C* to *S* isomer at C*.

Preparation of the pure *R* isomer or *S* isomer at C* is described in WO 02/18369 and is illustrated in Scheme 2.

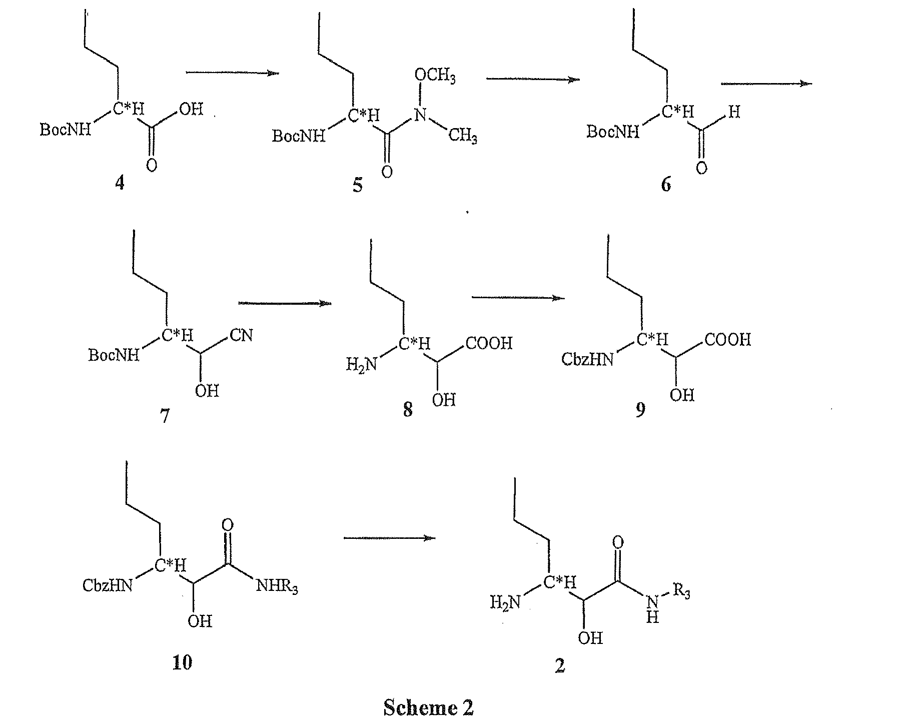

Referring to Scheme 2, an optical isomer of Boc-norvaline of formula **4** is first converted to the corresponding N-methyl(methoxy) amide of formula **5** by reacting with dimethylhydroxylamine in the presence of CDI. Reduction of the amide **5** with lithium aluminum hydride provides the norvalinal of formula **6**. The cyanohydrin of formula **7** is achieved by conversion of the norvalinal **6** to the bisulfite addition complex (not shown) followed by a reaction with potassium cyanide. Reaction of the cyanohydrin of formula **7** with concentrated hydrochloric acid results in hydrolysis of the cyano group and deprotection to give the amino-hydroxy acid of formula **8**. Reaction of the resultant amino-hydroxy acid **8** with N-(benzyloxycarbonyloxy)succinimide gives the Cbz derivative of formula **9**, which can be further converted to an amide of formula **10** by reaction with the amine R₃NH₂ in the presence of the coupling reagent PyBOP and FIOBT. Hydrogenation of amide **10** with 10% Pd/C gives the amine of formula **2**.

Mixtures of compounds of Formula I can be optionally separated into its constituent stereoisomers, e.g., by chromatographic separation procedures. See Tables 1 and 2.

**Table 1. Example Separation of R and S isomers at C* of compounds of formula I.**

| **Parameter** | **Value Parameter** |
|---|---|
| Analyte | Mixtures of *R* and *S* Isomers of compounds of Formula I |
| Analytical Instrumentation | PE SCIEX API 3000 |
| Type of Detection | MS/MS |
| Chromatography | Normal Phase |
| Column Type | ChiralPak AD |
| | L: 250 mm |
| Column Dimensions | D: 4.6 mm |
| | Particle size: 5 µm |
| Flow Rate | 1.3 mL/minute |
| Run Time | 16.0 minutes |
| Injection Volume | 20 µL |
| Mobile Phase | 20% isopropyl alcohol |
| | 80% hexane |
| Extraction Procedure | liquid/liquid |

A specific separation of a mixture containing R and S isomers of compounds of the formula 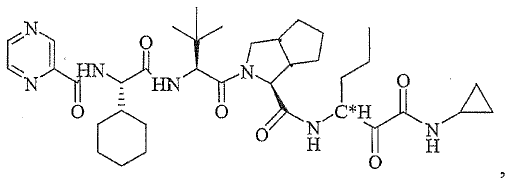 in which C* represents a mixture of *R* and *S* isomers, was performed according to Table 1. The retention times were measured to be 8 minutes 27 seconds for the *R* isomer at C*, and 6 minutes 35 seconds for the *S* isomer at C*, as illustrated in Table 2.

**Table 2. Separation of R and S isomers at position C* of compounds of formula I by HPLC**

| **HPLC Conditions** | |
|---|---|
| Eluent (isocratic) | 80:19:1 heptane:acetone:methanol |
| Flow Rate | 750 µL/min |
| Make-up Solution | 40:60:1:1 acetonitrile:acetone:methanol:formic acid |
| Flow Rate | 250 µL/minute |
| Autosampler | LEAP HTS PAL with cooling unit |
| Autosampler Temp | 2° C |
| Autosampler Needle Wash | 85:15 heptane:acetone |
| Injection Vol. | 100 µL |
| HPLC Column | Hypersil CPS-1, 250 mm × 2 mm, 5- µm. particle size |
| HPLC Column Temp | about -1 °C |
| Typical Initial Column Pressure | 97 bar |
| Autosampler Run Time | 7.75 minutes |
| Autosampler Needle Wash Program | PreClnSlv1 1 |
| | PreClnSlv1 0 |
| | PreClnSlv1 5 |
| | PreClnSlv1 0 |
| | PreClnSlv1 5 |
| | PreClnSlv1 0 |

Using the separation methods illustrated in Table 2, a retention time of 3.6 minutes was measured for the *R* isomer at the C* position and a retention time of 4.0 minutes was measured for the *S* isomer at the C* position.

IV. **FORMULATIONS, USES, AND ADMINISTRATIONS**

Compounds of the present invention can be desirable therapeutic agents because they were observed to have a greater bioavailability when the *R* isomer at position C* was greater than 50% of the mixture (e.g., about 60%, about 70%, about 80%, about 85%, about 90%, about 95%, or about 99%). Mixtures with greater than 50% *R* isomer at the C* position were about 2 times more bioavailable than the *S* isomer at the C* position. Specifically, the total bioavailability was about 98% for the orally dosed *R* isomer at C* and 50% for the orally dosed *S* isomer at C , when represented by the combined exposure of the 2 isomers. Furthermore, following oral dosing, the *R* isomer at C* to *S* isomer at C* conversion was more prominent than the *S* isomer at C* to *R* isomer at C* conversion. Interconversion occurred to a larger extent after an oral dose when compared with that after an IV dose.

Compounds of the present invention can be useful therapeutic treatments for HCV infection because these compounds inhibit serine protease activity, particularly the activity of hepatitis C virus NS3-NS4A protease. Some embodiments of the present invention that were greater than 50% *R* isomer at C* had measured Ki(app)'s of less than 3 µM (e.g., about 2 µM, about 1.5 µM, or about 1.190 µM), IC50's of less than about 0.9 µM (e.g., about 0.883 µM), and a CC50 of greater than 100 µM.

The invention includes a methods of administering mixtures of compounds of formula (I) for treating HCV in which the mixture contains greater than 50% of the *R* isomer at C* position.

One embodiment of this invention provides a pharmaceutical composition comprising a compound of formula I, or pharmaceutically acceptable salts or mixtures of salts thereof. According to another embodiment, the compound of formula I is present in an amount effective to decrease the viral load in a sample or in a patient, wherein said virus encodes a serine protease necessary for the viral life cycle, and a pharmaceutically acceptable carrier.

If pharmaceutically acceptable salts of the compounds of this invention are utilized in these compositions, those salts are preferably derived from inorganic or organic acids and bases. Included among such acid salts are the following: acetate, adipate, alginate, aspartate, benzoate, benzene sulfonate, bisulfate, butyrate, citrate, camphorate, camphor sulfonate, cyclopentane-propionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2 hydroxyethanesulfonate, lactate, maleate, methanesulfonate, 2 naphthalenesulfonate, nicotinate, oxalate, pamoate, pectinate, persulfate, 3 phenyl propionate, picrate, pivalate, propionate, succinate, tartrate, thiocyanate, tosylate and undecanoate. Base salts include ammonium salts, alkali metal salts, such as sodium and potassium salts, alkaline earth metal salts, such as calcium and magnesium salts, salts with organic bases, such as dicyclohexylamine salts, N methyl D glucamine, and salts with amino acids such as arginine, lysine, and so forth.

Also, the basic nitrogen containing groups may be quatemized with such agents as lower alkyl halides, such as methyl, ethyl, propyl, and butyl chloride, bromides and iodides; dialkyl sulfates, such as dimethyl, diethyl, dibutyl and diamyl sulfates, long chain halides such as decyl, lauryl, myristyl and stearyl chlorides, bromides and iodides, aralkyl halides, such as benzyl and phenethyl bromides and others. Water or oil soluble or dispersible products are thereby obtained.

The compounds utilized in the compositions and methods of this invention may also be modified by appending appropriate functionalities to enhance selective biological properties. Such modifications are known in the art and include those which increase biological penetration into a given biological system (e.g., blood, lymphatic system, central nervous system), increase oral availability, increase solubility to allow administration by injection, alter metabolism and alter rate of excretion.

Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose based substances, polyethylene glycol, sodium carboxymethylcellulose, polyacrylates, waxes, polyethylene polyoxypropylene block polymers, polyethylene glycol and wool fat.

According to another embodiment, the compositions of this invention are formulated for pharmaceutical administration to a mammal. In one embodiment said mammal is a human being.

Such pharmaceutical compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra articular, intra synovial, intrasternal, intrathecal, intrahepatic, intralesional and intracranial injection or infusion techniques. Preferably, the compositions are administered orally or intravenously.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non toxic parenterally acceptable diluent or solvent, for example as a solution in 1,3 butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono or di glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

In one embodiment, dosage levels of between about 0.01 and about 100 mg/kg body weight per day of the protease inhibitor compounds described herein are useful in a monotherapy for the prevention and treatment of antiviral, particularly anti-HCV mediated disease. In another embodiment, dosage levels of between about 0.5 and about 75 mg/kg body weight per day of the protease inhibitor compounds described herein are useful in a monotherapy for the prevention and treatment of antiviral, particularly anti-HCV mediated disease. Typically, the pharmaceutical compositions of this invention will be administered from about 1 to about 5 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (w/w). In one embodiment, such preparations contain from about 20% to about 80% active compound.

When the compositions of this invention comprise a combination of a compound of formula I and one or more additional therapeutic or prophylactic agents, both the compound and the additional agent should be present at dosage levels of between about 10 to 100% of the dosage normally administered in a monotherapy regimen. In another embodiment, the additional agent should be present at dosage levels of between about 10 to 80% of the dosage normally administered in a monotherapy regimen. The pharmaceutical compositions of this invention may be orally administered in any orally acceptable dosage form including, but not limited to, capsules, tablets, aqueous suspensions or solutions. In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutical compositions of this invention may be administered in the form of suppositories for rectal administration. These may be prepared by mixing the agent with a suitable non irritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract may be effected in a rectal suppository formulation (see above) or in a suitable enema formulation. Topically transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions may be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2 octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

In one embodiment, the pharmaceutical compositions are formulated for oral administration.

In another embodiment, the compositions of this invention additionally comprise another anti-viral agent, preferably an anti-HCV agent. Such anti-viral agents include, but are not limited to, immunomodulatory agents, such as α-, β-, and γ- interferons, pegylated derivatized interferon α compounds, and thymosin; other anti-viral agents, such as ribavirin, amantadine, and telbivudine; other inhibitors of hepatitis C proteases (NS2-NS3 inhibitors and NS3-NS4A inhibitors); inhibitors of other targets in the HCV life cycle, including helicase and polymerase inhibitors; inhibitors of internal ribosome entry; broad-spectrum viral inhibitors, such as IMPDH inhibitors (e.g., compounds of United States Patent 5,807,876,6,498,178,6,344,465,6,054,472, WO 97/40028, WO 98/40381, WO 00/56331, and mycophenolic acid and derivatives thereof, and including, but not limited to COMPOUND XX; or any combination of any of the above. See also W. Markland et al., Antimicrobial & Antiviral Chemotherapy, 44, p. 859 (2000) and U.S. Patent 6,541,496.

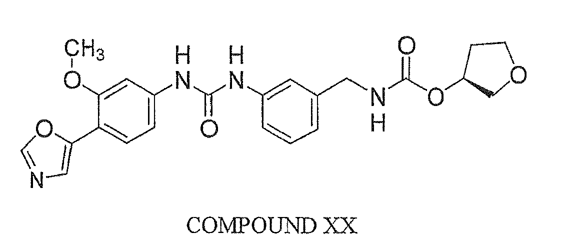

The following definitions are used herein (with trademarks referring to products available as of this application's filing date).

"Peg-Intron" means PEG-INTRON®, peginteferon alpha-2b, available from Schering Corporation, Kenilworth, NJ;

"Intron" means INTRON-A®, interferon alpha-2b available from Schering Corporation, Kenilworth, NJ;

"ribavirin" means ribavirin (1-beta-D-ribofuranosyl-1H-1,2,4-triazole-3-carboxamide, available from ICN Pharmaceuticals, Inc., Costa Mesa, CA; described in the Merck Index, entry 8365, Twelfth Edition; also available as REBETROL® from Schering Corporation, Kenilworth, NJ, or as COPEGASUS® from Hoffmann-La Roche, Nutley, NJ;

Pagasys means PEGASYS® peginterferon alfa-2a available Hoffmann-La Roche, Nutley, NJ;

"Roferon" mean ROFERON®, recombinant interferon alfa-2a available from Hoffmann-La Roche, Nutley, NJ;

"Berefor" means BEREFOR®, interferon alpha 2 available from Boehringer Ingelheim Pharmaceutical, Inc., Ridgefield, CT;

SUMIFERON®, a purified blend of natural alpha interferons such as Sumiferon available from Sumitomo, Japan;

WELLFERON®, interferon alpha n1 available from Glaxo Wellcome LTd., Great Britain;

ALFERON®, a mixture of natural alpha interferons made by Interferon Sciences, and available from Purdue Frederick Co., CT;

The term "interferon" as used herein means a member of a family of highly homologous species-specific proteins that inhibit viral replication and cellular proliferation, and modulate immune response, such as interferon alpha, interferon beta, or interferon gamma. The Merck Index, entry 5015, Twelfth Edition.

According to one embodiment of the present invention, the interferon is α-interferon. According to another embodiment, a therapeutic combination of the present invention utilizes natural alpha interferon 2a. Or, the therapeutic combination of the present invention utilizes natural alpha interferon 2b. In another embodiment, the therapeutic combination of the present invention utilizes recombinant alpha interferon 2a or 2b. In yet another embodiment, the interferon is pegylated alpha interferon 2a or 2b. Interferons suitable for the present invention include:

(a) INTRON-AV (interferon-alpha 2B, Schering Plough),

(b) PEG-INTRON®,

(c) PEGASYS®,

(d) ROFERONV,

(e) BEREFOR®,

(f) SUMIFERON®,

(g) WELLFERON®,

(h) consensus alpha interferon available from Amgen, Inc., Newbury Park, CA,

(i) ALFERON®;

(j) VIRAFERON®;

(k) INFERGEN®; and

(1') ALBUFERONTM.

As is recognized by skilled practitioners, a protease inhibitor would be preferably administered orally. Interferon is not typically administered orally. Nevertheless, nothing herein limits the methods or combinations of this invention to any specific dosage forms or regime. Thus, each component of a combination according to this invention may be administered separately, together, or in any combination thereof.

In one embodiment, the protease inhibitor and interferon are administered in separate dosage forms. In one embodiment, any additional agent is administered as part of a single dosage form with the protease inhibitor or as a separate dosage form. As this invention involves a combination of compounds, the specific amounts of each compound may be dependent on the specific amounts of each other compound in the combination. As recognized by skilled practitioners, dosages of interferon are typically measured in IU (e.g., about 4 million IU to about 12 million IU).

Accordingly, agents (whether acting as an immunomodulatory agent or otherwise) that may be used in combination with a compound of this invention include, but are not limited to, Albuferon™ (albumin-Interferon alpha) available from Human Genome Sciences; interferon-alpha 2B (INTRON-A®, Schering Plough); REBETRON® (Schering Plough, Inteferon-alpha 2B + Ribavirin); pegylated interferon alpha (Reddy, K.R. et al. "Efficacy and Safety of Pegylated (40-kd) interferon alpha-2a compared with interferon alpha-2a in noncrrhotic patients with chronic hepatitis C (Hepatology, 33, pp. 433-438 (2001); consensus interferon (Kao, J.H., et al., "Efficacy of Consensus Interferon in the Treatment of Chronic Hepatitis" J. Gastroenterol. Hepatol. 15, pp. 1418-1423 (2000), interferon-alpha 2A (Roferon A; Roche), lymphoblastoid or "natural"' interferon; interferon tau (Clayette, P. et al., "IFN-tau, A New Interferon Type I with Antiretroviral activity" Pathol. Biol. (Paris) 47, pp. 553-559 (1999); interleukin 2 (Davis, G.L. et al., "Future Options for the Management of Hepatitis C." Seminars in Liver Disease, 19, pp. 103-112 (1999); Interleukin 6 (Davis et al. "Future Options for the Management of Hepatitis C." Seminars in Liver Disease 19, pp. 103-112 (1999); interleukin 12 (Davis, G.L. et al., "Future Options for the Management of Hepatitis C." Seminars in Liver Disease, 19, pp. 103-112 (1999); Ribavirin; and compounds that enhance the development of type 1 helper T cell response (Davis et al., "Future Options for the Management of Hepatitis C." Seminars in Liver Disease, 19, pp. 103-112 (1999). Interferons may ameliorate viral infections by exerting direct antiviral effects and/or by modifying the immune response to infection. The antiviral effects of interferons are often mediated through inhibition of viral penetration or uncoating, synthesis of viral RNA, translation of viral proteins, and/or viral assembly and release.

Compounds that stimulate the synthesis of interferon in cells (Tazulakhova, E.B. et al., "Russian Experience in Screening, analysis, and Clinical Application of Novel Interferon Inducers" J. Interferon Cytokine Res., 21 pp. 65-73) include, but are not limited to, double stranded RNA, alone or in combination with tobramycin, and Imiquimod (3M Pharmaceuticals; Sauder, D.N. "Immunomodulatory and Pharmacologic Properties of Imiquimod" J. Am. Acad. Dermatol., 43 pp. S6-11 (2000).

Other non-immuuomodulatory or immunomodulatory compounds may be used in combination with a compound of this invention including, but not limited to, those specified in WO 02/18369, which is incorporated herein by reference (see, e.g., page 273, lines 9-22 and page 274, line 4 to page 276, line 11).

This invention may also involve administering a cytochrome P450 monooxygenase inhibitor. CYP inhibitors may be useful in increasing liver concentrations and/or increasing blood levels of compounds that are inhibited by CYP.

If an embodiment of this invention involves a CYP inhibitor, any CYP inhibitor that improves the pharmacokinetics of the relevant NS3/4A protease may be used in a method of this invention. These CYP inhibitors include, but are not limited to, ritonavir (WO 94/14436), ketoconazole, troleandomycin, 4-methyl pyrazole, cyclosporin, clomethiazole, cimetidine, itraconazole, fluconazole, miconazole, fluvoxamine, fluoxetine, nefazodone, sertraline, indinavir, nelfinavir, amprenavir, fosamprenavir, saquinavir, lopinavir, delavirdine, erythromycin, VX-944, and COMPOUND XX. Preferred CYP inhibitors include ritonavir, ketoconazole, troleandomycin, 4-methyl pyrazole, cyclosporin, and clomethiazole. For preferred dosage forms of ritonavir, see United States Patent 6,037, 157, and the documents cited therein: United States Patent 5,484,801, United States Application 08/402,690, and International Applications WO 95/07696 and WO 95/09614).

Methods for measuring the ability of a compound to inhibit cytochrome P450 monooxygenase activity are known (see US 6,037,157 and Yun, et al. Drug Metabolism & Disposition, vol. 21, pp. 403-407 (1993).

Upon improvement of a patient's condition, a maintenance dose of a compound, composition or combination of this invention may be administered, if necessary. Subsequently, the dosage or frequency of administration, or both, may be reduced, as a function of the symptoms, to a level at which the improved condition is retained when the symptoms have been alleviated to the desired level, treatment should cease. Patients may, however, require intermittent treatment on a long-term basis upon any recurrence of disease symptoms.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredients will also depend upon the particular described compound and the presence or absence and the nature of the additional anti-viral agent in the composition.

According to another embodiment, the invention provides a method for treating a patient infected with a virus characterized by a virally encoded serine protease that is necessary for the life cycle of the virus by administering to said patient a pharmaceutically acceptable composition of this invention. In one embodiment, the methods of this invention are used to treat a patient suffering from a HCV infection. Such treatment may completely eradicate the viral infection or reduce the severity thereof. In another embodiment, the patient is a human being.

In an alternate embodiment, the methods of this invention additionally comprise the step of administering to said patient an anti-viral agent preferably an anti-HCV agent. Such anti-viral agents include, but are not limited to, immunomodulatory agents, such as α-, β- or γ- interferons, pegylated derivatized interferon-α compounds, and thymosin; other anti-viral agents, such as ribavirin, amantadine, and telbivudine; other inhibitors of hepatitis C proteases (NS2-NS3 inhibitors and NS3-NS4A inhibitors); inhibitors of other targets in the HCV life cycle, including but not limited to helicase and polymerase inhibitors; inhibitors of internal ribosome entry; broad-spectrum viral inhibitors, such as IMPDH inhibitors (e.g., COMPOUND XX and other IMPDH inhibitors disclosed in United States Patents 5,807,876 and 6,498,178, mycophenolic acid and derivatives thereof); inhibitors of cytochrome P-450, such as ritonavir, or combinations of any of the above.

Additional agents can be administered to the patient as part of a single dosage form comprising both a compound of this invention and an additional anti-viral agent. Alternatively the additional agent may be administered separately from the compound of this invention, as part of a multiple dosage form, wherein said additional agent is administered prior to, together with or following a composition comprising a compound of this invention.

In yet another embodiment the present invention provides a method of pre-treating a biological substance intended for administration to a patient comprising the step of contacting Said biological substance with a pharmaceutically acceptable composition comprising a compound of this invention. Such biological substances include, but are not limited to, blood and components thereof such as plasma, platelets, subpopulations of blood cells and the like; organs such as kidney, liver, heart, lung, etc; sperm and ova; bone marrow and components thereof, and other fluids to be infused into a patient such as saline, dextrose, etc.

According to another embodiment the invention provides methods of treating materials that may potentially come into contact with a virus characterized by a virally encoded serine protease necessary for its life cycle. This method comprises the step of contacting said material with a compound according to the invention. Such materials include, but are not limited to, surgical instruments and garments (e.g. clothes, gloves, aprons, gowns, masks, eyeglasses, footwear, etc.); laboratory instruments and garments (e.g. clothes, gloves, aprons, gowns, masks, eyeglasses, footwear, etc.); blood collection apparatuses and materials; and invasive devices, such as, for example, shunts and stents.

In another embodiment, the compounds of this invention may be used as laboratory tools to aid in the isolation of a virally encoded serine protease. This method comprises the steps of providing a compound of this invention attached to a solid support; contacting said solid support with a sample containing a viral serine protease under conditions that cause said protease to bind to said solid support; and eluting said serine protease from said solid support. In one embodiment, the viral serine protease isolated by this method is HCV NS3-NS4A protease.

V. ASSAYS

**Example 1: Bioavailability of *R* and *S* isomers at position C* of a compound of formula I**

Three groups of male Sprague Dawley rats (n=6-7/group) were orally administered a compound of formula I wherein the mixture comprised about 92 % *R* isomer at position C* and about 8 % *S* isomer at position C*; a mixture that was about 7 % *R* isomer at position C* and about 93 % *S* isomer at position C*; or a compound of formula I wherein the mixture was about 54% *R* isomer at position C* and about 46% *S* isomer at position C*; at a nominal dose of 30 mg/kg. Serial blood samples were collected up to 24 hours (hr) post dose. Derived plasma samples (100 µL) were acidified by the addition of 5 µL of formic acid to prevent in vitro interconversion.

The determination of concentrations of both the *R* isomer and the *S* isomer, at position C*, in plasma and in dose solutions was conducted using a chiral liquid chromatography/mass spectrometry (LC/MS/MS, e.g., LC/tandem mass spectroscopy) method. Compounds for oral administration to rats include those listed in Table 3.

**Table 3 Compounds for Oral Administration to Rats**

| Diastereoisomer | | Mixture Number | *R* isomer at C* (%) | *S* isomer at C* (%) |
|---|---|---|---|---|
| *S* isomer at position C*: (1S,3aR,6aS)-2-[(2S)-2-[[(2S)-2-cyclohexyl-1-oxo-2-[(pyrazinylcarbonyl)amino]ethyl]amino ]-3,3-dimethyl-1-oxobutyl]-N-[(1S)-1-[2-(cyclopropylamino)-1,2-dioxoethyl]butyl]octahydro-cyclopenta[c]pyrrole-1-carboxamide | *S* isomer | 1 | 92 | 8 |
| *R* isomer at position C*: (1 S,3aR,6aS)- 2-[(2S)-2-[[(2S)-2-cyclohexyl-1-oxo-2-[(pyrazinylcarbonyl)amino]ethyl]amino ]-3,3-dimethyl-1-oxobutyl]-N-[(1R)-1-[2-(cyclopropylamino)-1,2- dioxoethyl]butyl]octahydro-cyclopenta[c]pyrrole-1-carboxamide | *R* isomer | 2 | 7 | 93 |
| A mixture of nominal ratio of 60% *S* isomer and 40% *R* isomer (at position C*) | R:*S* mixture | 3 | 54 | 46 |

The formulations of the solid dispersions containing either 10% of the *R*:*S* mixture, the *R* isomer, or the *S* isomer were prepared according to Table 4.

**Table 4 Compositions of Solid Dispersion Formulations**

| Formulation | 1 | 2 | 3 |
|---|---|---|---|
| Component | *S* isomer at C* | *R* isomer at C* | *R:S* at C* mixture |
| Active ingredient (mg) | 100 | 100 | 100 |
| Sodium lauryl sulfate (mg) | 30 | 30 | 30 |
| PVP K30^{a} (mg) | 967 | 967 | 967 |
| Ethanol (mL) | 10 | 10 | 10 |
| Total weight (gm) of dry powder | 1 | 1 | 1 |

| | | | |
|---|---|---|---|
| ^{a}PVP contained 10% water. Weights corrected for water. | | | |

For each preparation, the active ingredient was dissolved in the total volume of absolute ethanol in round bottom evaporation flask, then heated at 40° C and sonicated for approximately 5 to 6 minutes (min) until dissolved. The sodium lauryl sulfate and PVP were added to the flask containing the drug solution and were then mixed until dissolved. The mixtures were dried under Roto-vap for about 15 min.

After a dry powder was obtained, the solid was then scraped from the flask and transferred to a glass container. The solid was dried for 24 hr at 55° C in a vacuum oven with nitrogen bleed. The dry solid was then milled by mortar and pestle and was stored in a tightly capped vial. Before dosing the rats, 30 milliliters (mL) of water was added to the dry solid to yield 3 mg/mL of *R* isomer at C*, *S* isomer at C*, or R:*S* at C* mixture.

Male Sprague Dawley rats (Charles River Laboratories, Kingston, RI; n = 6-7/group) were used in the study. The day before dosing, the rats were cannulated in the carotid artery for collecting blood samples. Each rat was administered orally at a nominal dose of 30 mg/mL of either the *R* isomer at C*, the *S* isomer at C*, or the *R:S* at C* mixture. The experiments were performed with a parallel study design as 6 separate single dose studies as described in Table 5.

**Table 5 Experimental Design**

| No. of Rats | Dose Route | Dose Formulation | Compound Admmistered | Target Dose Level (mg/kg) | Target Dose Volume (mL/kg) |
|---|---|---|---|---|---|
| 7 | Oral Gavage | L/N 1832-071B | *R* isomer at C* | 30 | 10 |
| 6 | Oral Gavage | L/N 1832-071A | *S* isomer at C* | 30 | 10 |
| 6 | Oral Gavage | L/N1832-071C | R:*S* at C* mixture | 30 | 10 |

Following TV injection and oral administration, serial blood samples were collected at pre-dose and 0.25, 0.5, 1, 1.5, 2, 3, 4, 6, 8, and 24 hr after oral administration. The blood samples were collected in tubes containing potassium EDTA and were centrifuged to plasma within 15 min of collection. A diluted solution of formic acid was prepared by diluting 1 mL of formic acid with 9 mL of water. A total of 5 micro liters (µL) of this diluted formic acid was placed into each plasma tube. An aliquot of plasma (100 µL per tube) was placed into appropriately labeled tubes containing the formic acid, and the acidified plasma was stored frozen at -70° C until analysis. The acidification step was implemented to prevent in vitro interconversion.

Referring to Table 6, several bioavailability parameters for compounds undergoing reversible metabolism could be estimated by the following descriptions. All symbols contain a subscript representing the measured entity and a superscript representing the dosed entity:

FRR represents the bioavailability of the *R* isomer at C* after a dose of the *R* isomer at C*;

FSS represents the bioavailability of the *S* isomer at C* after a dose of the *S* isomer;

FR+SR represents the estimated total bioavailability based on the combined exposure to the *R* and *S* isomers (at position C*) following a dose of the *R* isomer;

FS+RS represents the estimated total bioavailability based on the combined exposure to the *R* and *S* isomers (at position C*) following a dose of the *S* isomer

FSR represents the bioavailability of the *S* isomer at C* after a dose of the *R* isomer at C*, which is the relative ratio of dose-normalized AUCS after an oral dose of the *R* isomer versus that after the IV dose of the *R* isomer; and

FRS represents bioavailability of the *R* isomer at C* after a dose of the *S* isomer at C*, which is the relative ratio of dose-normalized AUCR after an oral dose of the *S* isomer versus that after the IV dose of the *S* isomer.

**Table 6 Summary of all bioavailability parameters**

| Parameter Symbol | Parameter Value (%) | *S* Isomer at C* Contribution (%) | *R* Isomer at C* Contribution (%) |
|---|---|---|---|
| F_{R}^{R} | 80.59 | 0 | 80.59 |
| F_{S}^{S} | 42.88 | 42.88 | 0 |
| F_{R+S}^{R} | 96.35 | 15.76 | 80.59 |
| F_{R+S}^{S} | 49.23 | 42.88 | 6.35 |
| F_{S}^{R} | 139.99 | NA | NA |
| F_{R}^{S} | 157.88 | NA | NA |

| | | | |
|---|---|---|---|
| N/A = not applicable | | | |

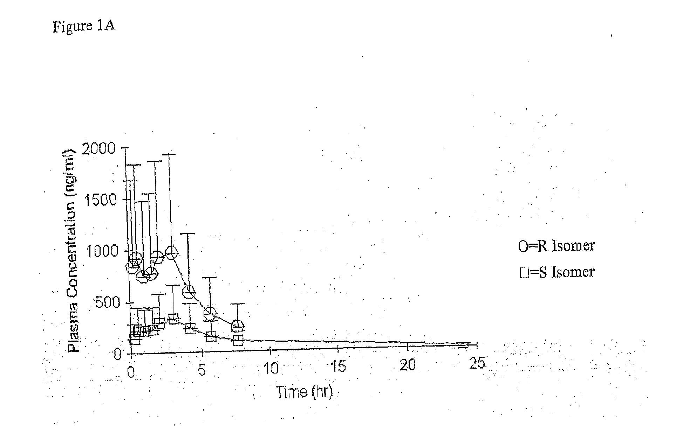

Referring also to Figs 1A-B, 2A-B, and 3A-B, the *R* isomer at C* was more readily absorbed than the *S* isomer at C* since the FRR value was greater than the FSS value, and the FR+SR value was greater than the FR+SS value (approximately 2-fold). Figures 1A, 2A, and 3A are rectilinear plots of plasma concentrations of a compound of formula (I) with greater than 50% *R* isomer at position C* and a compound with 50% or less *R* isomer at position C* versus time following oral administration of the compound. Figures 1B, 2B, and 3B are Log-linear plots of plasma concentrations of a compound of formula (I) with greater than 50% *R* isomer at position C* and a compound with 50% or less *R* isomer at position C* versus time following oral administration of the compound. The plasma concentrations in Figs 1A and 1B were measured in rats following oral administration at a nominal dose of 30 mg/kg (R isomer at C*). The plasma concentrations in Figs 2A and 2B were measured in rats following oral administration at a nominal dose of 30 mg/kg (S isomer at C*). The plasma concentrations in Figs 3A and 3B were measured in rats following oral administration at a nominal dose of 30 mg/kg (R:S at Position C* Mixture).

The *R* to *S* at position C* conversion was observed after an oral dose of the *R* isomer at C*, and the *S* to *R* at position C* conversion was observed after an oral dose of the *S* isomer at C*. The contribution of the *S* isomer to the total bioavailability after a dose of the *R* isomer was assessed by the difference between FR+SR and FRR, which was 15.76%. Similarly, the contribution of the *R* isomer at C* to the total bioavailability after a dose of the *S* isomer at C* was assessed with the difference between FR+SS and FRS, which was 6.35%.

The extent of the *R* to *S* at position C* conversion was greater for an orally administered dose of the *R* isomer at C* as compared with the intravenously administered dose of the *R* isomer at C*. The value of FSR was 139.99%, which means that the AUCINF (area under the curve from the time of dosing to infinity) of the *S* isomer observed after an oral dose was 1.39 times that observed after the same doses were given intravenously. Similarly, the FRS value was 157.88%, indicating that the *S* to *R* at position C* conversion was greater (approximately 1.57 times) for the orally administered *S* isomer than for the intravenously administered *S* isomer.

The *R* to *S* at position C* conversion was more prominent than the *S* to *R* conversion at position C*. The DN_AUCINF (dose-normalized AUCINF) of the *S* isomer at C* from a dose of the *R* isomer at C* was higher than that from a dose of the *S* isomer at C* (about 10-fold). Although a similar trend was observed for the exposure of the *R* isomer at C*, the DN_AUCINF of the *R* isomer from a dose of the *S* isomer at C* was higher than that from a dose of the *R* isomer at C* (2-fold), as shown in Table 6.

**Table 7 Summary of Dose-normalized AUC_{INF} of the R isomer and the S isomer.**

| Dose Group | *R*:*S* Dose Ratio | Dose (mg/kg) | | DN_AUC_{INF} (hr*µg/mL) Mean(±SD) | |
|---|---|---|---|---|---|
| | | *R* at C* | *S* at C* | *R* at C* | *S* at C* |
| *R* isomer at C* | 92:8 | 16.87 | 1.56 | 0.34 (±0.17) | 1.35 (±0.88) |
| *S* isomer at C* | 7:93 | 1.23 | 17.65 | 0.72 (±0.52) | 0.14 (±0.07) |
| *R*:*S* at C* mixture | 54:46 | 10.50 | 9.05 | 0.22 (±0.07) | 0.30 (±0.06)^{a} |

| | | | | | |
|---|---|---|---|---|---|
| ^{a} n=2; AUC_{INF} cannot be estimated in the remaining rats | | | | | |

Referring to Table 8, a similar exposure of the *S* isomer at position C* can be achieved by the administration of either a dose of the *R* isomer at C* or a dose of the *S* isomer at C*. The DN_AUCINF values of the *S* isomer at C* were similar (0.13 hr* µg/mL versus 0.14 hr*µg/mL), based on the assumption that the presence of the *S* isomer at C* (8% of the total) in the *R* isomer at C* dose made negligible contribution to the overall exposure of the *S* isomer at C*. A dose of the *S* isomer at C* achieved a much lower exposure of the *R* isomer at C* (approximately one-seventh) than a dose of the *R* isomer at C*.

**Table 8 Summary of DN_AUC_{INF} of the R isomer at C* and the S isomer at C* when dose-normalized to the dose of the Primary Isomer.**

| Dose Group | *R*:*S*Dose Ratio | Dose (mg/kg) | | DN_AUC_{INF} (hr*µg/mL) Mean (±SD) | |
|---|---|---|---|---|---|
| | | *R* at C* | *S* at C* | *R* at C* | *S* at C* |
| *R* isomer at C* | 92:8 | 16.87^{a} | 1.56 | 0.34(± 0.17) | 0.13(± 0.08) |
| *S* isomer at C* | 7:93 | 1.23 | 17,65^{a} | 0.050(± 0.036) | 0.14(± 0.07) |
| *R*:*S* at C* mixture | 54:46 | 10.50^{a} | 9.05^{a} | 0.22(± 0.07) | 0.30(± 0.06)^{b} |

| | | | | | |
|---|---|---|---|---|---|
| ^{a}Dose values used for calculating dose-normalized AUC_{INF}. ^{b}n-2; AUC_{INF} cannot be estimated in the remaining rats. Note: The contribution of the minor diastereoisomer to AUC_{INF} was assumed negligible. | | | | | |

Following the administration of the *R*:*S* at C* mixture, there was more *R* to *S* at C* conversion than *S* to *R* conversion. The value of DN_AUCINF of the *S* isomer (0.30 hr*µg/mL) was higher than expected for a single oral dose of the *S* isomer (0.14 hr*µg/mL). Furthermore, the DN_AUCINF value of 0.30 hr*µg/mL approximates the sum of the DN_AUCINF value from an *S* isomer dose and the DN_AUCINF value from a *R* isomer dose.

The DN_AUCINF value of the *R* isomer from an oral dose of *R*:*S* mixture did not appear to be different from that observed after an oral dose of the *R* isomer.

There was high variability in the observed time to reach the maximum concentrations of the *R* isomer and the *S* isomer. The mean (± SD) Tmax values were 2.11 (± 1.24) hr, 3.39 (± 2.38) hr, and 4.88 (± 3.53) hr for the *R* isomer after oral administration of the *R* isomer, the *S* isomer, and *R*:*S* mixture, respectively. The corresponding mean (±SD) Tmax values were 2.27 (± 1.44) hr, 2.52(± 2.19) hr, and 4.88 (± 3.53) hr for the *S* isomer.

Following an oral dose of the *R* isomer, the harmonic mean t½ value was 2.18 hr for the *R* isomer, which was greater than the IV t_{½} value of 0.75 hr. Following an oral dose of the *S* isomer, the harmonic mean t_{½} value was 3.60 hr for the *S* isomer, which was greater than the IV t_{½} value of 1.73 hr.

Therefore, in rats, the bioavailability of the *R* isomer at position C* was about 2 times that of the *S* isomer at position C*. The total bioavailability was about 98% for the orally dosed *R* isomer at position C* and about 50% for the orally dosed *S* isomer at position C*, when represented by the combined exposure of the 2 isomers. Furthermore, following oral dosing, the *R* at C* to *S* at C* conversion was more prominent than the *S* at C* to *R* at C* conversion. Interconversion occurred to a larger extent after an oral dose when compared with that after an IV dose.

**Example 2: HCV Enzyme Assay Protocol**

HPLC Microbore method for separation of 5AB substrate and products

Substrate:
NH2-Glu-Asp-Val-Val-(alpha)Abu-Cys-Mer-Met-Ser-Tyr-COOH
A stock solution of 20 mM 5AB was made in DMSO w/ 0.2M DTT. This was stored in aliquots at -20 C.

Buffer:

50 mM HEPES, pH 7.8; 20% glycerol; 100 mM NaCl

Total assay volume was 100 µL.

| Reagent | X1 (µL) | conc. in assay |
|---|---|---|
| Buffer | 86.5 | See above |
| 5 mM KK4A | 0.5 | 25 µM |
| 1 M DTT | 0.5 | 5 mM |
| DMSO or inhibitor | 2.5 | 2.5% v/v |
| 50 µM tNS3 | 0.05 | 25 nM |
| 250 µM 5AB (initiate) | 20 | 25 µM |

The buffer, KK4A, DTT, and tNS3 were combined; distributed 78 µL each into wells of 96 well plate. This was incubated at 30° C for ≈5-10 min.

2.5 µL of appropriate concentration of test compound was dissolved in DMSO (DMSO only for control) and added to each well. This was incubated at room temperature for 15 min.

Initiated reaction by addition of 20 µL of 250 µM 5AB substrate (25 µM concentration is equivalent or slightly lower than the Km for 5AB).

Incubated for 20 mm at 30° C.

Terminated reaction by addition of 25 µL of 10% TFA

Transferred 120 µL aliquots to HPLC vials

Separated SMSY product from substrate and KK4A by the following method:

Microbore separation method:

Instrumentation: Agilent 1100

Degasser G1322A

Binary pump G1312A

Autosampler G1313A

Column thermostated chamber G1316A

Diode array detector G1315A

Column:

Phenomenex Jupiter; 5 micron C18; 300 angstroms; 150x2 mm; P/O 00F-4053-B0

Column thermostat: 40° C

Injection volume: 100 µL

Solvent A = HPLC grade water + 0.1% TFA

Solvent B = HPLC grade acetonitrile + 0.1% TFA

| Time (min) | %B | Flow (ml/min) | Max press. |
|---|---|---|---|
| 0 | 5 | 0.2 | 400 |
| 12 | 60 | 0.2 | 400 |
| 13 | 100 | 0.2 | 400 |
| 16 | 100 | 0.2 | 400 |
| 17 | 5 | 0.2 | 400 |

Stop time: 17 min

Post-run time: 10 min.

Compounds with Ki's below 1µM are designated A. Compounds with Ki's ranging from 1µM to 5µM are designated B. Compounds with Ki's above 5µM are designated C. Table 2 below depicts Mass Spec., HPLC, 1H-NMR, and Ki data for certain compounds of the invention. "ND" means no data. 1H-NMR spectra were recorded at 500 MHz using a Broker AMX 500 instrument.

In a 15 minute incubation period, the *S* isomer exhibited a Ki in category A and the *R* isomer exhibited a Ki in category B. The Ki are determined by the Fluorescence Peptide Cleavage Assays for HCV NS3 Protease and HPLC-based Peptide Cleavage Assay for HCV NS3 Serine Protease described in examples 3 and 4.

Pharmacokinetic Assays:

**Example 3: Fluorescence Peptide Cleavage Assays for HCV NS3 Protease:**

The steady-state inhibition constant, Ki*, of several compounds of formula I was determined in an assay that was modified slightly from a fluorescence peptide cleavage assay described in Taliani, M., E. Bianchi, F. Narjes, M. Fossatelli, A. Rubani, C. Steinkuhler, R. De Francesco, and A. Pessi. 1996. A Continuous Assay of Hepatitis C Virus Protease Based on Resonance Energy Transfer Depsipeptide Substrates. Anal. Biochem. 240:60-67; hereby incorporated by reference.

The assay was performed in a buffer containing 50 mM HEPES (pH 7.8), 100 mM NaCl, 20% glycerol, and 5 mM dithiothreitol (Buffer A), using the RET-S1 fluorescent peptide as substrate. Reactions were continuously monitored using an fMax fluorescence microtitre plate reader (Molecular Devices; Sunnyvale, CA) thermostatted at 30° C, with excitation and emission filters of 355 nm and 495 nm, respectively. A stock solution of HCV NS3 protease in Buffer A containing 25 µM KK4A peptide was pre-incubated for 10 min at room temperature, followed by an additional 10 min incubation at 30° C. An aliquot of a compound of formula I with 50% or less *R* isomer, dissolved in 100% dimethyl sulfoxide (DMSO), was added to a solution of RET-S1 in Buffer A containing 25 µM KK4A peptide and pre-incubated at 30° C for 10 min. The reaction was initiated by the addition of an aliquot of the NS3 protease/KK4A stock to the compound/RET-S1/KK4A/Buffer A mixture to yield final concentrations of 12 µM RET-S1, 2% (v/v) DMSO, 25 µM KK4A peptide, and 0.5-1.0 nM HCV NS3 protease. Steady-state reaction rates were determined from linear regression of the fluorescence vs. time data points obtained over a 5-min window at a reaction time of 4 h. Ki* of the compounds was determined by fitting activity vs. inhibitor concentration data to the Morrison equation for tight-binding enzyme inhibition. See Morrison, J. F. 1969. Kinetics of the reversible inhibition of enzyme-catalyzed reactions by tight-binding inhibitors. Biochim. Biophys. Acta 185:269-86; hereby incorporated by reference.

The dissociation rate constant of the complex between HCV NS3 protease and compounds was determined using the RET-S1 substrate as follows. A stock solution of HCV NS3 protease in Buffer A containing 25 µM KK4A peptide was prepared as described above. A 1 µL aliquot of 100 µM of a compound of formula I with 50% or less *R* isomer dissolved in 100% DMSO was added to a 49 µL aliquot of the pre-warmed enzyme stock to yield a mixture of 320 nM enzyme and 2 µM of the compound, which was then incubated at 30° C for 4 h to allow formation of the enzyme-inhibitor complex to reach equilibrium. The dissociation reaction was initiated by serial dilution of an 8 µL aliquot of the enzyme-inhibitor mixture, into 192 µL of Buffer A containing 25 µM KK4A peptide and 2% DMSO (v/v), and then into 192 µL of RET-S1 in Buffer A containing 25 µM KK4A peptide and 2% DMSO, both pre-warmed to 30° C. Final concentrations were 0.5 nM HCV NS3 protease, 25 µM KK4A peptide, 12 µM RET-S1, and 3 nM (1S,3aR,6aS)-2-[(2S)-2-[[(2S)-2-cyclohcxyl-1-oxo-2-[(pyrazinylearbonyl)amino]ethyl]amino]-3,3-dimethyl-1-oxobutyl]-N-[(1R)-1-[2-(cyclopropylamino)-1,2-dioxoethyl]butyl]octahydro-cyclopenta[c]pyrrole-1-carboxamide. The change in fluorescence was monitored over a 4h window, and the fluorescence vs. time data plots were fit to the following equation: F(t) = Vs × t + (Vi - Vs) × (1-exp (-kobs × t))/kobs + C, by non-linear regression. Control rates were determined from a reaction containing neat DMSO. Under these experimental conditions kobs is within 20% of koff. The half-life of the complex (t_{1/2}) was calculated from koff using the following equation: t_{1/2} = 0.693/koff.

**Example 4: HPLC-based Peptide Cleavage Assay for HCV NS3 Serine Protease:**

This assay is a slightly modified version of what has been previously described in Landro, J. A., S. A. Raybuck, Y. P. Luong, E. T. O'Malley, S. L. Harbeson, K. A. Morgenstern, G. Rao, and D. J. Livingston. 1997. Mechanistic Role of an NS4A Peptide Cofactor with the Truncated NS3 Protease of Hepatitis C Virus: Elucidation of the NS4A Stimulatory Effect via Kinetic Analysis and Inhibitor Mapping. Biochemistry 36:9340-9348; hereby incorporated by reference.

The NS3 protease (10-25 nM) and 25 µM KK-4A was pre-incubated for 5 min in a buffer containing of 50 mM HEPES (pH 7.8), 100 mM NaCl, 20% glycerol, and 5 mM dithiothreitol, at room temperature. HCV protease inhibitors, dissolved in DMSO, were added to the enzyme mixture, with a final DMSO concentration of 2% (v/v), and incubated for 15 min at room temperature. The proteolysis reaction was initiated by the addition of NS5A/NS5B substrate at a concentration equal to its Km (25 µM) and incubated for 15 min at 30° C. The reaction was quenched by the addition of one-forth volume of 10% trifluoroacetic acid and analyzed on a reversed phase HPLC column. Sample analysis was completed within 24 hours of reaction termination. The apparent inhibition constant, Ki(app), of HCV protease inhibitors were calculated using a least-squares fitting method of nonlinear regression based on Morrison's equation for tight binding competitive inhibition. See Morrison, et al.

**Example 5: IC₅₀ Determination in HCV Replicon Cells:**

Several compounds of formula I possess concentrations at which the HCV RNA level in the replicon cells is reduced by 50% (IC₅₀) or by 90% (IC90), or the cell viability is reduced by 50% (CC₅₀) were determined in HCV Conl sub-genomic replicon cells (Lohmann, V., F. Korner, J. Koch, U. Herian, L. Theilmann, and R. Bartenschlager. 1999. Replication of subgenomic hepatitis C virus RNAs in a hepatoma cell line. Science 285:110-3.16; hereby incorporated by reference) using four-parameter curve fitting (SoftMax Pro). Briefly, the replicon cells were incubated with compounds diluted in medium containing 2% fetal bovine serum (FBS) and 0.5% DMSO at 37° C. Total cellular RNA was extracted using an RNeasy-96 kit (Qiagen, Valencia, CA) and the copy number of the HCV RNA was determined in a quantitative, real-time, multiplex reverse transcription-PCR (QRT-PCR or Taqman) assay. The cytotoxicity of compounds in the HCV replicon cells was measured under the same experimental settings using the tetrazolium-based cell viability assay as described before. See Lin, C., K. Lin, Y. P. Luong, B. G. Rao, Y. Y. Wei, D. L. Brennan, J. R. Fulghum, H. M. Hsiao, S. Ma, J. P. Maxwell, K. M. Cottrell, R. B. Pemi, C. A. Gates, and A. D. Kwong. 2004. In vitro resistance studies of hepatitis C virus serine protease inhibitors, (1S,3aR,6aS)-2-[(2S)-2-[[(2S)-2-cyclohexyl-1-oxo-2-[(pyrazinylcarbonyl)amino]ethyl]amino]-3,3-dimethyl-1-oxobutyl]-N-[(1R)-1-[2-(cyclopropylamino)-1,2-dioxoethyl]butyl]octahydro-cyclopenta[c]pyrrole-1-carboxamide.

**Example 6: Pharmacokinetie Studies in Animals:**

The intravenous pharmacokinetics of compounds of formula I were evaluated in rats and dogs. A group of 3 male Sprague-Dawley rats weighing between 250 to 300 g was administered an intravenous bolus dose of 0.95 mg/kg of an S-diastereomer of formula I, in a vehicle consisting of 15% ethanol, 10% dimethyl isosorbide, 35% PEG400 and 40% D5W (5% dextrose in water). Serial blood samples were collected in heparinized tubes at 0 (pre-dose), 0.083, 0.167, 0.25, 0.5, 1, 1.5, 2, 3, 4, 6, and 8 h, post-dose administration. A group of 3 male Beagle dogs (8 to 12 kg; Charles River, MA) were administrated an intravenous bolus dose of 3.5 mg/kg of the diastereomer in 10% ethanol, 40% PEG400 and 50% D5W. Serial blood samples were collected in heparinized tubes prior to dosing, and at 0.083, 0.167, 0.25, 0.5, 1, 1.5, 2, 4, 6, 8, 12 and 24 h following dose administration. For oral studies in rats and dogs, an S-diastereomer compound of formula I was formulated in polyvinylpyrrolidone (PVP) K30 plus 2% sodium lauryl sulfate and then dosed as an oral gavage. A group of 3 male Sprague-Dawley rats (250 to 300 g, Harlan, MD) was dosed orally with 40 mg/kg of the compound, and a group of 3 male Beagle dogs (10.9-12.0 kg) was administered an oral dose of 13.2 mg/kg of the compound. In both oral studies, blood samples were taken at pre-dose, 0.25, 0.5, 1, 1.5, 2, 3, 4, 6, 8, 12, and 24 h following dose administration. In both intravenous and oral studies, plasma samples were obtained by centrifugation and stored at -70° C until analysis. Samples obtained from the rat intravenous study were subjected to a chiral liquid chromatography/mass spectrometry (LC/MS/MS) analysis, while samples of all other studies were analyzed using a non-chiral LC/MS/MS method. Standard techniques were employed to conduct non-compartmental analysis of data using WinNonlin Enterprise/Pro Version 4.0.1 (Pharsight Corporation, Mountain View, CA) for calculation of the following pharmacokinetic parameters, such as Cₘₐₓ or Cₘᵢₙ or C_{avg} (maximum or minimum or average concentration of drug in serum, respectively), AUC0-8 or AUCO-inf (total area under the concentration curve from 0 to 8 h or from 0 to infinite, respectively), t_{1/2} (half-life of elimination), CL (total body clearance), and Vss (volume of distribution at steady state).

**Example 7: Evaluation of Liver to Plasma Ratio of Several Compounds of Formula I in Rats**

The liver to plasma ratio of a diastereomer of a compound of formula I was evaluated in rats following the oral administration of a solution of a compound in propylene glycol. Six groups (3 animals per group) of male Fisher rats were orally administered a nominal dose of 30 mg/kg of the diastereomer of a compound of formula I. At 0 (pre-dose), 0.5, 1, 2, 4 or 8 h post-dose administration, one group of 3 animals was sacrificed per time point, and one blood and the corresponding liver sample were obtained from each animal. Plasma samples were obtained by centrifuging the blood samples. The whole liver was removed from the animal and perfused with normal saline to remove traces of blood. After weighing, the liver was cut into small pieces and homogenized with an equal volume of water. The plasma and liver samples were stored at -70° C until analysis using the non-chiral LC/MS/MS method.

**Example 8: Mouse Model for HCV NS3-4A Serine Protease:**

The details of this mouse model for the HCV NS3-4A serine protease will be described elsewhere. A brief description of this model is given here. An HCV cDNA fragment encoding an initiation Met codon, a His-tag (SHHHHHHAM), the full-length 631 amino acids of HCV NS3 protein, the full-length 54 residues of HCV NS4A protein, and the N-terminal 6 (ASHLPY) amino acid of HCV NS4B protein, was fused to a full-length secreted placental alkaline phosphatase (SEAP) gene by overlapping PCR from pYes2-NS3-4A plasmid and pSEAP2 (Clontech, Palo Alto, CA), and then subcloned into an adenovirus expression vector, pAdenovirus (Clontech) to generate pAd-WT-HCVpro-SEAP. A corresponding version of this fusion gene with an Ala substitution of the catalytic Ser-139 in the active triad of HCV NS3-4A serine protease, pAd-MT-HCVpro-SEAP, was generated by the same overlapping PCR and subcloning method using a pYes2/NS3-4A containing the Ser139-to-Ala mutation. Adenovirus was packaged by transfection of HEK293 cells (ATCC, Rockville, MD) with PacI-linearized pAdenovirus plasmid, pAd-WT-HCVpro-SEAP or pAd-MT-HCVpro-SEAP, in the presence of Lipofectamine 2000 (Invitrogen). Recombinant adenoviruses were purified by cesium chloride density gradient centrifugation and desalted by diafiltration with Centriprep YM-50 filters (Millipore, Bedford, MA). Adenovirus rapid titer kits (Clontech) were used to determine the amount of infectious units (IFU) of recombinant adenoviruse stocks. Six week-old SCID mice (≈ 20 g, Charles River, Wilmington, MA) were dosed by oral gavage with a compound of formula (I) with 50% or less R isomer or with vehicle alone. Two hours after dosing, recombinant adenovirus, Ad-WT-HCVpro-SEAP or Ad-MT-HCVpro-SEAP, was injected in the lateral tail vein of the mice.

Experimental criteria prospectively stated that animals with incomplete injections would not be included in the data analysis. Mice were anesthetized with isofluorane, and blood samples were collected at different time points post-injection using retro orbital eye bleeds, or at the ultimate time point by cardiac heart puncture. Mouse serum was diluted 5-fold with distilled water and the activity of SEAP in the serum was measured using a Phospha-Light detection system (Applied Biosystems, Foster City, CA) and a Tropix TR717 microplate luminometer (Tropix, Bedford, MA). For the pharmacokinetic analysis, plasma samples were stored at -80° C prior to analysis. The mouse liver samples were mixed with 2 volumes (v/w) of 2M formic acid, homogenized and stored at -80 °C prior to analysis. The samples were analyzed using a chiral LC/MS/MS system.

**Example 9: Pharmaceutical Compositions**

The mixture of diastereomeric compounds of this invention can be formulated in any manner suitable to deliver a therapeutically effective amount of the mixture of compounds to the subject (e.g., a mammal). In some embodiments, the mixture of diastereomeric compounds of Formula I can be formulated in polyvinylpyrrolidone (PVP) K-30 plus sodium lauryl sulfate (SLS).

Mixture of diastereomeric compounds: 49.5%

PVP K30: 49.5%

SLS: 1%

The composition can be prepared by dissolving the mixture of diastereomeric compounds, PVP K30, and suspending SLS in a solvent such as methanol:methylene chloride followed by spray-drying to remove the solvent. Other pharmaceutical compositions contain different amounts of the mixture of diastereomeric compounds of Formula I (49%), PVP K-30 (49%), and SLS (2%).

Additional examples of pharmaceutical compositions of the diastereomeric compounds of formula I can be formulated similarly to the compositions described in WO 2005/123076, the entire content of which is hereby incorporated in its entirety.

VI. OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

The present invention further comprises the aspects defined in the following clauses (which form part of the present description but are not considered as claims in accordance with decision J 15/88 of the Legal Board of Appeal of the European Patent Office):
1. A mixture of diastereomeric compounds of Formula I: 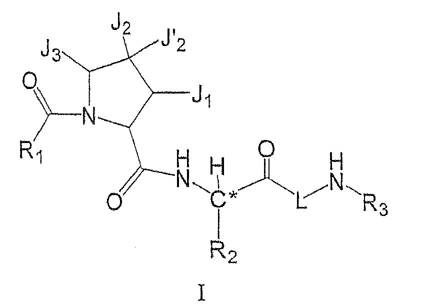 or a pharmaceutically acceptable salt or mixtures thereof, wherein
   C* represents a diasteromeric carbon atom; and
   the R isomer is greater than 50% of the mixture relative to the S isomer at the C* position;
   R₁ is RW-, P₃-, or P₄-L₂-P₃-;
   R is an optionally substituted aliphatic, an optionally substituted cycloaliphatic, an optionally substituted heterocycloaliphatic, an optionally substituted aryl, or an optionally substituted heteroaryl;
   W is a bond, -NR₄-, -O-, or -S-;
   R₄ is H, an optionally substituted aliphatic, an optionally substituted cycloaliphatic, an optionally substituted heterocycloaliphatic, an optionally substituted aryl, or an optionally substituted heteroaryl;
   P₃- is 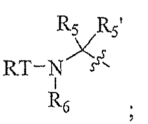
   T is -C(O)-, -OC(O)-, -NHC(O)-, -C(O)C(O)-, or -SO₂-;
   Each of R₅ and R₅' is independently H, an optionally substituted aliphatic, an optionally substituted cycloaliphatic, an optionally substituted heterocycloaliphatic, an optionally substituted phenyl, or an optionally substituted heteroaryl;
   R₆ is an optionally substituted aliphatic, an optionally substituted heteroaryl, an optionally substituted phenyl; or R₅ and R₆, together with the atoms to which they are attached, may form a 5- to 7-membered optionally substituted monocyclic heterocycloaliphatic, or a 6- to 12-membered optionally substituted bicyclic heterocycloaliphatic, in which each heterocycloaliphatic ring optionally contains an additional heteroatom selected from -O-, -S- or -NR₅₀-;
   R₅₀ is H, an optionally substituted aliphatic, an optionally substituted heteroaryl, or an optionally substituted phenyl;
   P₄-L₂-P₃ is 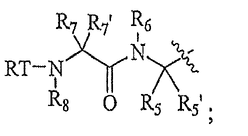
   Each of R₇ and R₇' is independently H, an optionally substituted aliphatic, an optionally substituted cycloaliphatic, an optionally substituted heterocycloaliphatic, an optionally substituted phenyl, or an optionally substituted heteroaryl; or
   R₇ and R₇', together with the atom to which they are attached, may form a 3- to 7-membered cycloaliphatic or heterocycloaliphatic ring; or
   R₇ and R₆, together with the atoms to which they are attached, may form a 5- to 7-membered optionally substituted monocyclic heterocycloaliphatic, a 5- to 7-membered optionally substituted monocyclic heteroaryl, a 6- to 12-membered optionally substituted bicyclic heterocycloaliphatic, or a 6- to 12-membered optionally substituted bicyclic heteroaryl, in which each heterocycloaliphatic or heteroaryl ring optionally contains an additional heteroatom selected from -O-, -S- or -NR₅₀-, or
   When R₅ and R₆, together with the atoms to which they are attached, may form a ring; R₇ and the ring system formed by R₅ and R₆ may form an 8- to 14-membered optionally substituted bicyclic fused ring system, wherein the bicyclic fused ring system is optionally further fused with an optionally substituted phenyl to form an optionally substituted 10- to 16-membered tricyclic fused ring system;
   R₈ is H or a protecting group; and
   R₂ is an optionally substituted aliphatic, an optionally substituted cycloaliphatic, an optionally substituted heterocycloaliphatic, an optionally substituted heteroaryl, or an optionally substituted phenyl;
   R₃ is H, an optionally substituted aliphatic, an optionally substituted cycloaliphatic, an optionally substituted heterocycloaliphatic, an optionally substituted aryl, or an optionally substituted heteroaryl;
   L is a bond, -CF₂-, -C(O)-, or -SO₂-;
   Each of J₁, J₂, J'₂, and J₃ is independently halogen, -OR', -OC(O)N(R')₂, -NO₂, -CN, - CF₃, -OCF₃, -R', oxo, thioxo, -N(R')₂, -SR', -COR', -SO₂R', -SO₂N(R')₂, -SO₃R', -C(O)R', -C(O)C(O)R', -C(O)CH₂C(O)R', -C(S)R', -C(O)OR', -OC(O)R', -C(O)N(R')₂, -OC(O)N(R')₂, -C(S)N(R')₂, -(CH₂)₀₋₂NHCC(O)R', -N(R')N(R')COR', -N(R')N(R')C(O)OR -N(R')N(R')CON(R')₂, -N(R')SO₂R', -N(R')SO₂N(R')₂, -N(R')C(O)OR', -N(R')C(O)R', -N(R')C(S)R', -N(R')C(O)N(R')₂, -N(R')C(S)N(R')₂, -N(COR')COR', -N(OR')R', - C(=NH)N(R')₂, -C(O)N(OR')R', -C(=NOR')R', -OP(O)(OR')₂, -P(O)(R')₂, -P(O)(OR')₂, or - P(O)(H)(OR'), or of J₂ and J'₂ is H, wherein;
   Two R' groups together with the atoms to which they are bound may form a 3- to 10-membered aromatic or non-aromatic ring system having up to 3 heteroatoms independently selected from N, O, or S, wherein the ring is optionally fused to a C₆-C₁₀ aryl, a C₅-C₁₀ heteroaryl, a C₃-C₁₀ cycloalkyl, or a C₃-C₁₀ heterocycloaliphatic, and wherein any ring has up to 3 substituents each independently selected from J₂;
   Each R' is independently selected from H, C₁-C₁₂ aliphatic, C₃-C₁₀ cycloalkyl, or C₃-C₁₀ cycloalkenyl, C₃-C₁₀ cycloalkyl-C₁-C₁₂ aliphatic, C₃-C₁₀ cycloalkenyl-C₁-C₁₂ aliphatic, C₆-C₁₀ aryl, C₆-C₁₀ aryl-C₁-C₁₂ aliphatic, 3- to 10-membered heterocycloaliphatic, 6- to 10-membered heterocyc1oaliphatic-C₁-C₁₂ aliphatic, 5- to 10-membered heteroaryl, or 5- to 10-membered heteroaryl-C₁-C₁₂ aliphatic, wherein R' has up to 3 substituents each independently selected from J₂; or
   J₁ and J₂, together with the atoms to which they are attached, may form a C₈ to C₁₂ optionally substituted bicyclic ring;
   J₁ and J₃, together with the atoms to which they are attached, may form a C₈ to C₁₂ optionally substituted bicyclic ring;
   J₂ and J'₂, together with the carbon atom to which they are attached, may form an optionally substituted 5-10 membered cycloaliphatic, or an optionally substituted 5- to 10-membered heterocycloaliphatic ring; or
   J₂ and J₃, together with the atoms to which they are attached, may form a C₈ to C₁₂ optionally substituted bicyclic ring.
2. The mixture of diastereomeric compounds according to clause 1, wherein the 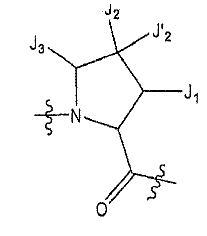 moiety is 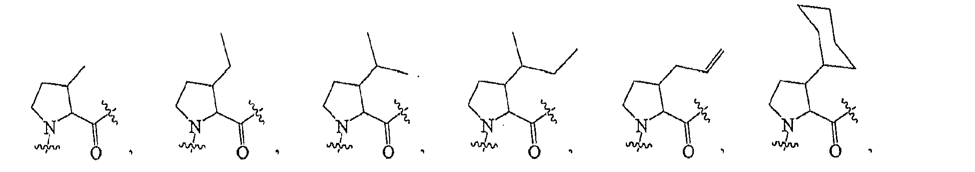 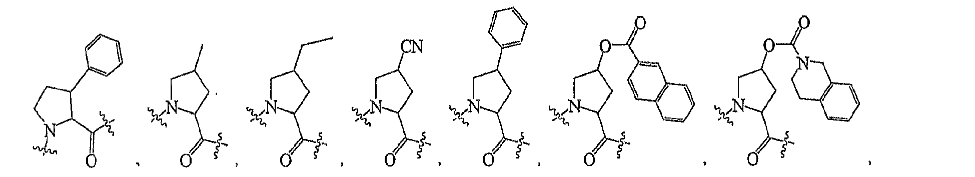 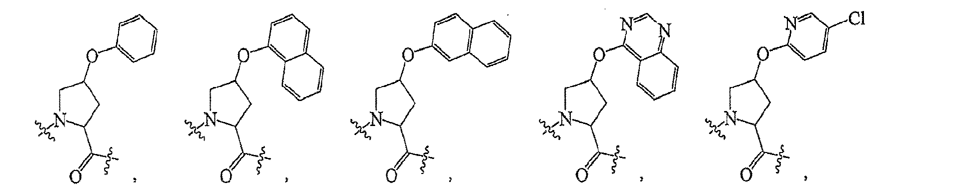 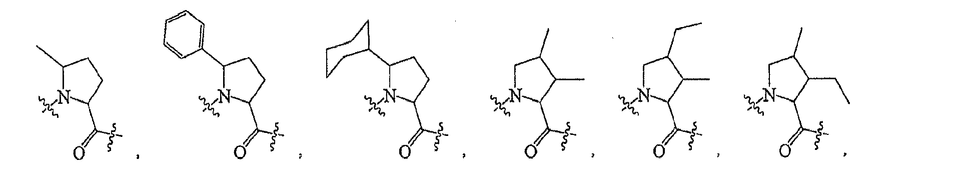 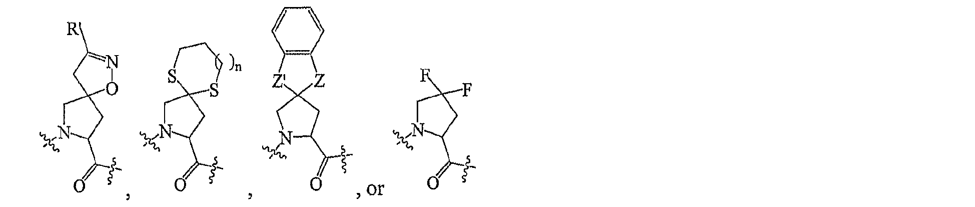 wherein n is 0 or 1; and each of Z and Z' is independently -CR'R'-, S, or O.
3. The mixture of diastereomeric compounds according to clause 1, wherein J₁ and J₂, together with the atoms to which they are attached, form an optionally substituted mono- or bicyclic ring such that the 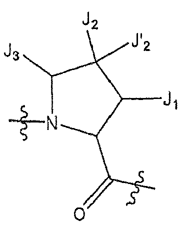 moiety is 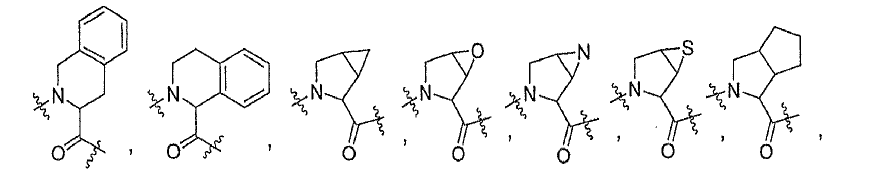 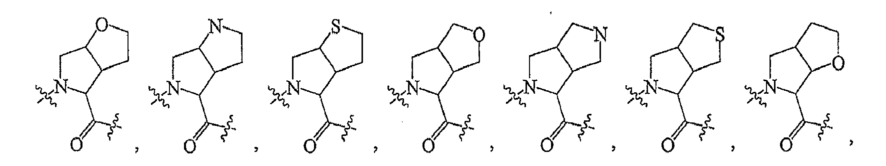 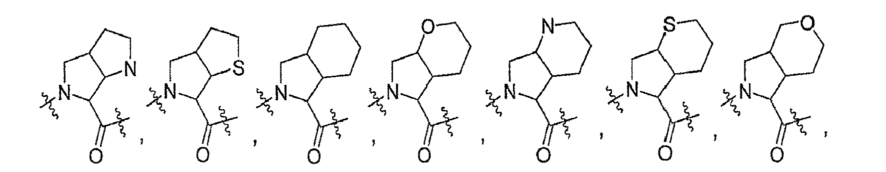 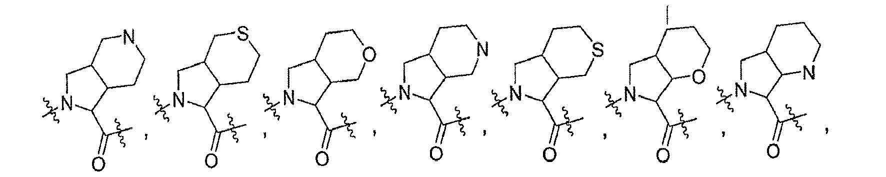 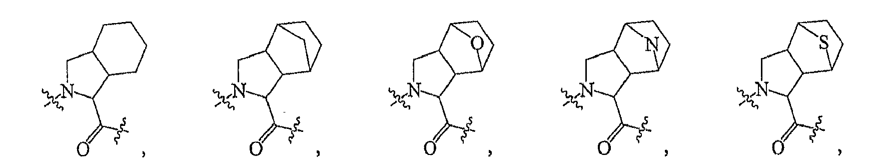 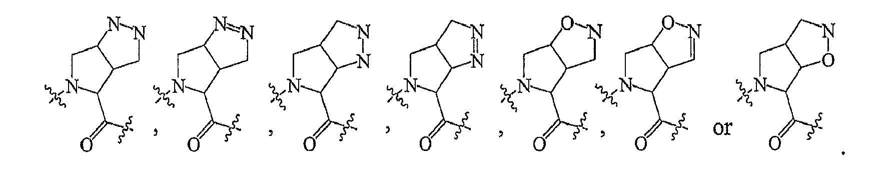
4. The mixture of diastereomeric compounds according to clause 1, wherein J₂ and J₃, together with the atoms to which they are attached, form an optionally substituted mono- or bicyclic ring such that the 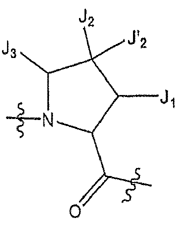 moiety is 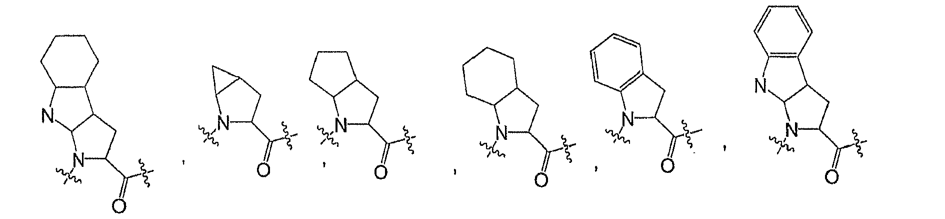 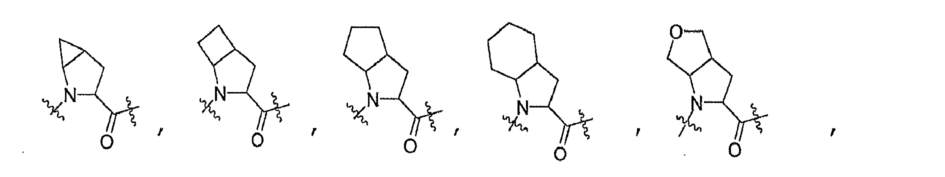 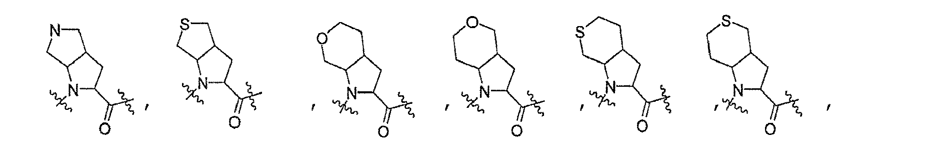 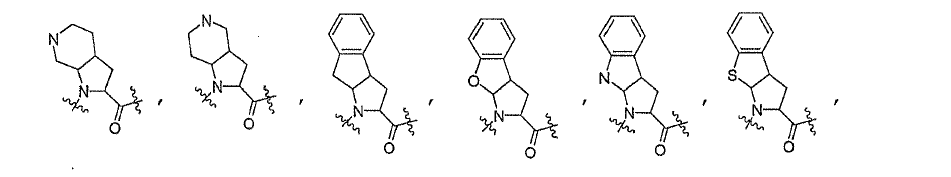 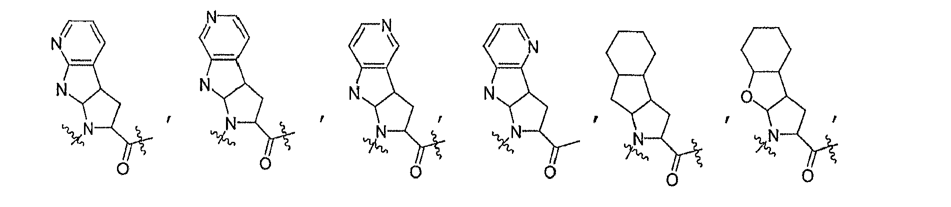 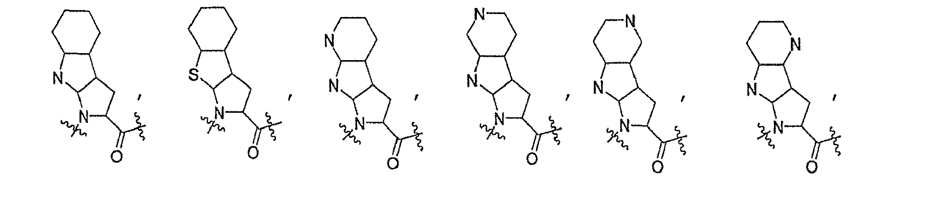 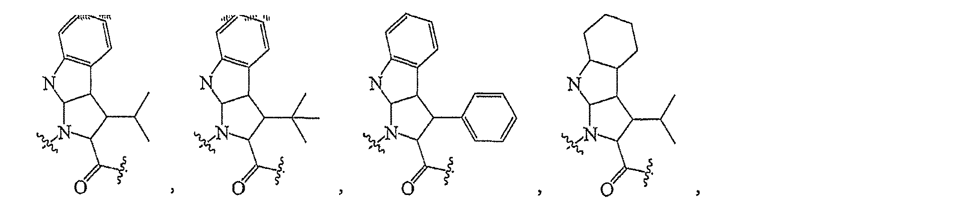 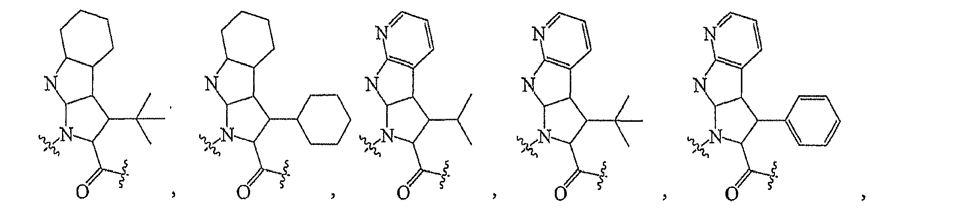 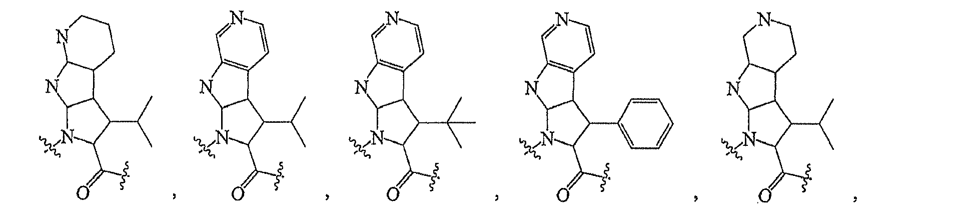 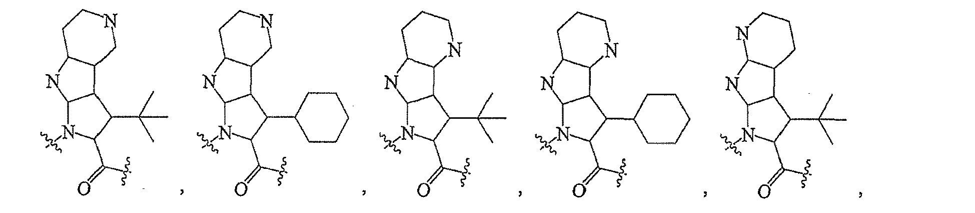 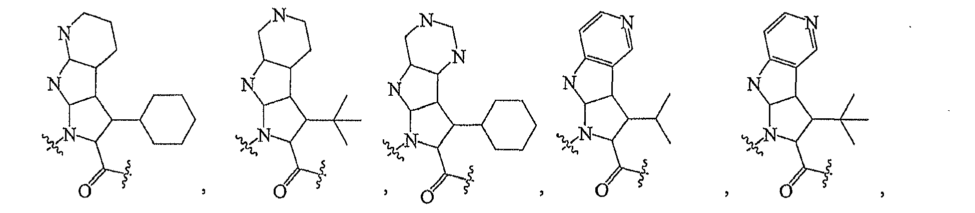 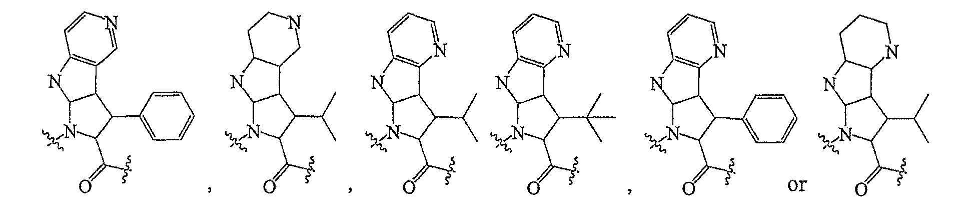
5. The mixture of diastereomeric compounds according to clause 1, wherein when J₁ and J₃, together with the atoms to which they are attached, form an optionally substituted Monocyclic aliphatic ring such that the 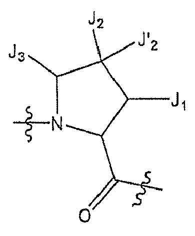 moiety is 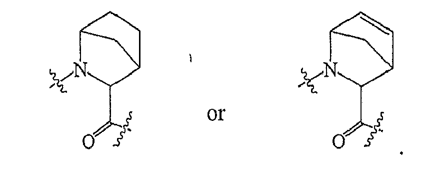
6. The mixture of diastereomeric compounds according to clause 1, wherein J₁ and J₂ together with the atoms to which they are attached form a monocyclic ring such that the 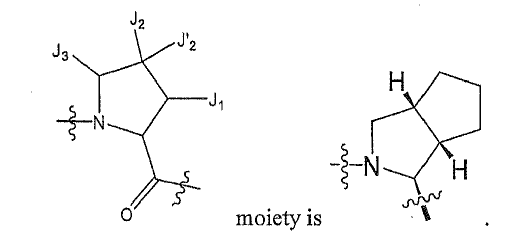
7. The mixture of diastereomeric compounds of clause 6, wherein L is -C(O)-.
8. The mixture of diastereomeric compounds according clause 7, wherein R₁ is 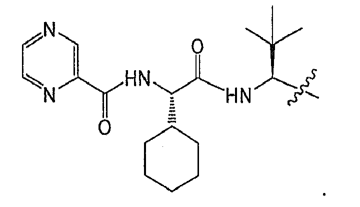
9. The mixture of diastereomeric compounds according to clause 1, wherein R₁ is RW-.
10. The mixture of diastereomeric compounds according to clause 9, wherein R is an optionally substituted aliphatic, an optionally substituted cycloaliphatic, an optionally substituted heterocycloaliphatic, an optionally substituted aryl, or an optionally substituted heteroaryl, and W is a bond, -O-, -S-, or -NR₄-.
11. The mixture of diastereomeric compounds according to clause 10, wherein R is an optionally substituted aryl or an optionally substituted heteroaryl; and W is -O-.
12. The mixture of diastereomeric compounds according to clause 10, wherein R is an optionally substituted aliphatic or an optionally substituted cycloaliphatic.
13. The mixture of diastereomeric compounds according to clause 12, wherein R is an optionally substituted arylalkyl or an optionally substituted heteroarylalkyl.
14. The mixture of diastereomeric compounds according to clause 10, wherein RW- is 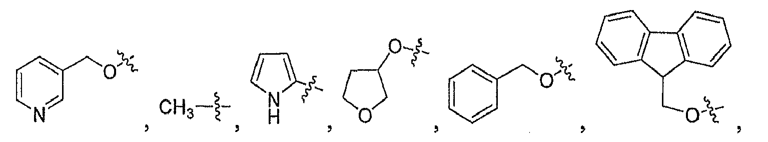 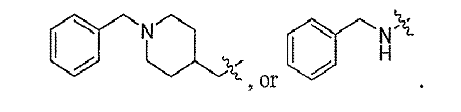
15. The mixture of diastereomeric compounds according to clause 6, wherein R₁ is P₃, P₃ is 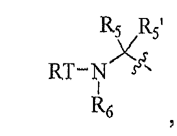 and
   Each of R₅ and R'₅ is independently an optionally substituted aliphatic, an optionally substituted cycloaliphatic, an optionally substituted heterocycloaliphatic, an optionally substituted aryl, or an optionally substituted heteroaryl;
   R₆ is an optionally substituted aliphatic, an optionally substituted cycloaliphatic, an optionally substituted heteroaryl, an optionally substituted phenyl; or
   R₅ and R₆, together with the atoms to which they are attached, form a 5- to 7-membered optionally substituted monocyclic heterocycle, or a 6- to 12-membered optionally substituted bicyclic heterocycle, in which each heterocycle ring optionally contains an additional heteroatom selected from -O-, -S- or -NR₅₀-; and
   T is -C(O)-, -OC(O)-, -NHC(O)-, -C(O)C(O)-, or -SO₂-,
16. The mixture of diastereomeric compounds according to clause 15, wherein T is -C(O)-,
17. The mixture of diastereomeric compounds according to clause 15, wherein T is - OC(O)-.
18. The mixture of diastereomeric compounds according to clause 15, wherein T is -NHC(O)-.
19. The mixture of diastereomeric compounds according to clause 15, wherein T is -C(O)C(O)-.
20. The mixture of diastereomeric compounds according to clause 15, wherein T is - S(O)₂-.
21. The mixture of diastereomeric compounds according to clause 6, wherein R₁ is P₄-L₂-P₃-; and P₄-L₂-P₃- is 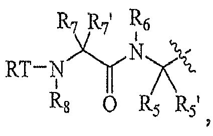 wherein
   Each of R₇ and R₇' is independently H, an optionally substituted aliphatic, an optionally substituted heteroaryl, or an optionally substituted phenyl; or R₇ and R₇', together with the atom to which they are attached, may form a 3- to 7-membered cycloaliphatic or heterocycloaliphatic ring; or R₇ and R₆, together with the atoms to which they are attached, may form a 5- to 7-membered optionally substituted monocyclic heterocycloaliphatic, a 5- to 7-membered optionally substituted monocyclic heteroaryl, a 6- to 12-membered optionally substituted bicyclic heterocycloaliphatic, or a 6- to 12-membered optionally substituted bicyclic heteroaryl, in which each heterocycloaliphatic or heteroaryl ring optionally contains an additional heteroatom selected from -O-, -S- or -NR₅₀-; or
   When R₅ and R₆, together with the atoms to which they are attached, form a ring, R₇ and the ring system formed by R₅ and R₆ may form an 8- to 14-membered optionally substituted bicyclic fused ring system, wherein the bicyclic fused ring system is optionally further fused with an optionally substituted phenyl to form an optionally substituted 10- to 16-membered tricyclic fused ring system;
   R₈ is H br a protecting group.
   R₅₀ is H, an optionally substituted aliphatic, an optionally substituted heteroaryl, or an optionally substituted phenyl.
22. The mixture of diastereomeric compounds according to clause 21, wherein R₇' is H; and R₇ is C₁-C₆ alkyl, C₃-C₁₀ cycloalkyl, C₃-C₁₀ cycloalkyl-C₁-C₁₂ alkyl, C₆-C₁₀ aryl, C₆-C₁₀ aryl-C₁-C₆ alkyl, 3- to 10-membered heterocycloaliphatic, 6- to 10-membered heterocycloaliphatic-C₁-C₁₂ alkyl, 5- to 10-membered heteroaryl, or 5- to 10-membered heteroaryl-C₁-C₁₂ alkyl.
23. The mixture of diastereomeric compounds of clause 22, wherein R₇ is 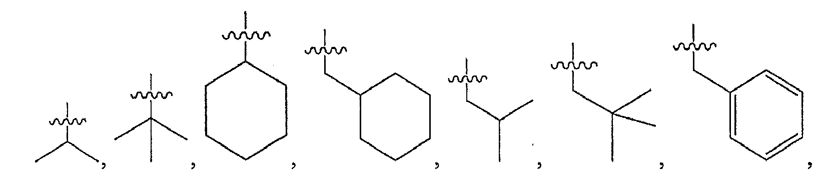 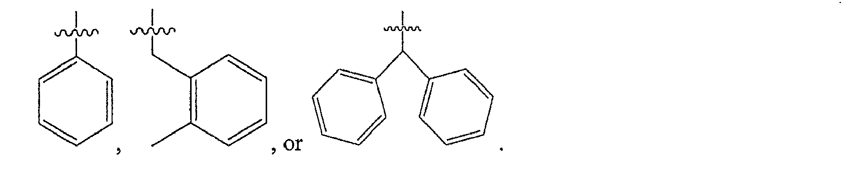
24. The mixture of diastereomeric compounds of clause 21, wherein R₇ and R₇', together with the atom to which they are attached, form a 3- to 7-membered optionally substituted cycloaliphatic ring.
25. The mixture of diastereomeric compounds of clause 24, wherein R₇ and R₇', together with the atom to which they are attached, form 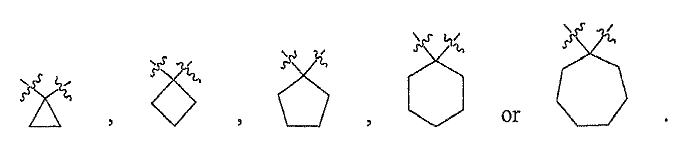
26. The mixture of diastereomeric compounds of clause 22, wherein R is 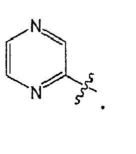
27. The mixture of diastereomeric compounds of clause 22, wherein R is 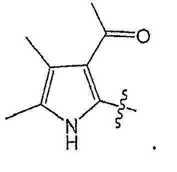
28. The mixture of diastereomeric compounds of clause 22, wherein R is: 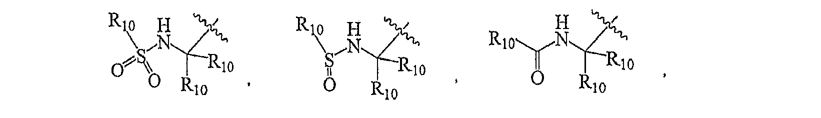 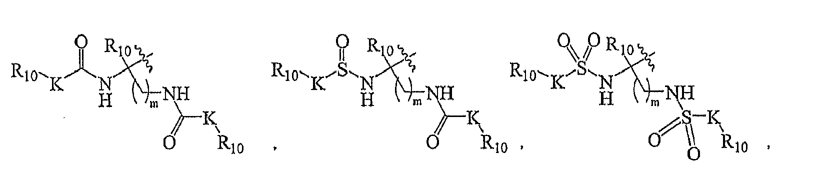 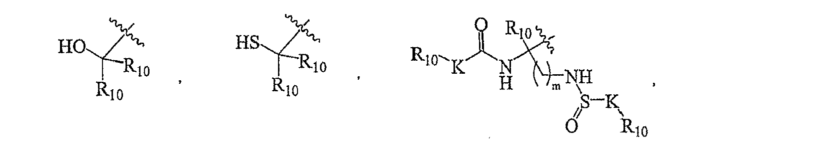 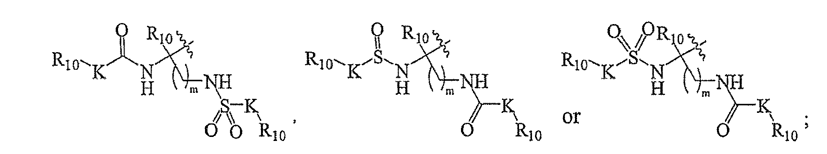
   Wherein R₁₀ is H, C₁-₁₂ aliphatic, C₆-₁₀ aryl, C₆-₁₀ aryl-C₁-₁₂ aliphatic, C₃-₁₀ cycloalkyl, C₃-₁₀ cycloalkenyl, C₃-₁₀ cycloalkyl-C₁-₁₂ aliphatic, C₃-₁₀ cycloalkenyl-C₁-₁₂ aliphatic, 3- to 10-membered heterocycloaliphatic, 6- to 10-membered heterocycloaliphatic-C₁-₁₂ aliphatic, 5- to 10-membered heteroaryl, or 5- to 10-membered heteroaryl-C₁-₁₂ aliphatic;
   K is a bond, C₁-₁₂ aliphatic, -O-, -S-, -NR₉-, -C(O)-, or -C(O)NR₉-, wherein R₉ is H or C₁-₁₂ aliphatic; and
   m is 1, 2, or 3.
29. The mixture of diastereomeric compounds of clause 22, wherein R is 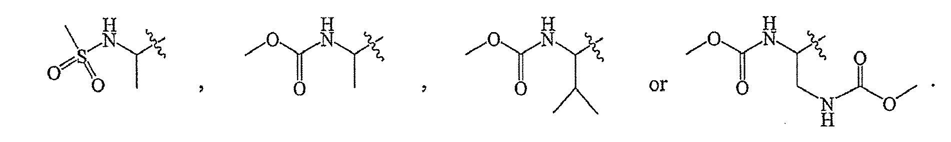
30. The mixture of diastereomeric compounds of clause 22, wherein R is 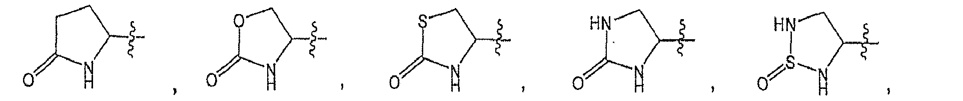 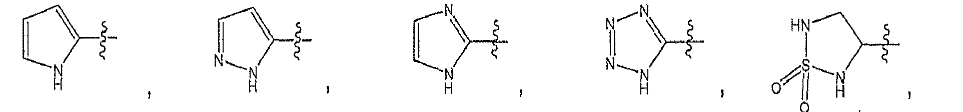 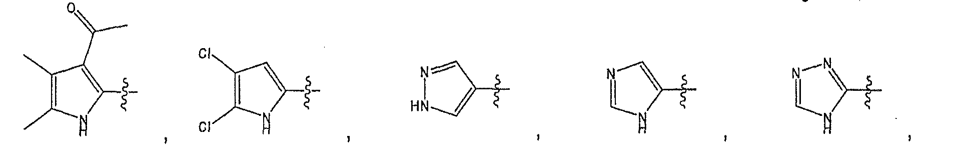 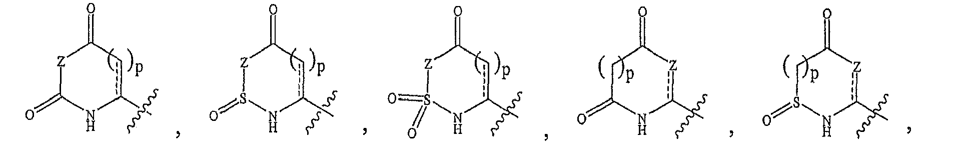 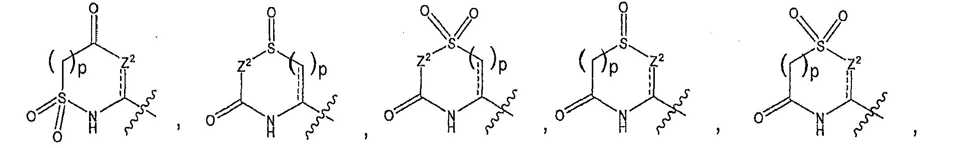 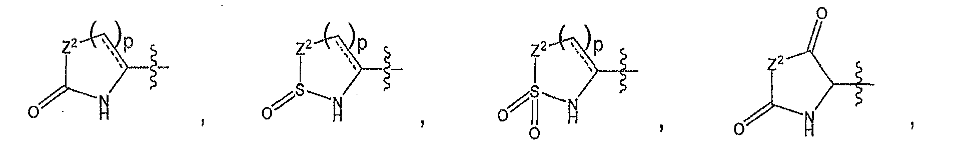 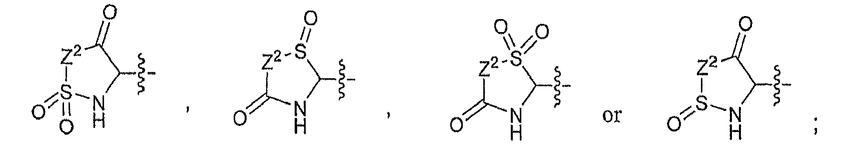 wherein
   Z² is O, S, NR₁₀, or C(R₁₀)₂;
   Each R₁₀ is independently H, C₁-₁₂ aliphatic, C₆-₁₀ aryl, C₆-₁₀ aryl-C₁-₁₂ aliphatic, C₃-₁₀ cycloalkyl, C₃-₁₀ cycloalkenyl, C₃-₁₀ cycloalkyl-C₁-₁₂ aliphatic, C₃-₁₀ cycloalkenyl-C₁-₁₂ aliphatic, 3- to 1 0-membered heterocycloaliphatic, 6- to 10-membered heterocycloaliphatic-C₁-₁₂ aliphatic, 5- to 10-membered heteroaryl, or 5- to 10-membered heteroaryl-C₁-₁₂ aliphatic;
   p is 1 or 2; and
   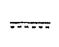 is a single bond or a double bond.
31. The mixture of diastereomeric compounds according to clause 21, wherein T is a bond; and R is an optionally substituted (heterocycloaliphatic)aliphatic.
32. The mixture of diastereomeric compounds according to clause 21, wherein T is a bond; and R is an optionally substituted aryl or an optionally substituted heteroaryl.
33. The mixture of diastereomeric compounds according to clause 21, wherein T is -C(O)-; and R is -NR₄.
34. The mixture of diastereomeric compounds according to clause 1, wherein R₂ is an optionally substituted aliphatic, an optionally substituted phenyl, an optionally substituted cycloaliphatic, or an optionally substituted heterocycloaliphatic.
35. The mixture of diastereomeric compounds of clause 34, wherein R₂ is 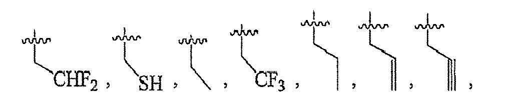 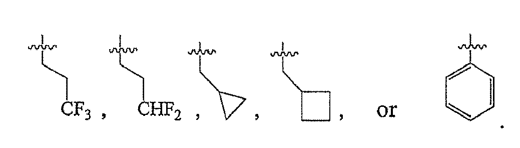
36. The mixture of diastereomeric compounds according to clause 34, wherein R₂ is *n-*propyl.
37. The mixture of diastereomeric compounds according to clause 1, wherein R₃ is an optionally substituted C₁-C₇ aliphatic, an optionally substituted cycloaliphatic, an optionally substituted aryl, or an optionally substituted heteroaryl.
38. The mixture of diastereomeric compounds according to clause 37, wherein R₃ is an optionally substituted C₁-C₆ alkyl or an optionally substituted C₁-C₆ cycloalkyl.
39. The mixture of diastereomeric compounds according to clause 38, wherein R₃ is 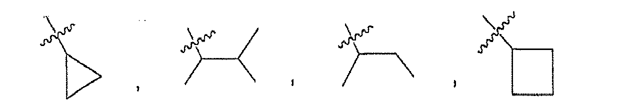 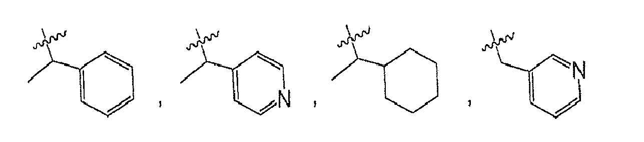 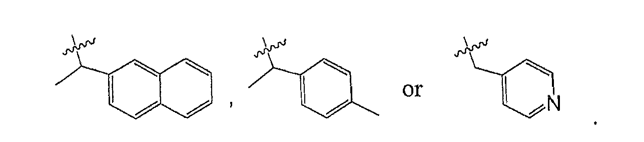
40. The mixture of diastereomeric compounds according to clause 39, wherein R₃ is cyclopropyl.
41. A mixture of diastereomeric compounds, comprising: 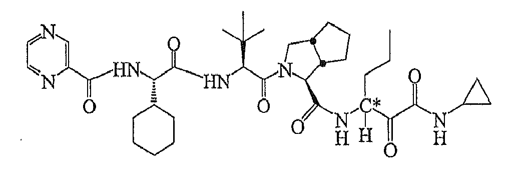 or a pharmaceutically acceptable salt or mixtures thereof, wherein C* represents a mixture of the R and S isomers; and the R isomer is greater than 50% of the mixture relative to the S isomer at the C* position.
42. The mixture of diastereomeric compounds according to clause 41, wherein the percentage of the R isomer in the mixture is greater than 60%.
43. The mixture of diastereomeric compounds according to clause 42, wherein the percentage of the R isomer in the mixture is greater than 70%.
44. The mixture of diastereomeric compounds according to clause 43, wherein the percentage of the R isomer in the mixture is greater than 80%.
45. The mixture of diastereomeric compounds according to clause 44, wherein the percentage of the R isomer in the mixture is greater than 90%.
46. The mixture of diastereomeric compounds according to clause 45, wherein the percentage of the R isomer in the mixture is greater than 95%.
47. The mixture of diastereomeric compounds according to clause 46, wherein the percentage of the R isomer in the mixture is greater than 98%.
48. The mixture of diastereomeric compounds according to clause 47, wherein the percentage of the R isomer in the mixture is greater than 99%.
49. A pharmaceutical composition comprising a mixture of diastereomeric compounds according to clause 1, in an amount effective to inhibit a serine protease; and an acceptable carrier, adjuvant or vehicle.
50. A pharmaceutical composition comprising a mixture of diastereomeric compounds according to clause 41, in an amount effective to inhibit a serine protease; and an acceptable carrier, adjuvant or vehicle.
51. The composition according to clause 50, wherein said composition is formulated for administration to a patient.
52. The composition according to clause 50, further comprising an immunomodulatory agent, an antiviral agent, a second inhibitor of HCV protease, an inhibitor of another target in the HCV life cycle, and a cytochrome P-450 inhibitor, or any combination thereof.
53. The composition according to clause 51, wherein said immunomodulatory agent is α-, β-, or γ-interferon or thymosin; said antiviral agent is ribavirin, amantadine, or telbivudine; or said inhibitor of another target in the HCV life cycle is an inhibitor of HCV helicase, polymerase, or metalloprotease.
54. The composition according to clause 52, wherein said cytochrome P-450 inhibitor is ritonavir.
55. A method of inhibiting the activity of a serine protease comprising the step of contacting said serine protease with a mixture of diastereomeric compounds according to clause 1, or a composition according to clause 49, in a pharmaceutically effective amount.
56. A method of inhibiting the activity of a serine protease comprising the step of contacting said serine protease with a mixture of diastereomeric compounds according to clause 41, or a composition according to clause 50, in a pharmaceutically effective amount.
57. The method according to clause 56, wherein said serine protease is an HCV NS3 protease.
58. A method of treating an HCV infection in a patient comprising the step of administering to said patient a mixture of diastereomeric compounds according to clause 1, or a composition according to clause 49, in a pharmaceutically effective amount.
59. A method of treating an HCV infection in a patient comprising the step of administering to said patient a mixture of diastereomeric compounds according to clause 41, or a composition according to clause 50, in a pharmaceutically effective amount.
60. The method according to clause 59, further comprising the additional step of administering to said patient an additional agent selected from an immunomodulatory agent; an antiviral agent; a second inhibitor of HCV protease; an inhibitor of another target in the HCV life cycle; or combinations thereof; wherein said additional agent is administered to said patient as part of said composition according to clause 49 or as a separate dosage form.
61. The method according to clause 60, wherein said immunomodulatory agent is α-, β-, or γ-interferon or thymosin; said antiviral agent is ribavarin or amantadine; or said inhibitor of another target in the HCV life cycle is an inhibitor of HCV helicase, polymerase, or metalloprotease.
62. A method of eliminating or reducing HCV contamination of a biological sample or medical or laboratory equipment, comprising the step of contacting said biological sample or medical or laboratory equipment with a mixture of diastereomeric compounds according to clause 1, or a composition according to clause 49.
63. A method of eliminating or reducing HCV contamination of a biological sample or medical or laboratory equipment, comprising the step of contacting said biological sample or medical or laboratory equipment with a mixture of diastereomeric compounds according to clause 41, or a composition according to clause 50.
64. The method according to clause 63, wherein said sample or equipment is selected from blood, other body fluids, biological tissue, a surgical instrument, a surgical garment, a laboratory instrument, a laboratory garment, a blood or other body fluid collection apparatus; a blood or other body fluid storage material.
65. The method according to clause 64, wherein said body fluid is blood.
66. The compound (1S,3aR,6aS)-2-[(28)-2-[[(2S)-2-cyclohexyl-1-oxo-2-[(pyrazinylcarbonyl)amino]ethyl]amino]-3,3-dimethyl-1-oxobutyl]-N-[(1R)-1-[2-(cyclopropylamino)-1,2-dioxoethyl]butyl]octahydro-cyclopenta[c]pyrrole-1-carboxamide.
67. A pharmaceutical composition comprising the compound according to clause 46, in an amount effective to inhibit a serine protease; and a acceptable carrier, adjuvant or vehicle.
68. A method of inhibiting the activity of a serine protease comprising the step of contacting said serine protease with the compound according to clause 66 or the composition according to clause 67.

## Claims

1. A mixture of diastereomeric compounds of Formula I: 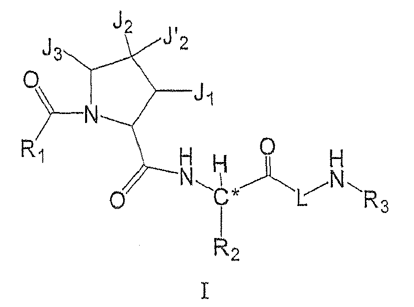 or a pharmaceutically acceptable salt or mixtures thereof, wherein
C* represents a diasteromeric carbon atom; and
the R isomer is greater than 50% of the mixture relative to the S isomer at the C* position;
R₁ is RW-, P₃-, or P₄-L₂-P₃-;
R is an optionally substituted aliphatic, an optionally substituted cycloaliphatic, an optionally substituted heterocycloaliphatic, an optionally substituted aryl, or an optionally substituted heteroaryl;
W is a bond, -NR₄-, -O-, or -S-;
R₄ is H, an optionally substituted aliphatic, an optionally substituted cycloaliphatic, an optionally substituted heterocycloaliphatic, an optionally substituted aryl, or an optionally substituted heteroaryl;
P₃- is 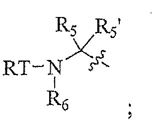
T is -C(O)-, -OC(O)-, -NHC(O)-, -C(O)C(O)-, or -SO₂-;
Each of R₅ and R₅' is independently H, an optionally substituted aliphatic, an optionally substituted cycloaliphatic, an optionally substituted heterocycloaliphatic, an optionally substituted phenyl, or an optionally substituted heteroaryl;
R₆ is an optionally substituted aliphatic, an optionally substituted heteroaryl, an optionally substituted phenyl; or R₅ and R₆, together with the atoms to which they are attached, may form a 5- to 7-membered optionally substituted monocyclic heterocycloaliphatic, or a 6- to 12-membered optionally substituted bicyclic heterocycloaliphatic, in which each heterocycloaliphatic ring optionally contains an additional heteroatom selected from -O-, -S- or -NR₅₀-;
R₅₀ is H, an optionally substituted aliphatic, an optionally substituted heteroaryl, or an optionally substituted phenyl;
P₄-L₂-P₃ is 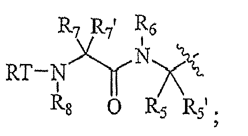
Each of R₇ and R₇' is independently H, an optionally substituted aliphatic, an optionally substituted cycloaliphatic, an optionally substituted heterocycloaliphatic, an optionally substituted phenyl, or an optionally substituted heteroaryl; or
R₇ and R₇', together with the atom to which they are attached, may form a 3- to 7-membered cycloaliphatic or heterocycloaliphatic ring; or
R₇ and R₆, together with the atoms to which they are attached, may form a 5- to 7-membered optionally substituted monocyclic heterocycloaliphatic, a 5- to 7-membered optionally substituted monocyclic heteroaryl, a 6- to 12-membered optionally substituted bicyclic heterocycloaliphatic, or a 6- to 12-membered optionally substituted bicyclic heteroaryl, in which each heterocycloaliphatic or heteroaryl ring optionally contains an additional heteroatom selected from -O-, -S- or -NR₅₀-, or
When R₅ and R₆, together with the atoms to which they are attached, may form a ring; R₇ and the ring system formed by R₅ and R₆ may form an 8- to 14-membered optionally substituted bicyclic fused ring system, wherein the bicyclic fused ring system is optionally further fused with an optionally substituted phenyl to form an optionally substituted 10- to 16-membered tricyclic fused ring system;
R₈ is H or a protecting group; and
R₂ is an optionally substituted aliphatic, an optionally substituted cycloaliphatic, an optionally substituted heterocycloaliphatic, an optionally substituted heteroaryl, or an optionally substituted phenyl;
R₃ is H, an optionally substituted aliphatic, an optionally substituted cycloaliphatic, an optionally substituted heterocycloaliphatic, an optionally substituted aryl, or an optionally substituted heteroaryl;
L is a bond, -CF₂-, -C(O)-, or -SO₂-;
Each of J₁, J₂, J'₂, and J₃ is independently halogen, -OR', -OC(O)N(R')₂, -NO₂, -CN, - CF₃, -OCF₃, -R', oxo, thioxo, -N(R')₂, -SR', -COR', -SO₂R', -SO₂N(R')₂, -SO₃R', -C(O)R', -C(O)C(O)R', -C(O)CH₂C(O)R', -C(S)R', -C(O)OR', -OC(O)R', -C(O)N(R')₂, -OC(O)N(R')₂, -C(S)N(R')₂, -(CH₂)₀₋₂NHC(O)R', -N(R')N(R')COR', -N(R')N(R')C(O)OR', -N(R')N(R')CON(R')₂, -N(R')SO₂R', -N(R')SO₂N(R')₂, -N(R')C(O)OR', -N(R')C(O)R', -N(R')C(S)R', -N(R')C(O)N(R')₂, -N(R')C(S)N(R')₂, -N(COR')COR', -N(OR')R', - C(=NH)N(R')₂, -C(O)N(OR')R', -C(=NOR')R', -OP(O)(OR')₂, -P(O)(R')₂, -P(O)(OR')₂, or - P(O)(H)(OR'), or of J₂ and J'₂ is H, wherein;
Two R' groups together with the atoms to which they are bound may form a 3- to 10-membered aromatic or non-aromatic ring system having up to 3 heteroatoms independently selected from N, O, or S, wherein the ring is optionally fused to a C₆-C₁₀ aryl, a C₅-C₁₀ heteroaryl, a C₃-C₁₀ cycloalkyl, or a C₃-C₁₀ heterocycloaliphatic, and wherein any ring has up to 3 substituents each independently selected from J₂;
Each R' is independently selected from H, C₁-C₁₂ aliphatic, C₃-C₁₀ cycloalkyl, or C₃-C₁₀ cycloalkenyl, C₃-C₁₀ cycloalkyl-C₁-C₁₂ aliphatic, C₃-C₁₀ cycloalkenyl-C₁-C₁₂ aliphatic, C₆-C₁₀ aryl, C₆-C₁₀ aryl-C₁-C₁₂ aliphatic, 3- to 10-membered heterocycloaliphatic, 6- to 10-membered hetexocycloaliphatic-C₁-C₁₂ aliphatic, 5- to 10-membered heteroaryl, or 5- to 10-membered heteroaryl-C₁-C₁₂ aliphatic, wherein R' has up to 3 substituents each independently selected from J₂; or
J₁ and J₂, together with the atoms to which they are attached, may form a C₈ to C₁₂ optionally substituted bicyclic ring;
J₁ and J₃, together with the atoms to which they are attached, may form a C₈ to C₁₂ optionally substituted bicyclic ring;
J₂ and J'₂, together with the carbon atom to which they are attached, may form an optionally substituted 5-10 membered cycloaliphatic, or an optionally substituted 5- to 10-membered heterocycloaliphatic ring; or
J₂ and J₃, together with the atoms to which they are attached, may form a C₈ to C₁₂ optionally substituted bicyclic ring.

2. The mixture of diastereomeric compounds according to claim 1, wherein the 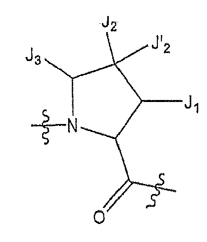 moiety is 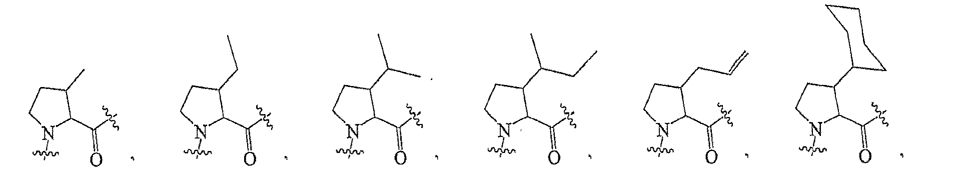 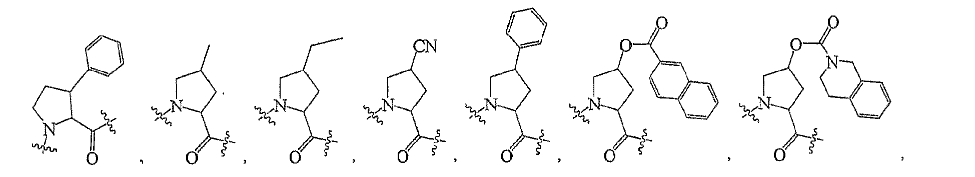 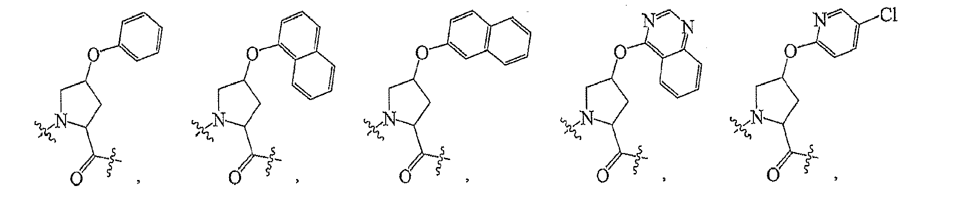 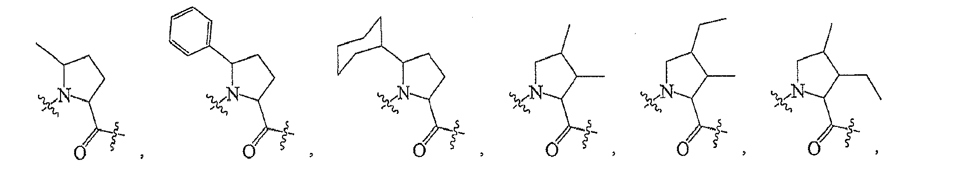 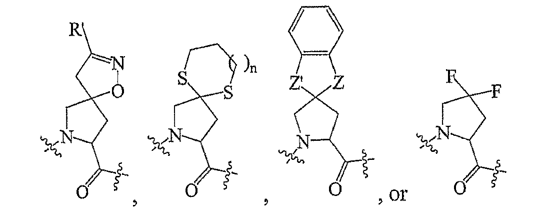 wherein n is 0 or 1; and each of Z and Z' is independently -CR'R'-, S, or O; or
wherein J₁ and J₂, together with the atoms to which they are attached, form an optionally substituted mono- or
bicyclic ring such that the 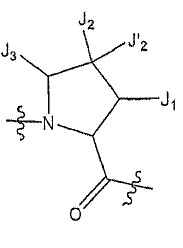 moiety is 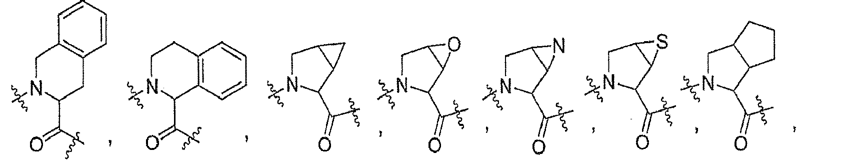 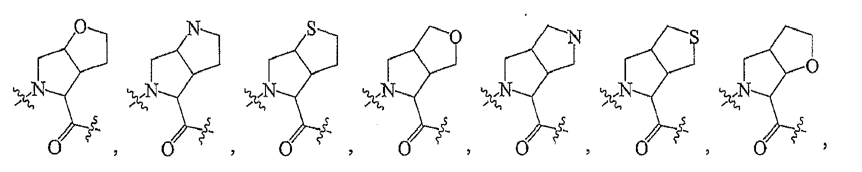 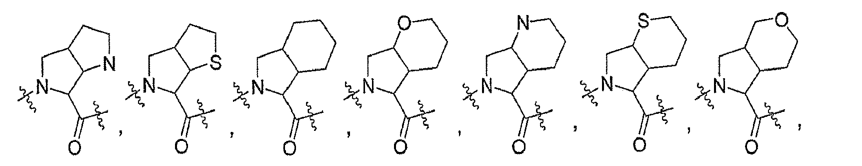 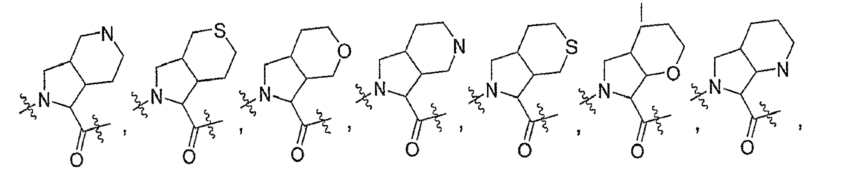 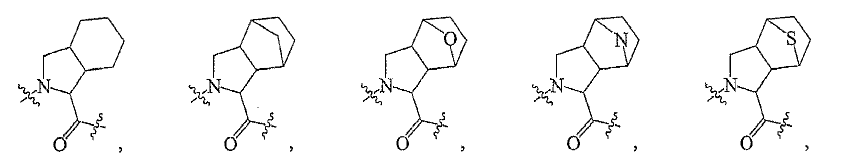 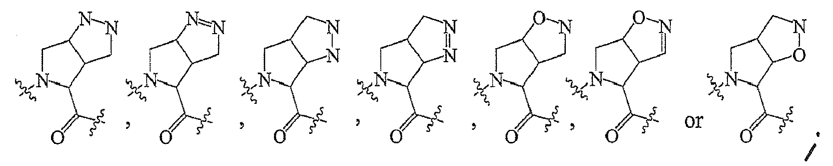 ; or wherein J₂ and J₃, together with the atoms to which they are attached, form an optionally substituted mono- or bicyclic ring such that the 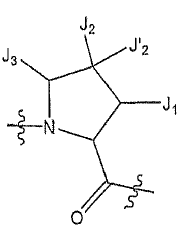 moiety is 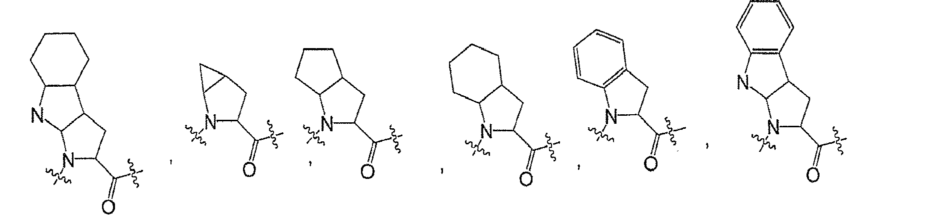 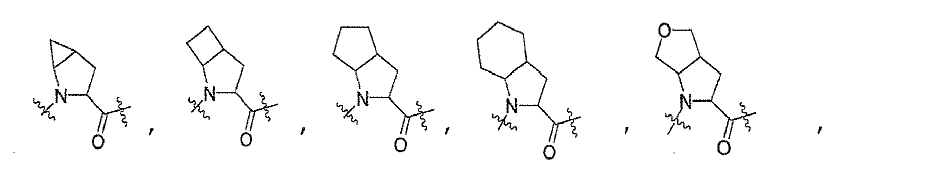 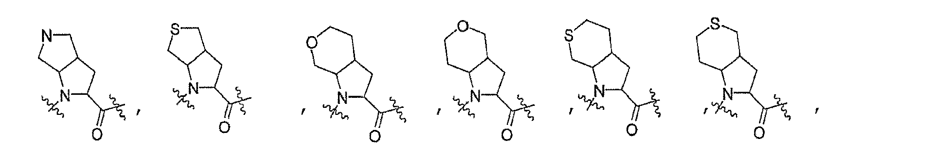 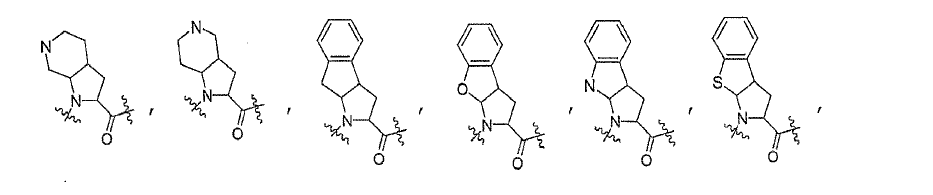 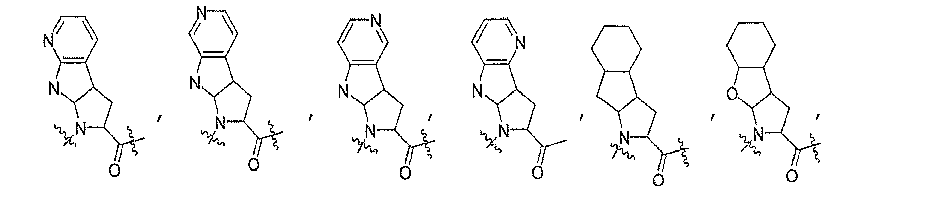 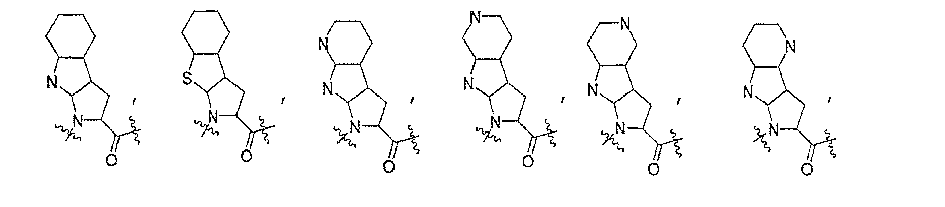 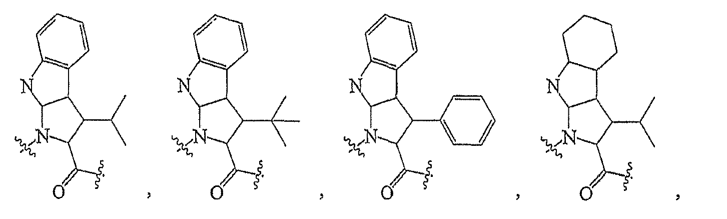 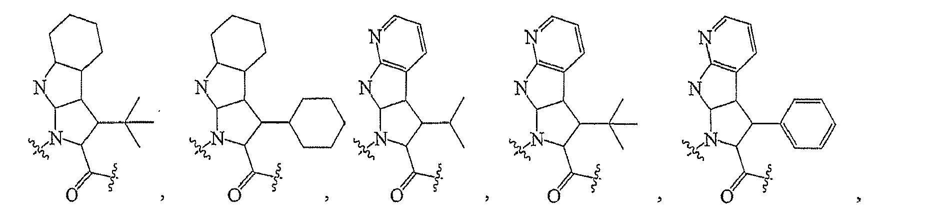 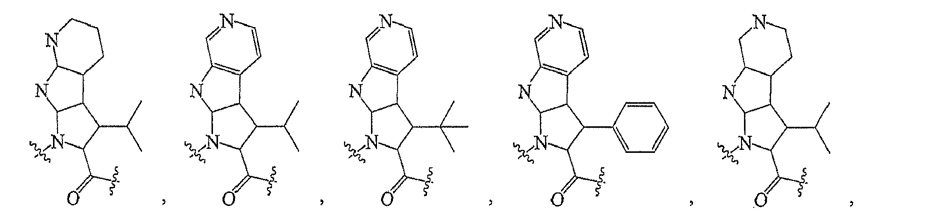 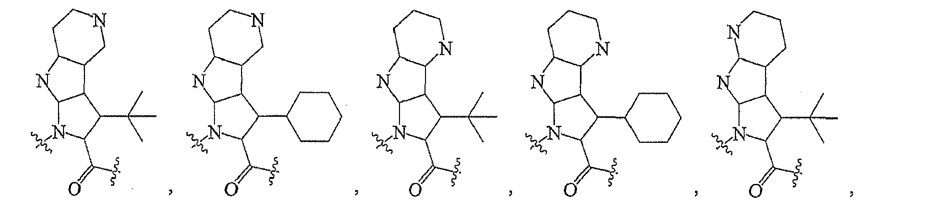 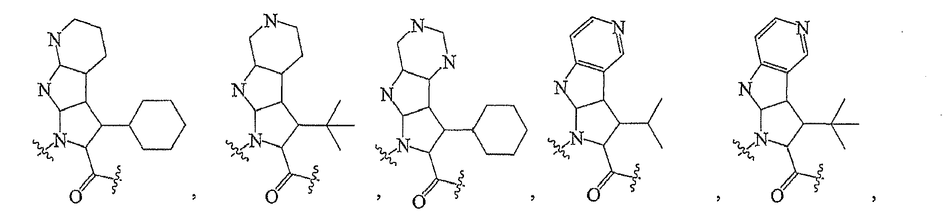 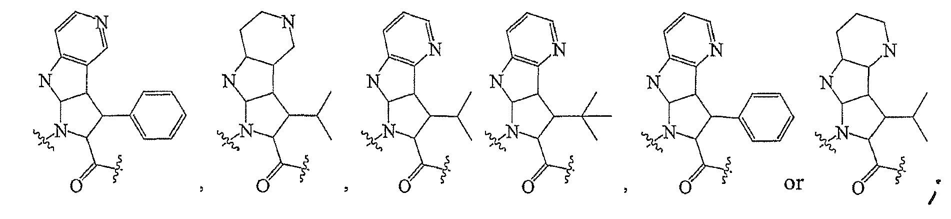 ; or
wherein when J₁ and J₃, together with the atoms to which they are attached, form an optionally substituted monocyclic aliphatic ring such that the 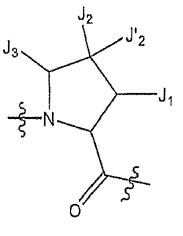 moiety is

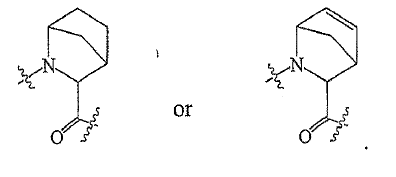

3. The mixture of diastereomeric compounds according to claim 1, wherein J₁ and J₂ together with the atoms to which they are attached form a monocyclic ring such that the 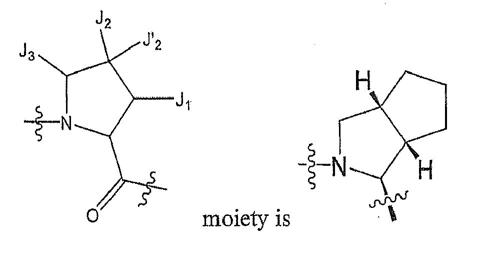 with L optionally beign -C(O)-.

4. The mixture of diastereomeric compounds according to claim 3, wherein R₁ is

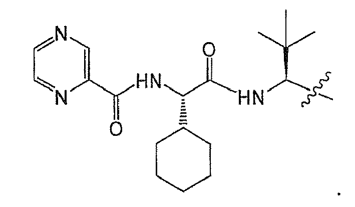

5. The mixture of diastereomeric compounds according to claim 1, wherein R₁ is RW-.

6. The mixture of diastereomeric compounds according to claim 5, wherein R is an optionally substituted aliphatic, an optionally substituted cycloaliphatic, an optionally substituted heterocycloaliphatic, an optionally substituted aryl, or an optionally substituted wherein R is an optionally substituted aryl or an optionally substituted heteroaryl; and W is -O-; or
wherein R is an optionally substituted aliphatic or an optionally substituted cycloaliphatic; or
wherein R is an optionally substituted arylalkyl or an optionally substituted heteroarylalkyl.

7. The mixture of diastereomeric compounds according to claim 6, wherein RW- is

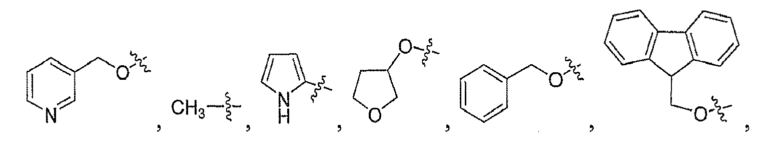

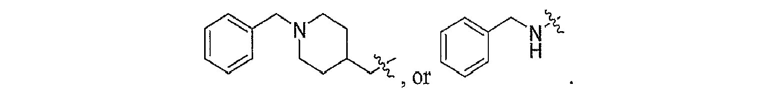

8. The mixture of diastereomeric compounds according to claim 3, wherein R₁ is P₃, P₃ is 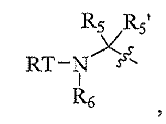 and
Each of R₅ and R'₅ is independently an optionally substituted aliphatic, an optionally substituted cycloaliphatic, an optionally substituted heterocycloaliphatic, an optionally substituted aryl, or an optionally substituted heteroaryl;
R₆ is an optionally substituted aliphatic, an optionally substituted cycloaliphatic, an optionally substituted heteroaryl, an optionally substituted phenyl; or
R₅ and R₆, together with the atoms to which they are attached, form a 5- to 7-membered optionally substituted monocyclic heterocycle, or a 6- to 12-membered optionally substituted bicyclic heterocycle, in which each heterocycle ring optionally contains an additional heteroatom selected from -O-, -S- or -NR₅₀-; and
T is -C(O)-, -OC(O)-, -NHC(O)-, -C(O)C(O)-, or -SO₂-.

9. The mixture of diastereomeric compounds according to claim 8, wherein T is -C(O)-; or
wherein T is - OC(O)-; or
wherein T is -NHC(O)-; or
wherein T is -C(O)C(O)-; or
wherein T is - S(O)₂-.

10. The mixture of diastereomeric compounds according to claim 3, wherein R₁ is P₄-L₂-P₃-; and P₄-L₂-P₃- is 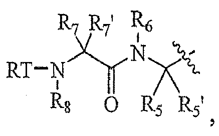 wherein
Each of R₇ and R₇' is independently H, an optionally substituted aliphatic, an optionally substituted heteroaryl, or an optionally substituted phenyl; or R₇ and R₇', together with the atom to which they are attached, may form a 3- to 7-membered cycloaliphatic or heterocycloaliphatic ring; or R₇ and R₆, together with the atoms to which they are attached, may form a 5- to 7-membered optionally substituted monocyclic heterocycloaliphatic, a 5- to 7-membered optionally substituted monocyclic heteroaryl, a 6- to 12-membered optionally substituted bicyclic heterocycloaliphatic, or a 6- to 12-membered optionally substituted bicyclic heteroaryl, in which each heterocycloaliphatic or heteroaryl ring optionally contains an additional heteroatom selected from -O-, -S- or -NR₅₀-; or
When R₅ and R₆, together with the atoms to which they are attached, form a ring, R₇ and the ring system formed by R₅ and R₆ may form an 8- to 14-membered optionally substituted bicyclic fused ring system, wherein the bicyclic fused ring system is optionally further fused with an optionally substituted phenyl to form an optionally substituted 10- to 16-membered tricyclic fused ring system;
R₈ is H or a protecting group.
R₅₀ is H, an optionally substituted aliphatic, an optionally substituted heteroaryl, or an optionally substituted phenyl.

11. The mixture of diastereomeric compounds according to claim 10, wherein R₇' is H; and R₇ is C₁-C₆ alkyl, C₃-C₁₀ cycloalkyl, C₃-C₁₀ cycloalkyl-C₁-C₁₂ alkyl, C₆-C₁₀ aryl, C₆-C₁₀ aryl-C₁-C₆ alkyl, 3- to 10-membered heterocycloaliphatic, 6- to 10-membered heterocycloaliphatic-C₁-C₁₂ alkyl, 5- to 10-membered heteroaryl, or 5- to 10-membered heteroaryl-C₁-C₁₂ alkyl.

12. The mixture of diastereomeric compounds of claim 11, wherein R₇ is 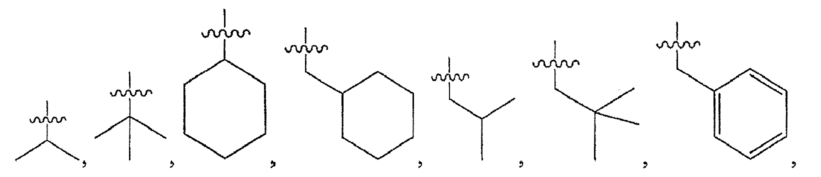 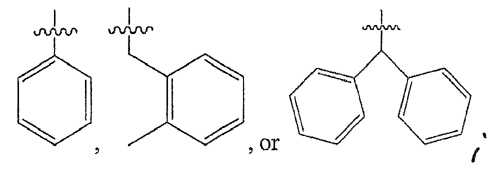 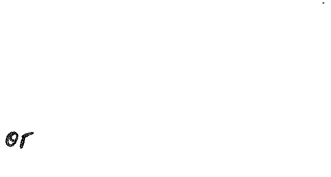 wherein R₇ and R₇', together with the atom to which they are attached, form a 3- to 7-membered optionally substituted cycloaliphatic ring.

13. The mixture of diastereomeric compounds of claim 12, wherein R₇ and R₇', together with the atom to which they are attached, form

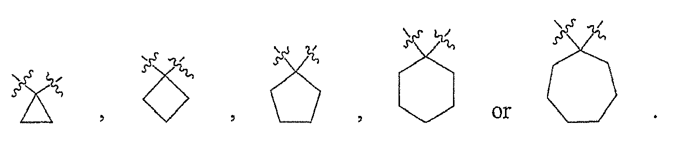

14. The mixture of diastereomeric compounds of claim 11, wherein R is 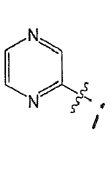 or
wherein R is 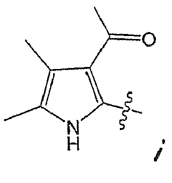 or
wherein R is: 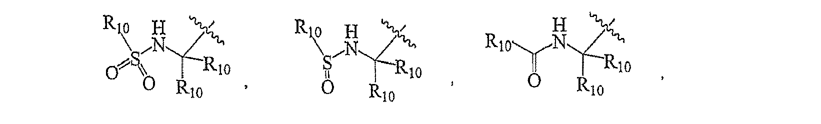 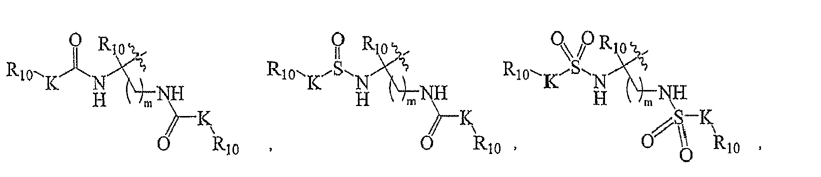 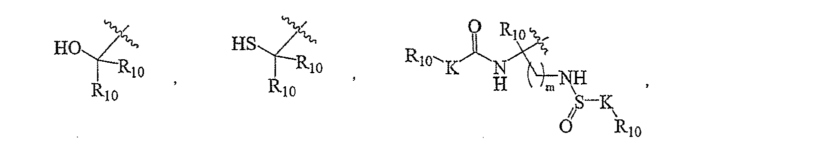 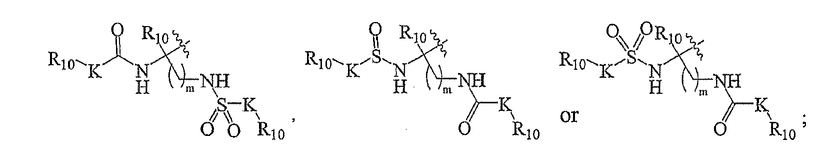 Wherein R₁₀ is H, C₁₋₁₂ aliphatic, C₆₋₁₀ aryl, C₆₋₁₀ aryl-C₁₋₁₂ aliphatic, C₃₋₁₀ cycloalkyl, C₃₋₁₀ cycloalkenyl, C₃-₁₀ cycloalkyl-C₁₋₁₂ aliphatic, C₃-₁₀ cycloalkenyl-C₁₋₁₂ aliphatic, 3- to 10-membered heterocycloaliphatic, 6- to 10-membered heterocycloaliphatic-C₁-₁₂ aliphatic, 5- to 10-membered heteroaryl, or 5- to 10-membered heteroaryl-C₁₋₁₂ aliphatic;
K is a bond, C₁₋₁₂ aliphatic, -O -S-, -NR₉-, -C(O)-, or -C(O)NR₉-, wherein R₉ is H or C₁₋₁₂ aliphatic; and
m is 1,2, or 3; or
wherein R is 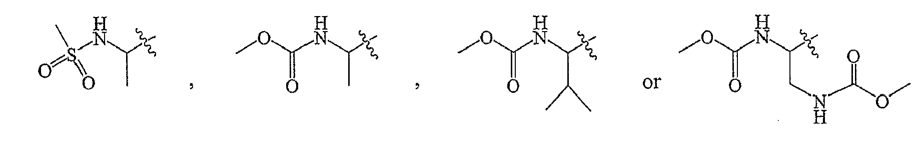 ; or wherein R is 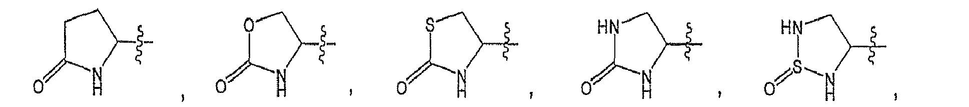 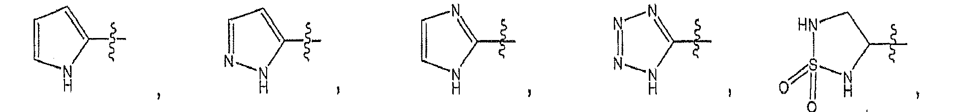 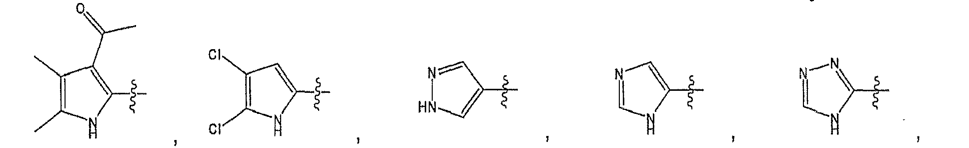 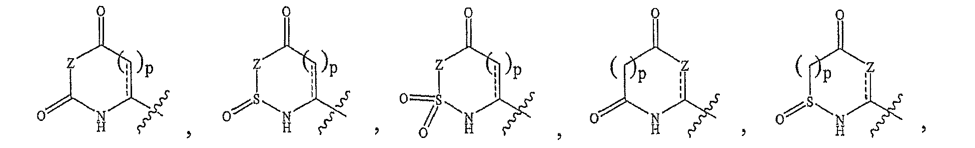 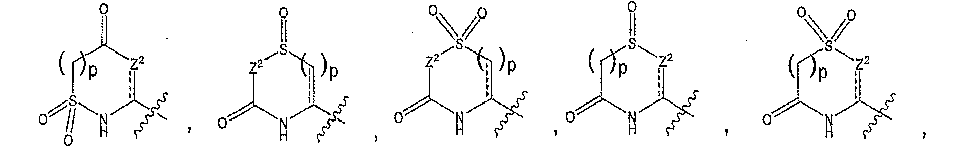 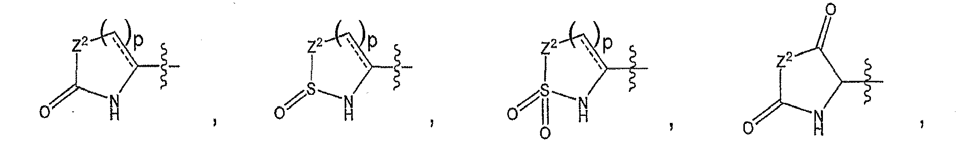 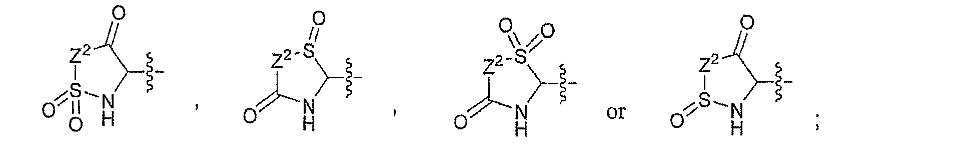 wherein
Z² is O, S, NR₁₀, or C(R₁₀)₂;
Each R₁₀ is independently H, C₁-₁₂ aliphatic, C₆-₁₀ aryl, C₆-₁₀ aryl-C₁-₁₂ aliphatic, C₃-₁₀ cycloalkyl, C₃-₁₀ cycloalkenyl, C₃-₁₀ cyloakyl-C₁-₁₂ aliphatic, C₃-₁₀ cyloakenyl-C₁₋₁₂ aliphatic, 3- to 10-membered heterocycloaliphatic, 6- to 10-membered heterocycloaliphatic-C₁-₁₂ aliphatic, 5- to 10-membered heteroaryl, or 5- to 10-membered heteroaryl-C₁₋₁₂ aliphatic;
p is 1 or 2; and
is a single bond or a double bond.

15. The mixture of diastereomeric compounds according to claim 10, wherein T is a bond; and R is an optionally substituted (heterocycloaliphatic)aliphatic; or
wherein T is a bond; and R is an optionally substituted aryl or an optionally substituted heteroaryl; or
wherein T is -C(O)-; and R is -NR₄.

16. The mixture of diastereomeric compounds according to claim 1, wherein R₂ is an optionally substituted aliphatic, an optionally substituted phenyl, an optionally substituted cycloaliphatic, or an optionally substituted heterocycloaliphatic.

17. The mixture of diastereomeric compounds of claim 16, wherein R₂ is 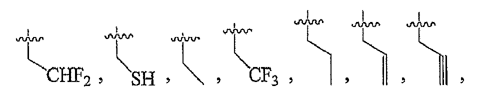 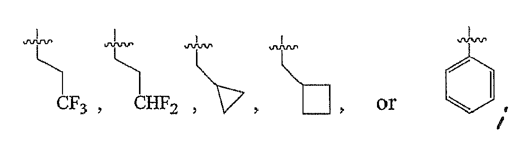 or
wherein R₂ is *n-*propyl.

18. The mixture of diastereomeric compounds according to claim 1, wherein R₃ is an optionally substituted C₁-C₇ aliphatic, an optionally substituted cycloaliphatic, an optionally substituted aryl, or an optionally substituted heteroaryl; or
wherein R₃ is an optionally substituted C₁-C₆ alkyl or an optionally substituted C₁-C₆ cycloalkyl; or
wherein R₃ is 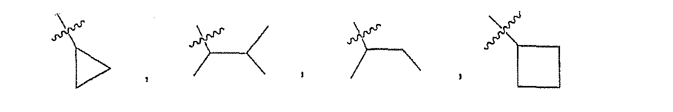 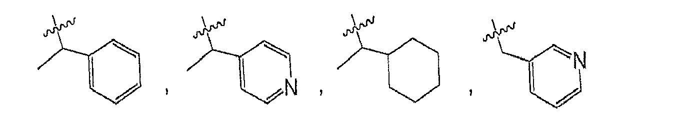 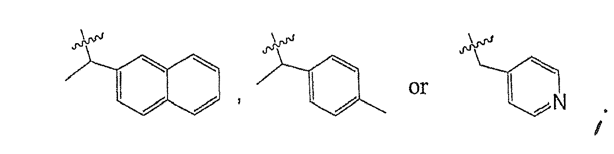 or
wherein R₃ is cyclopropyl.

19. A pharmaceutical composition comprising a mixture of diastereomeric compounds according to claim 1, in an amount effective to inhibit a serine protease; and an acceptable carrier, adjuvant or vehicle.

20. A method of inhibiting the activity of a serine protease in vitro comprising the step of contacting said serine protease with a mixture of diastereomeric compounds according to claim 1, or a composition according to claim 19, in a pharmaceutically effective amount.

21. A mixture of diastereomeric compounds according to claim 1, or a composition according to claim 19, in a pharmaceutically effective amount, for use in a method of treating an HCV infection in a patient

22. A method of eliminating or reducing HCV contamination of a biological sample or medical or laboratory equipment in vitro comprising the step of contacting said biological sample or medical or laboratory equipment with a mixture of diastereomeric compounds according to claim 1, or a composition according to claim 19.
